(19) Europäisches Patentamt
European Patent Office
Office européen des brevets

(11) EP 1 670 491 B1

# (12) EUROPEAN PATENT SPECIFICATION

(45) Date of publication and mention of the grant of the patent:
**29.07.2015 Bulletin 2015/31**

(21) Application number: **04809909.7**

(22) Date of filing: **08.10.2004**

(51) Int Cl.:
***A61K 31/704*** *(2006.01)* ***A61K 36/77*** *(2006.01)*
***A61P 35/00*** *(2006.01)*

(86) International application number:
**PCT/US2004/033359**

(87) International publication number:
**WO 2005/037200 (28.04.2005 Gazette 2005/17)**

(54) **COMPOSITION COMPRISING XANTHOCERAS SORBIFOLIA EXTRACTS, COMPOUNDS ISOLATED FROM SAME, METHODS FOR PREPARING SAME AND USES THEREOF**

ZUSAMMENSETZUNG MIT XANTHOCERAS SORBIFOLIA EXTRAKTEN, DARAUS ISOLIERTE VERBINDUNGEN, VERFAHREN FÜR IHRE HERSTELLUNG UND IHRE VERWENDUNGEN

COMPOSITION COMPRENANT DES EXTRAITS DE XANTHOCERAS SORBIFOLIA, COMPOSES ISOLES A PARTIR DE CES DERNIERS, PROCEDES DE PREPARATION ET UTILISATIONS DE CES DERNIERS

(84) Designated Contracting States:
**AT BE BG CH CY CZ DE DK EE ES FI FR GB GR HU IE IT LI LU MC NL PL PT RO SE SI SK TR**

(30) Priority: **09.10.2003 US 509851 P**
**23.12.2003 US 532101 P**

(43) Date of publication of application:
**21.06.2006 Bulletin 2006/25**

(73) Proprietor: **Pacific Arrow Limited**
**Hong Kong (CN)**

(72) Inventors:
- **CHAN, Pui-Kwong**
  **Sugarland, TX 77479 (US)**
- **MAK, May, Sung**
  **Kowloon**
  **Hong Kong (CN)**
- **WANG, Yun**
  **Dunedin (NZ)**

(74) Representative: **Lecca, Patricia S.**
**Cabinet Lecca**
**21 rue de Fécamp**
**75012 Paris (FR)**

(56) References cited:
**US-A1- 2003 082 293** **US-A1- 2003 096 030**
**US-B2- 6 616 943**

- **KONOSHIMA T ET AL: "ANTITUMOR AGENTS 82. CYTOTOXIC SAPOGENOLS FROM AESCULUS-HIPPOCASTANUM" JOURNAL OF NATURAL PRODUCTS (LLOYDIA), vol. 49, no. 4, 1986, pages 650-656, XP002510554 ISSN: 0163-3864**
- **D'ACQUARICA I ET AL: "Isolation and structure elucidation of four new triterpenoid estersaponins from fruits of Pittosporum tobira ait" TETRAHEDRON, ELSEVIER SCIENCE PUBLISHERS, AMSTERDAM, NL, vol. 58, no. 51, 16 December 2002 (2002-12-16), pages 10127-10136, XP004396869 ISSN: 0040-4020**
- **CHEN Y ET AL: "Studies on the constituents of xanthoceras sorbifolia BUNGE. III. Minor prosapogenins from the fruits of xanthoceras sorbifolia BUNGE" CHEMICAL AND PHARMACEUTICAL BULLETIN, PHARMACEUTICAL SOCIETY OF JAPAN, TOKYO, vol. 33, no. 1, 1 January 1985 (1985-01-01), pages 127-134, XP002961625 ISSN: 0009-2363**
- **SEO YOUNGWAN ET AL: "A new triterpene saponin from Pittosporum viridiflorum from the Madagascar rainforest." JOURNAL OF NATURAL PRODUCTS JAN 2002, vol. 65, no. 1, January 2002 (2002-01), pages 65-68, XP002510555 ISSN: 0163-3864**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 670 491 B1

- **APERS S ET AL: "New acylated triterpenoid saponins from Maesa lanceolata" PHYTOCHEMISTRY, PERGAMON PRESS, GB, vol. 52, no. 6, 1 November 1999 (1999-11-01), pages 1121-1131, XP004291125 ISSN: 0031-9422**
- **APERS SANDRA ET AL: "Antiviral, haemolytic and molluscicidal activities of triterpenoid saponins from Maesa lanceolata: Establishment of structure-activity relationships" PLANTA MEDICA, vol. 67, no. 6, August 2001 (2001-08), pages 528-532, XP002510116 ISSN: 0032-0943**
- **AHMAD V U ET AL: "THE SAPOGENINS FROM DODONAEA-VISCOSA" FITOTERAPIA, vol. 58, no. 5, 1987, pages 361-362, XP009110614 ISSN: 0367-326X**
- **DIZES C ET AL: "Harpuloside a triterpenoid saponin from Harpullia ramiflora" PHYTOCHEMISTRY, PERGAMON PRESS, GB, vol. 48, no. 7, 1 August 1998 (1998-08-01), pages 1229-1232, XP004289959 ISSN: 0031-9422**
- **JIANG Z ET AL: "Six triterpenoid saponins from Mmaesa laxiflora." JOURNAL OF NATURAL PRODUCTS JUN 1999, vol. 62, no. 6, June 1999 (1999-06), pages 873-876, XP002510553 ISSN: 0163-3864**


Remarks:

The file contains technical information submitted after the application was filed and not included in this specification

## Description

This application claims benefit of U.S. Serial Nos. 60/509, 851, Filed October 9, 2003 and U.S. Serial No. 60/532,101, Filed December 23, 2003.

## FIELD OF THE INVENTION

This invention relates to extracts from a plant called Wenguanguo (*Xanthoceras Sorbifolia*), their usages and functions, and methods of their preparation.

## BACKGROUND OF THE INVENTION

Wenguanguo is a species of the sapindaceae family. Its scientific name is Xanthoceras sorbifolia Bunge. Wenguanguo is the common Chinese name; others are Wenguannguo, Wenguanmu, Wenguanhua, Xilacedeng, Goldenhorn and Yellowhorn. Its seeds, leaves and flowers are edible and have been used as a folk medicine to treat enuresis for centuries. Its branches and woods are also used as a folk medicine.

Chinese patent applications CN 1092991A and CN 1092992A provided the methods for producing a medicine from Wenguanguo kernel powder for curing enuresis and enhancing cerebral functions Chinese patent CN 1052636C provided a method for producing a medicine with ethanol extract from the Wenguanguo kernel powder for curing enuresis and enhancing cerebral functions. Journal of Shenyang University of Pharmacy (2001), 18(1), 53-56 disclosed the n-butanol extract from the wood of Wenguanguo, which had anti-inflammatory effect.

United States Patent Application Publication No. 20030096030 provided the extracts from the husks of Wenguanguo which are Bunkankasaponin A. B. C. D and two sterols for proventing cerebral aging, improving cerebral functions and curing enuresis, frequent micturition, urinary incontinence, dementia, weak intelligence, and increasing the body's ability to resist the activity of glycosuria.

United States Patent Application Publication No. 20030082293 disclosed the extracts Bunkankasaponin A. B. C. D, crude fats, crude protein and sugars from the shell of Wenguanguo.

U.S. Patent Number 6,616,943, issued on September 9, 2003, provides the composition comprising Wenguanguo combined extracts and the methods for preparing them and uses thereof. The methods for preparing the combined extract from the husks comprise the following steps: extracting Wenguanguo husks with an organic solvent (e.g. ethanol) to form an organic (e.g. ethanol) extract; removing the organic solvent (e.g. ethanol) from the organic (e.g. ethanol) extract to form aqueous extracts; and drying and sterilizing the aqueous extracts to form the combined extracts. The combined extracts contain saponins, saccharides, proteins and others. The extracts can be used for producing medicines or health foods for preventing cerebral aging, improving memory, improving cerebral functions and curing enuresis, frequent micturition, urinary incontinence, dementia, weak intelligence and Alzheimer's disease, autism, brain trauma, Parkinson's disease and other diseases caused by cerebral dysfunction. The medicines or health foods further comprise Vitamin B, Vitamin D, K, anti-oxidant, Cordyceps or its extracts, gingko or its extracts, Echinacea, or its extracts, Huperzine A, folic acid, amino acids, creatine, fiber supplement or a combination thereof.

Yingjie Chen, Tadahiro Takeda and Yukio Ogihara in Chem. Pharm. Bull 33(4)1387-1394(1985) describe a study on the constituent of Xanthoceras sorbifolia Bunge. See Section V. Saponins from the Fruits of Xanthoceras sorbifolia. Four new saponins were isolated from the fruits of Xanthoceras sorbifolia Bunge. The structures of these saponins are bunkankasaponins A, B, C and D:

(A) 22-O-acetyl-21-O-(4-O-acetyl-3-O-angeloyl)-β-D-fucopyran osyl-3-O-[β-D-glucopyranosyl-(1→2)-β-D-glucuronopyranosyl]protoaecigenin

(B) 22-O-acetyl-21-O-(3,4-di-O-angeloyl) -β-D-fucopyranosyl -3-O-[β-D-glucopyranosyl- (1→2)-β-D-glucuronopyranosyl]protoaecigenin

(C) 28-O-acetyl-21-O-(4-O-acetyl-3-O-angeloyl)-β-D-fucopyran osyl-3-O-[β-D-glucopyranosyl-(1→2)-β-D-glucuronopyranosyl]protoaecigenin

(D) 28-O-acetyl-21-O-(3,4-di-O-angeloyl) -β-D-fucopyranosyl -3-O-[β-D-glucopyranosyl- (1→2)-β-D-glucuronopyranosyl]protoaecigenin

Yingjie Chen, Tadahiro Takeda and Yukio Ogihara in Chem. Pharm. Bull 33(3)1043-1048(1985) describe studies on the constituent of Xanthoceras sorbifolia Bunge. See Section IV. Structures of the Miner Prosapogenin. The prosapogenins from the partial hydrilyzate of fruit saponin of Xanthoceras sorbifolia were examinated, and are characterized as:

16-O-acetyl-21-O-(3,4-di-O-angeloyl-β-D-fucopyranosyl) protoaecigenin

22-O-acetyl-21-O-(3,4-di-O-angeloyl-β-D-fucopyranosyl) protoaecigenin 3-O-β-D-glucuronopyranoside.

Yingjie Chen, Tadahiro Takeda and Yukio Ogihara in Chem. Pharm. Bull 33(1)127-134(1985) describe studies on the constituent of Xanthoceras sorbifolia Bunge. See Section III. Minor Prosapogenins aponins from the Fruits of Xanthoceras sorbifolia Bunge. The structure of 3 minor prosapogenins, obtained by acid hydrolysis of the crude saponin faction, were characterized as:

21-O-(3,4-di-O-angeloyl)-β-D-fucopyranosyltheasapogenol B,

21-O-(4-O-acetyl-3-O-angeloyl)-β-D-fucopyranosyltheasapogen ol B,

21-O-(4-O-acetyl-3-O-angeloyl)-β-D-fucopyranosyl-22-O-acety lprotoaescigenin

Yingjie Chen, Tadahiro Takeda and Yukio Ogihara in Chem. Pharm. Bull 33(4)1387-1394(1985) described a study on the constituent of Xanthoceras sorbifolia Bunge. See Section II. Major Sapogenol and prosapogenin from the Fruits of Xanthoceras sorbifolia.

Cancer cells are defined by two heritable properties: (1) they reproduce in defiance of normal restraints on cell division; and (2) they invade and colonize territories normally reserved for other cells.

Cancers require mutations of many genes to develop, and they are classified according to the tissue and cell type from which they arise. Cancers arising from epithelial cells are named carcinomas; those arising from connective tissue or muscle cells are named sarcomas. In addition, there are cancers called leukemias, which are derived from hemopaietic cells; and cancers derived from cells of the nervous system.

Cancers originating from different types of cells are, in general, very different diseases. Each cancer has characteristics that reflect its origin. Even when a cancer has metastasized and proliferated out of control, its origins can be traced back to a single, primary tumor. Therefore it is important to develop drugs against target cells with a specified character.

Ovarian cancer is the 5th leading cause of cancer death in women and the leading cause of death from gynecologic. In the United States, females have a 1.4 to 2.5% (1 out of 40-60 women) lifelong chance of developing ovarian cancer. Older women are at highest risk. More than half of the deaths from ovarian cancer occur in women between 55 and 74 years of age and approximately one quarter of ovarian cancer deaths occur in women between 35 and 54 years of age. (See http://www.nlm.nih.gov/medlineplus/ency/article/000889.htm)

Ovarian cancer is disproportionately deadly for a number of reasons. First, symptoms are vague and non-specific, so women and their physicians frequently attribute them to more common conditions. By the time the cancer is diagnosed, the tumor has often spread beyond the ovaries. Also, ovarian cancers shed malignant cells that frequently implant on the uterus, bladder, bowel, and lining of the bowel wall (omentum). These cells can begin forming new tumor growths before cancer is even suspected. Second, because no cost-effective screening test for ovarian cancer exists, more than 50 percent of women with ovarian cancer are diagnosed in the advanced stages of the disease.

This invention provides compounds or compositions extracted from Xanthoceras Sorbifolia or synthesized which have substantial potency against ovarian cancer.

## SUMMARY OF THE INVENTION

In accordance with these and other objects of the invention, a brief summary of the present invention is presented. Some simplifications and omission may be made in the following summary, which is intended to highlight and introduce some aspects of the present invention, but not to limit its scope. Detailed descriptions of a preferred exemplary embodiment adequate to allow those of ordinary skill in the art to make and use the invention concepts will follow in later sections. Extract from husks, leaves, branch or stem, and fruit-stem, root and bark of the Wenguanguo may be combined and this invention discloses method of their preparation.

This invention provides a composition comprising the combined extracts from the husks, leaf or branch, stem and or fruit-stem, root, seed's shell and bark of the Wenguanguo. The organic extracts contain saponins, saccharides, proteins, glycosides, flavonoids and others.

This invention provides the crude saponins from the husks, leaf or branch, stem and or fruit-stem root, seed's shell and bark of Wenguanguo and methods of their preparation. This invention provides a composition comprising the crude saponins from the husks, leave or branch, stem and or fruit-stem root and bark of the Wenguanguo. The crude saponins contain saponins, saccharides, proteins, and others.

Wenguanguo extracts may be used for accelerating the growth of bladder, for suppressing deep sleep, for increasing alterness in a sleeping subject, for modulating the release, breakdown and uptake of Antidieuretic hormone

(ADH) and its receptors, for modulating the secretion, breakdown and uptake of Adrenocorticotropic hormone (ACTH) and its receptors, for modulating the release, breakdown and uptake of 5-hydroxytryptamine and its receptors, for modulating the release, breakdown and uptake of Acetycholine (Ach) and its receptors, for modulating the release, breakdown and uptake of Adrenaline (AD) and its receptors, for modulating the release, breakdown and uptake of Dopamine (DA) and its receptors, for modulating the release, breakdown and uptake of Norepinephrine (NE) and its receptors, for preventing sleep paralysis, for modulating the formation, release, breakdown and activity of neuropeptides and their receptors, for curing cancer, including but not limited to breast cancer, leukocyte cancer, liver cancer, ovary cancer, bladder cancer, prostate cancer and brain cancer, and for improving the functions of the lung and the bladder.

This invention provides a compound comprising the following structure:

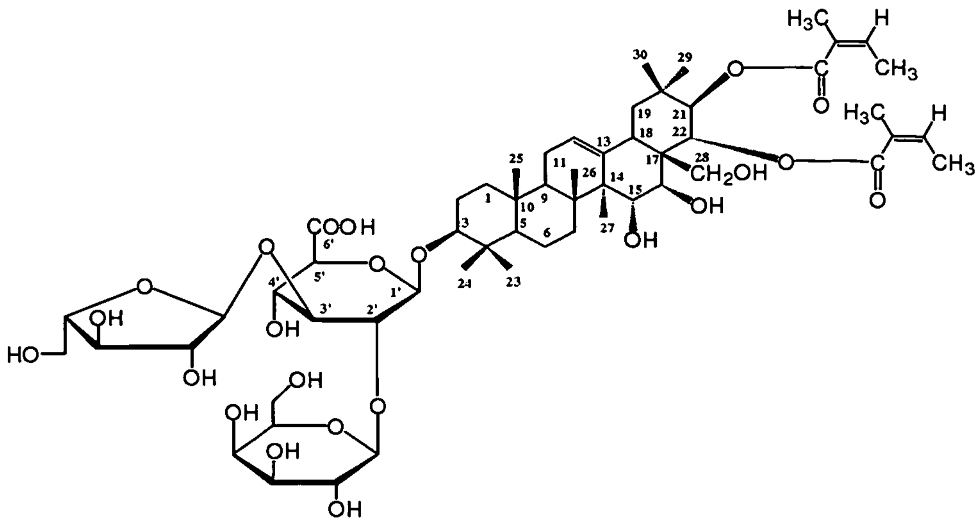

This invention provides a salt of the above-described compounds.

This invention provides a composition comprising the above-described compounds and a suitable carrier. This invention provides a pharmaceutical composition comprising an effective amount of the above-described compounds and a pharmaceutically acceptable carrier

This invention provides a method for isolating compounds from Xanthoceras Sorbifolia comprising steps of: extracting Xanthoceras Sorbifolia powder with an appropriate amount of one or more organic solvent for an appropriate amount of time to form an organic extract; collecting the organic extract; refluxing the organic extract with an organic solvent for an appropriate amount of time to form a second extract; removing the organic solvent from the second extract; and drying and sterilizing the second extract to form a Xanthoceras Sorbifolia extract powder.

This invention provides a triterpenoid saponins with three sugars and one or more biangeloyl groups attached to the backbond.

This invention provides a triterpenoid saponins with two sugars and one or more angeloyl groups attached to the backbond.

This invention provides a triterpenoid saponins with four sugars and one or more angeloyl groups attached to the backbond. This invention provides a triterpenoid saponins with three sugars and one or more angeloyl group attached to the backbond.

This invention provides a method for isolating compounds from Xanthoceras Sorbifolia comprising steps of: extracting Xanthoceras Sorbifolia powder of with an appropriate amount of one or more organic solvent for an appropriate amount of time to form an organic extract; collecting the organic extract; refluxing the organic extract to form a second extract; removing the organic solvent from the second extract; drying and sterilizing the second extract to form a Xanthoceras Sorbifolia extract powder; fractionating the extract powder to obtain one or more components of the extract powder; identifying the bioactive components of the extract powder; purifying one or more bioactive components of the extract powder with FPLC to obtain one or more fraction of the bioactive component; and isolating the compound with preparative HPLC.

This invention provides a compound having a NMR spectral data on the Heteronuclear Multiple Quantum Correlation (HMQC) study as shown in Table 5.2.

This invention provides a compound having a NMR spectral data on Heteronuclear Multiple Bond Correlation (HMBC) study as shown in Table 5.3.

In accordance with these and other objects of the invention, a brief summary of the present invention is presented. Some simplifications and omission may be made in the following summary, which is intended to highlight and introduce some aspects of the present invention, but not to limit its scope. Detailed descriptions of a preferred exemplary embodiment adequate to allow those of ordinary skill in the art to make and use the invention concepts will follow in later sections. This invention provides the pathways of a compound having the formula $C_{57}H_{88}O_{23}$ and the chemical name 3-O-[β-D-galactopyranosyl(1→2)]-α-L-arabinofuranosyl(1→3)-β-D-glucuronopyranosyl-21,22-O-diangeloyl-3β, 15α, 16α, 21β, 22α, 28-hexahydroxyolean-12-ene, and derivative compounds which are effective against cancer. The compounds or compositions of the present invention regulate the receptors or components of cell such as G-protein receptor, Fas protein, receptor Tyrosine Kinases, Mitogen, mitogen receptor. The compounds can be isolated from the plant called Xanthoceras Sorbifolia or can be synthesized chemically, or extracted from other biological sources.

Normal cells cease to divide when they mature into terminally differentiated, specialized cells. Once cells progress through a certain number of population doubling, they stop proliferating, a process termed replicative cell senescence. However, if a cell persists in dividing so as to generate a cancer, it escapes the restraints of replicative senescence and avoids differentiation. These cells are genetically unstable, and disregard the external and internal signals that regulate cell proliferation. They escape from their home tissues, and are therefore invasive. They can survive and proliferate in foreign sites, thereby causing more tumors.

Abnormal changes in components' activities in pathways cause the cells to fail to stop proliferating so as to form cancer. The pathways include TGF Beta-smad, FGF, TGF-beta and TGF-alphaa, ras-GTPase-MAP kinase, jun-fos, Src-fyn, Jak-Jnk-STAT, BMP, Wnt, myc-cell proliferation, etc. The mutation of cancer cell causes the cell-death program to become inactive, allowing cells to divide indefinitely. The Xanthoceras Sorbifolia derived compound and/or composition regulates the components and receptors and re-activates the cell death program.

## DETAILED DESCRIPTION OF THE FIGURES

**Figure** 1. Shows the efficacy of Wanguanguo extracts in inhibiting breast cancer cells, where IC50 is 65μg/ml and IC0 is 105μg/ml. Experimental results in Figure 6 also showed that the extracts inhibit leukocyte cancer cells, where IC50 is 35μg/ml and IC0 is 50μg/ml.

**Figure** 2. Shows the efficacy of the Wanguanguo extracts in increasing the activity of bladder cells. The bladder cells grow up to 125% in extract concentration of 10μg/ml. Experimental results in Figure 7 also showed that wanguanguo extracts inhibit bladder cancer cells in high concentration extract with IC50 - 45 and IC0 - 60. Experimental results in Figure 7 showed that Wanguanguo extracts inhibit ovary cancer cells, where IC50 is 15μg/ml and IC0 is 20μg/ml.

**Figure** 3. Shows the efficacy of Wanguanguo extracts in increasing the activity of brain cells. Brain cells grow up to 108% in extract concentration of 10μg/ml. Experimental results in Figure 8 showed that the extracts inhibit prostate cancer cells, where IC50 is 40μg/ml and IC0 is 70μg/ml. Experimental results in Figures 8 shows that the extracts inhibit brain cancer cells, where IC50 is 70μg/ml and IC0 is 100μg/ml.

**Figure 4.** Shows the efficacy of the Wanguanguo extracts in increasing the activity of lung cells. Experimental results in Figure 9 showed that lung cells began to grow in culture medium RPMI-1640 containing extract concentration of 10ug/ml. Experimental results in Figure 9 also showed that lung cells grew up to 120% in extract concentration of 50ug/ml. Figure 9 also showed the efficacy of Wanguanguo extract in increasing the activity of bladder cells. The bladder cells grow up to 125% in culture medium RPMI-1640 containing extract concentration of 10μg/ml. Experimental results in Figure 9 also showed that the extracts inhibit bladder cancer cells in high concentration extract with IC50 - 45 and IC0 - 60. Experimental results in Figure 9 also showed that the extracts inhibit liver cancer cells, where IC50 is 68μg/ml and IC0 is 95μg/ml.

**Figure 5.** Shows the Wanguanguo extracts has slight effect on cervix cells.

**Figure** 6. Shows that the extracts inhibit bone cancer cells in high concentration extract with IC50 - 40 and IC0 - 50.

**Figure** 7. Shows the separation of an extract of Xanthoceras Sorbifolia components by HPLC with a μbondapak C18 column.

**Figure 8A-B.** Shows the isolation of Fraction Y From an extract of Xanthoceras Sorbifolia by FPLC. A and B are representative elution profile of FPLC chromatograms.

**Figure 9**. Shows the absorption spectrum of Xanthoceras Sorbifolia extract.

Abscissa: Wavelength in nm
Ordinate: Optical Density.
The extract has three absorption maximum at 207nm, 278nm and 500nm.

**Figure 10A-10E.** Shows the growth curves of cells after treatment with the extract of Xanthoceras Sorbifolia as determined by MTT assay These studies reported the sensitivities of different cell lines toward the extract of Xanthoceras Sorbifolia.

(10A) Most sensitive: Ovary.
(10B) Sensitive: bladder and bone.
(10C) Semi-sensitive: leukocyte and liver.
(10D) Marginal sensitive: prostate, breast and brain.
(10E) Least sensitive: colon, cervix and lung.

**Figure 11.** Shows the elution profile of an extract of Xanthoceras Sorbifolia in FPLC with 10-80% gradient.

Ordinate: optical density.
Abscissa: fraction volume (5ml/fraction).

**Figure 12.** Shows screening of cell growth activity of FPLC fractions (conducted with bladder cells). 12A Shows the elultion fractions obtained from FPLC. These fractions were subsequently used in MTT assay to determine which fraction is active. 12B shows that different components of *Xanthoceras Sorbifolia* extracts (as fractionated by FPLC) cause either growth or inhibition effects on cells. Only fraction 5962 (Y components) cause cell inhibition. Fractions 610 and 1116 cause minor stimulation of cell growth.
Abscissa: concentration (ul/ml)
Ordinate: % Cell Growth (determined by MTT assay).

**Figure 13.** Shows elution profile of Fraction 5962 with 64% isocratic elution. Two major FPLC fractions X and Y are separated.)
Ordinate: optical density.
Abscissa: fraction number (1ml/fraction).

**Figure 14.** Shows the comparison of inhibition activity in bladder cells by Fractions X (2021)and Y (2728). Only Y has inhibition activity.

**Figure 15.** Shows the HPLC profile of Fraction Y with 35% isocratic elution. Figure 28 also shows on 35% isocratic analysis, the Y (2728) fraction contains 4-5 components (Y0, Y1, Y2, Y3 and Y4).

**Figure 16.** Shows HPLC profile of Fraction Y with 45% Acetonitrile isocratic elution. Figure 16 also shows on 45% isocratic analysis, Y3 and Y4 peaks merge into one peak. In this condition, the fractions Y1 and 2 are nicely separated from fractions Y3 and Y4.

**Figure 17.** Shows HPLC profile of Fraction Y with 45% Acetonitrile isocratic elution in a preparative C18 coloum (Delta Pak C18). Under these conditions, fractions Y1 and Y2 are well separated from each other and they are collected individually.

**Figure 18.** Compares Proton-NMR profiles of ascending (upper curve) and descending (lower curve) portion of peak Y(3/4) and shows they are identical.

**Figure 19.** Shows HPLC profile of collected Fraction Y with 45% isocratic elution in a preparative C18 column.

**Figure 20.** Shows Potency of purified Fraction Y on ovarian cancer cells (OCAR-3).

Abscissa: concentration (ug/ml). **A:** Point scale. **B** Linear scale.
Ordinate: % Cell Growth (determined by MTT). The IC50 is approximately 1 ug/ml.

**Figure 21.** Shows comparison of potency of Fraction Y toward ovarian cancer cells and cervical cancer cells. Ovarian cancer cells are sensitive to Fraction Y while cervical cancer cells are not. The IC50 for Fraction Y in ovary cells is

about 1. This result confirms that ovary cancer is sensitive to purified Fraction Y.

**Figure 22.** Shows the chemical shift of the proton NMR of Fraction Y. Also see Table 5.1 for listed chemical shift data.

**Figure 23.** Shows HMQC results of Sample Y. Also see Table 5.2 for listed chemical shift data.

**Figure 24.** Shows HMBC results of Y. **A:** level one. **B:** level 2. Also see Table 5.3 for listed chemical shift data.

**Figure 25.** Shows the Mass spectroscopy of Y compound. **(A)** MALDI-TOF (high mass) **A1:** Y + Matrix (CHCA) + Angiotensin 1 "two point calibration". **A2:** Y + Matrix (CHCA) + Angiotensin "one point calibration". **A3:** Y + Matrix (CHCA). **A4:** Matrix (CHCA) only. **A5:** Angiotensin 1 + Matrix (CHCA). **(B)** ESI-MS.

**Figure 26.** Shows the structure and chemical name of Y: 3-O-[β-D-galactopyranosyl(1→2)]-α-L-arabinofuranosyl(1→3)-β-D-glucuronopyranosyl-21,22-O-diangeloyl-3β, 15α, 16α, 21β, 22α, 28-hexahydroxyolean-12-ene.

## DETAILED DESCRIPTION OF THE INVENTION

Wenguanguo is a species of the sapindaceae family. Its scientific name is Xanthoceras sorbifolia Bunge. Wenguanguo is the common Chinese name; others are Wenguannguo, Wenguanmu, Wenguanhua, and Xilacedeng. This plant can grow up to 8 meters in height. It features odd pinnately compound leaf, eraceme with white flowers, capsules with thick and woody husks. Wenguanguo is grown in Liaoning, Jilin, Hebei, Shandong, Jiangsu, Henan, Shanxi, Shaanxi, Gansu, Ningxia and inner Mongolia, China. Its seeds are edible and have been used as a folk medicine to treat enuresis for centuries. Its branches and woods are also used as a folk medicine.

This invention provides the crude saponins from the husks or fruit-stem of Wenguanguo. The methods for preparing the crude saponins from husk or fruit-stem comprise the following steps: extracting Wenguanguo powder of the husks or fruit-stem with an organic solvent (ethanol, methanol and others) at ratio of 1:2 for 4-5 times, 20-35 hours for each time to form an organic extract; collect and reflux the organic extract 2-3 times at 80oC to form second extracts; resolve the second extracts in water to form an aqueous solution; extract the aqueous solution by n-butanol to form a n-butanol extracts; chromatograph the n-butanol extracts to form the crude saponins.

**The combined extract comprises saponins.**

This invention provides the crude saponins from the leaf of Wenguanguo and method for their preparation. The methods for preparing the crude saponins from the leaves comprise the following steps: extracting Wenguanguo powder of the leaves with an organic solvent (ethanol, methanol and others) at ratio of 1:2, 4-5 times, 20-35 hours each time to form an organic extract; collect and reflux the organic extract 2-3 times at 80oC to form a second extract; resolve the second extracts in water to form an aqueous solution; extract the aqueous solution by n-butanol to form a n-butanol extracts; chromatograph the n-butanol extracts to form the crude saponins.

**The crude extract comprises saponins.**

This invention provides the crude saponins from the branch and stem of Wenguanguo. The methods for preparing the crude saponins from the branches or stem comprise the following steps: extracting Wenguanguo powder of the branches or stem with an organic solvent (ethanol, methanol and others) at ratio of 1:2, 4-5 times, 20-35 hours each time to form an organic extract; collect and reflux the organic extract 2-3 times at 80oC to form second extracts; resolve the second extracts in water to form an aqueous solution; extract the aqueous solution by n-butanol to form a n-butanol extracts; chromatograph the n-butanol extracts to form the crude saponins.

**The crude extract comprises saponins.**

This invention provides the crude saponins from the kernel of Wenguanguo. The methods for preparing the crude saponins from the kernel comprise the following steps: removing oil by pressing the kernel to form kernel cakes; grinding and extracting the kernel cakes with n-hexane to from n-hexane extract; removing the n-hexane from the n-hexane extract and dry them to form the kernel powder; extracting the kernel powder with an organic solvent (ethanol, methanol and others) at ratio of 1:2, 4-5 times, 20-35 hours each time to form an organic extract; collect and reflux the organic extract for 2-3 times at 80o C to form second extracts; resolve the second extracts in water to form an aqueous solution; Extract the aqueous solution by n-butanol to form a n-butanol extracts; chromatograph the n-butanol extracts to form the crude saponins.

**The crude extracts comprise saponins.**

This invention provides the crude saponins from the root of Wenguanguo and method for their preparation. The methods for preparing the crude saponins from the root comprise the following steps: extracting Wenguanguo powder of the root with an organic solvent (ethanol, methanol and others) at ratio of 1:2, 4-5 times, 20-35 hours each time to form an organic extract; collect and reflux the organic extract 2-3 times at 80oC to form second extracts; resolve the second extracts in water to form an aqueous solution; extract the aqueous solution by n-butanol to form a n-butanol extracts; chromatograph the n-butanol extracts to form the crude saponins.

**The crude extracts contain saponins.**

This invention provides the crude saponins from the bark of Wenguanguo and method for their preparation. The methods for preparing the crude saponins from the bark comprise the following steps: extracting Wenguanguo powder of the bark with an organic solvent (ethanol, methanol and others) at a ratio of 1:2, 4-5 times, 20-35 hours each time to form an organic extract; collect and reflux the organic extract 2-3 times at 80oC to form second extracts; resolve the second extracts in water to form an aqueous solution; extract the aqueous solution by n-butanol to form a n-butanol extracts; chromatograph the n-butanol extracts to form the crude saponins.

**The crude extracts comprise saponins.**

This invention provides the medicines or health foods which further comprise Vitamin B, Vitamin D, Vitamin K, grape seed extract and other antioxidants, Cordyceps, or its extract, gingko, or its extract, Panax ginseng and P. quinquefolium or their extracts, Huangpi (Clausena lansium), or its extracts, Echinacea or its extract, St John's Wort (Hypericum perforatum), or its extract, Gegen (Pueraria lobata), or its extract, Tianma (Gastrodia elata), or its extract, Armillariella mellea, or its extract, Danshen (Salvia miltiorrhiza), or its extract, Sanqi (Panax notoginsen), or its extract, Monascus or Honqu (Red yeast rice), Huanqi (Hedysarum polybotrys), or its extract, D ihuang (Rehmannia glutinosa), or its extract, Danggui (Angelica sinensis), Yuanzhi (Polygala tenuifoila), or its extract, Lingzhi (Ganoderma spp.), or its extracts, Fuling (Poria cocos), or its extract, enokitake(Flammulina velutipes), or its extract, Gan Cao (Glycyrrhiza uralensis Fisch)or its extract, Huperzine A, Lacithin, Metrifonate, Nocetile, folic acid, amino acids, creatine, fiber supplement, or any combination thereof.

This invention provides the extract of wenguanguo against cancer growth. The cancer includes, but is not limited to bladder cancer, cervix cancer, prostate cancer, lung cancer, breast cancer, leukocytes cancer, colon cancer, liver cancer, bone cancer, brain cancer, and ovary cancer.

This invention provides a compound comprising a sugar, a triterpene or Sapogenin, and a side chain at Carbon 21 and 22 or Angeloyl groups. In an embodiment, the compound comprises two or more sugars.

This invention provides a compound comprising the following structure:

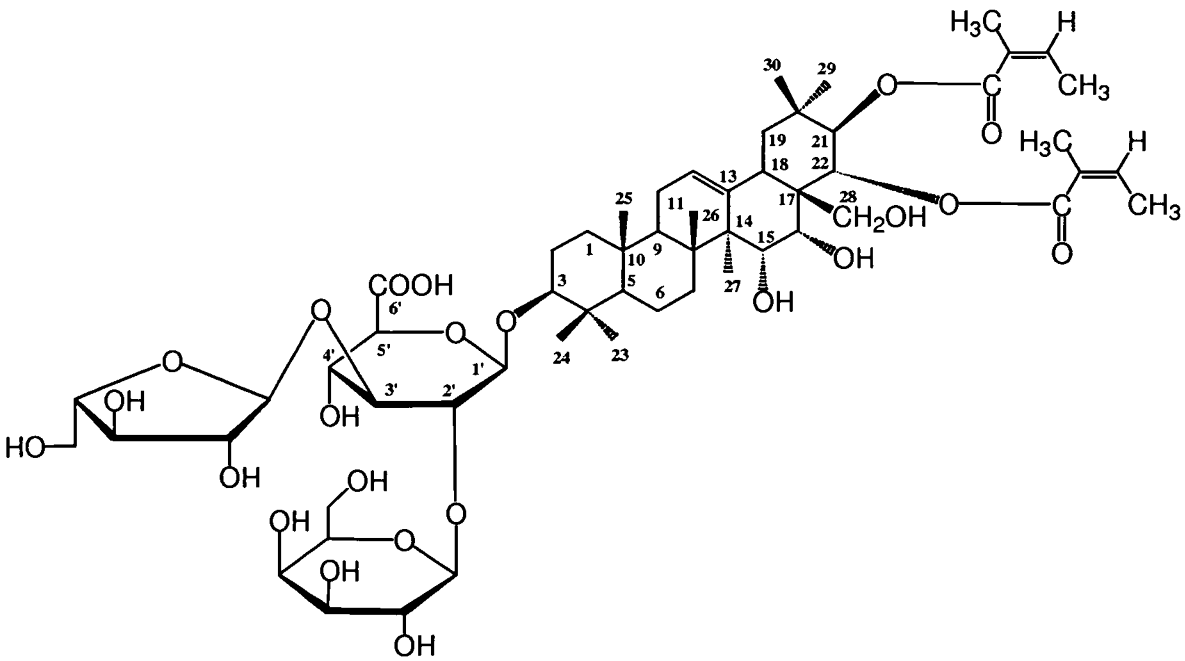

3-O-[β-D-galactopyranosyl(1→2)]-α-L-arabinofuranosyl(1→3)-β-D-glucuronopyranosyl-21,22-O-diangeloyl-3β, 15α, 16α, 21β, 22α, 28-hexahydroxyolean-12-ene, also known as Xanifolia Y In other embodiments, the structures of the compounds are as follows:

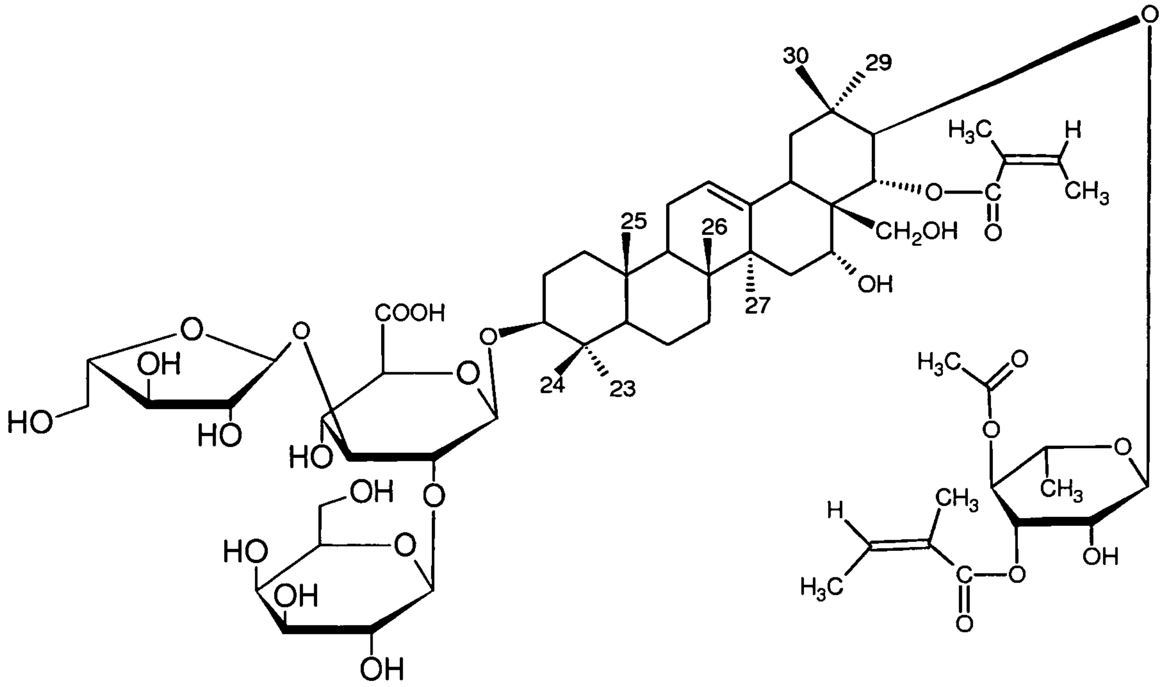

Structure 2

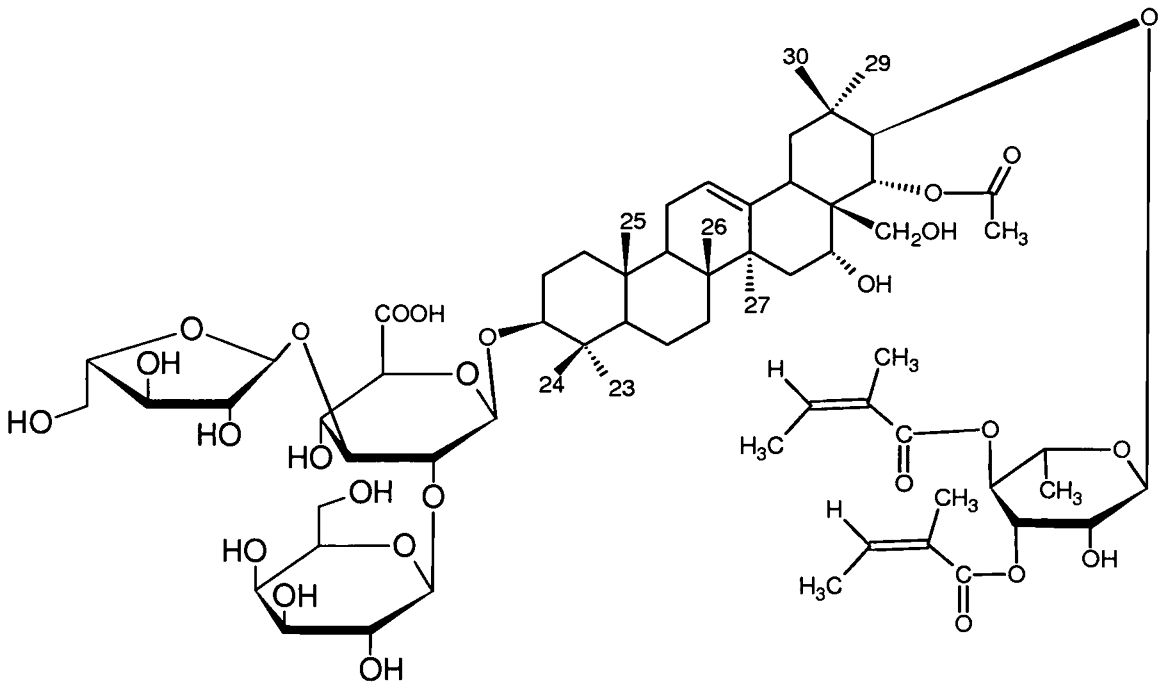

Structure 3

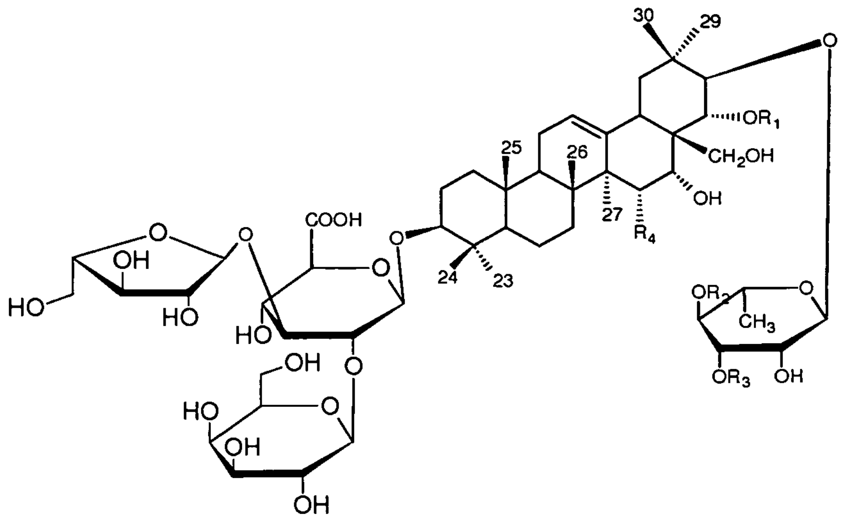

Wherein R1 =A or B or C; R2 =A or B or C; R3 =A or B or C; R4 =H or OH; A=angeloy group; B = acetyl group; C= Hydrogen

| | R1 | R2 | R3 |
|---|---|---|---|
| 1 | A | A | A |
| 2 | A | A | B |
| 3 | A | A | C |
| 4 | A | B | A |
| 5 | A | B | B |
| 6 | A | B | C |
| 7 | A | C | A |
| 8 | A | C | B |
| 9 | A | C | C |
| 10 | B | A | A |
| 11 | B | A | B |
| 12 | B | A | C |
| 13 | B | B | A |
| 14 | B | B | B |
| 15 | B | B | C |
| 16 | B | C | A |
| 17 | B | C | B |
| 18 | B | C | C |
| 19 | C | A | A |
| 20 | C | A | B |
| 21 | C | A | C |
| 22 | C | B | A |
| 23 | C | B | B |
| 24 | C | B | C |
| 25 | C | C | A |

(continued)

| | R1 | R2 | R3 |
|---|---|---|---|
| 26 | C | C | B |
| 27 | C | C | C |

Structure 4

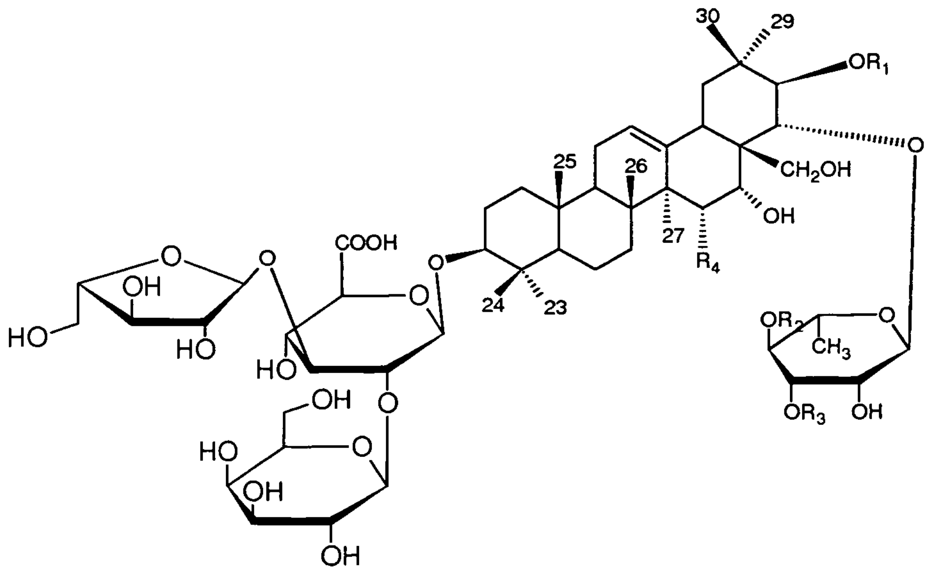

Wherein R1 =A or B or C; R2 =A or B or C; R3 =A or B or C; R4 =H or OH; A=angeloy group; B = acetyl group; C= Hydrogen;

| | R1 | R2 | R3 |
|---|---|---|---|
| 1 | A | A | A |
| 2 | A | A | B |
| 3 | A | A | C |
| 4 | A | B | A |
| 5 | A | B | B |
| 6 | A | B | C |
| 7 | A | C | A |
| 8 | A | C | B |
| 9 | A | C | C |
| 10 | B | A | A |
| 11 | B | A | B |
| 12 | B | A | C |
| 13 | B | B | A |
| 14 | B | B | B |
| 15 | B | B | C |

(continued)

| | R1 | R2 | R3 |
|---|---|---|---|
| 16 | B | C | A |
| 17 | B | C | B |
| 18 | B | C | C |
| 19 | C | A | A |
| 20 | C | A | B |
| 21 | C | A | C |
| 22 | C | B | A |
| 23 | C | B | B |
| 24 | C | B | C |
| 25 | C | C | A |
| 26 | C | C | B |
| 27 | C | C | C |

This invention provides a salt of the above-described compounds.

This invention provides a composition comprising the above-described compounds and a suitable carrier.

This invention provides a pharmaceutical composition comprising an effective amount of the above-described compounds and a pharmaceutically acceptable carrier.

This invention provides an anti-ovarian cancer and composition comprising the above-described composition.

The following methods and materials were used in the examples/experiments described below:

Cells. Human cancer cell lines were obtained from American Type Culture Collection: HTB-9 (bladder), HeLa-S3 (cervix), DU145 (prostate), H460 (lung), MCF-7 (breast), K562 (leukocytes), HCT116 (colon), HepG2 (liver), U2OS (bone), T98G (brain) and OVCAR-3 (ovary). Cells were grown in culture medium (HeLa-S3, DU145, MCF-7, Hep-G2 and T98G in MEN (Earle's salts); HTB-9, H460, K562, OVCAR-3 in RPMI-1640; HCT-116, U2OS in McCoy-5A) supplemented with 10% fetal calf serum, glutamine and antibiotics in a 5% CO2 humidified incubator at 37°C.

MTT Assay. The procedure for MTT assay followed the method described in (Carmichael et al., 1987) with only minor modifications. Cells were seeded into a 96-wells plate at concentrations of 10,000/well (HTB-9, HeLa, H460, HCT116, T98G, OVCAR-3), 15,000/well (DU145, MCF-7, HepG2, U2OS), or 40,000/well (K562), for 24 hours before drug-treatment. Cells were then exposed to drugs for 48 hours (72 hours for HepG2, U2OS, and 96 hours for MCF-7). After the drug-treatment, MTT (0.5 mg/ml) was added to cultures for an hour. The formation of formazan (product of the reduction of tetrazolium by viable cells) was dissolved with DMSO and the O.D. at 490nm was measured by an ELISA reader. The MTT level of cells before drug-treatment was also measured (TO). The % cell-growth (%G) is calculated as:

$$\%G = (TD-T0 / TC-T0) \times 100, \quad (1)$$

where TC or TD represent O.D. readings of control or drug-treated cells.

When T0 > TD, then the cytotoxicity (LC) expressed as % of the control is calculated as:

$$\%LC = (TD-T0 / T0) \times 100. \quad (2)$$

This invention provides the pathways interacted by compounds isolated from Xanthoceras Sorbifolia. In an embodiment, a compound has the formula C57H88O23 and the chemical name 3-O-[β-D-galactopyranosyl(1→2)]-α-L-arabinofuranosyl(1→3)-β-D-glucuronopyranosyl-21,22-O-diangeloyl-3β, 15α, 16α, 21β, 22α, 28-hexahydroxyolean-12-ene, and derivative compounds which are effective against cancer. They regulate the receptors or components of cells.

The compounds can be isolated from the plant called Xanthoceras Sorbifolia or can be synthesized.

The compound is:

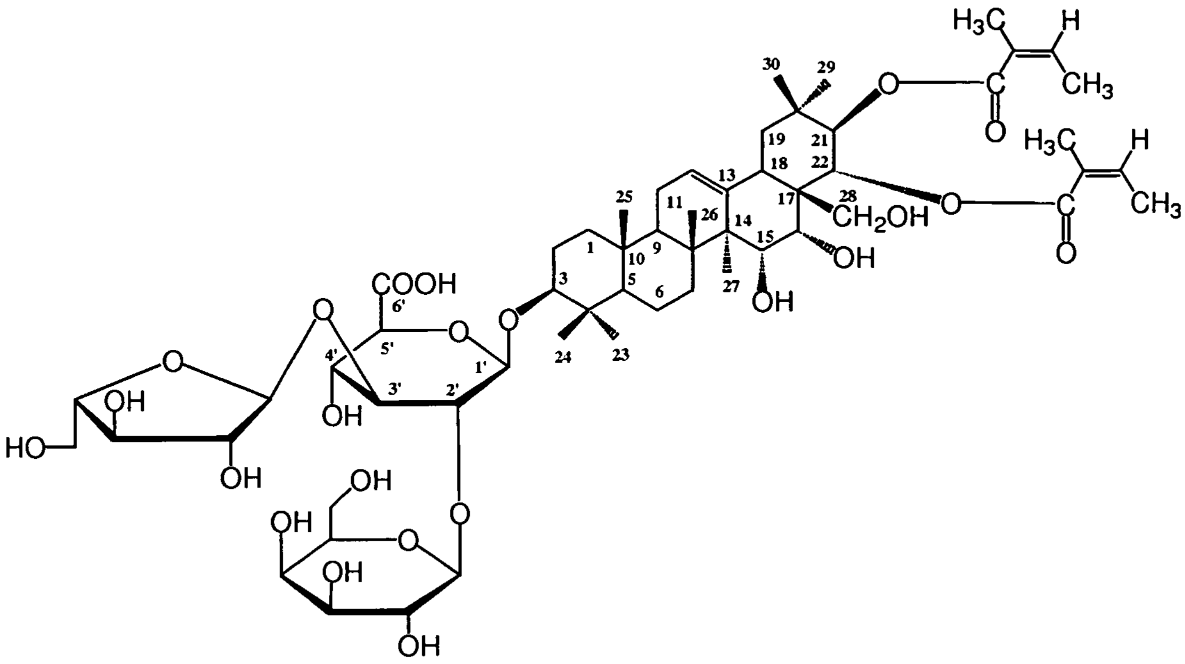

3-O-[β-D-galactopyranosyl(1→2)]-α-L-arabinofuranosyl(1→3)-β-D-glucuronopyranosyl-21,22-O-diangeloyl-3β, 15α, 16α, 21β, 22α, 28-hexahydroxyolean-12-ene

Structure 1

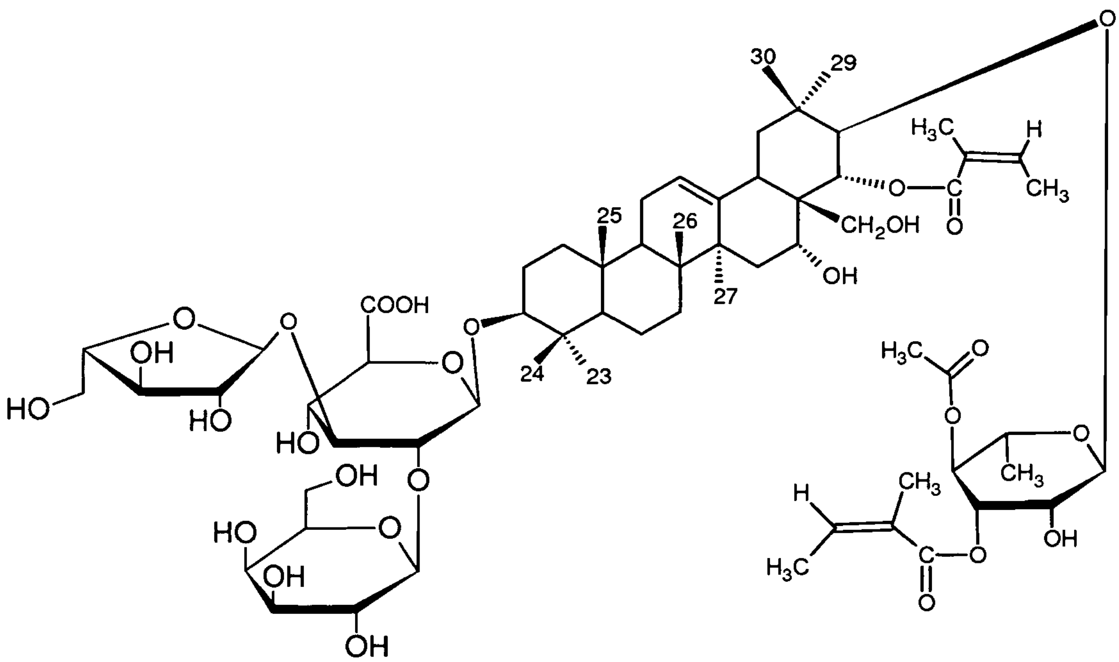

Structure 2

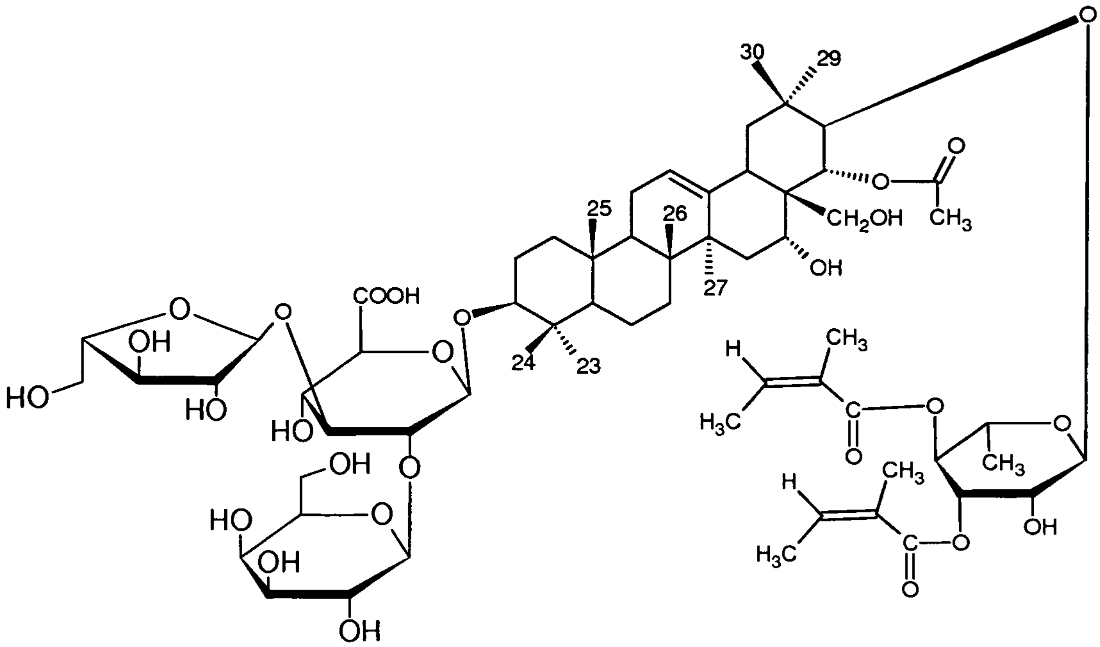

Structure 3

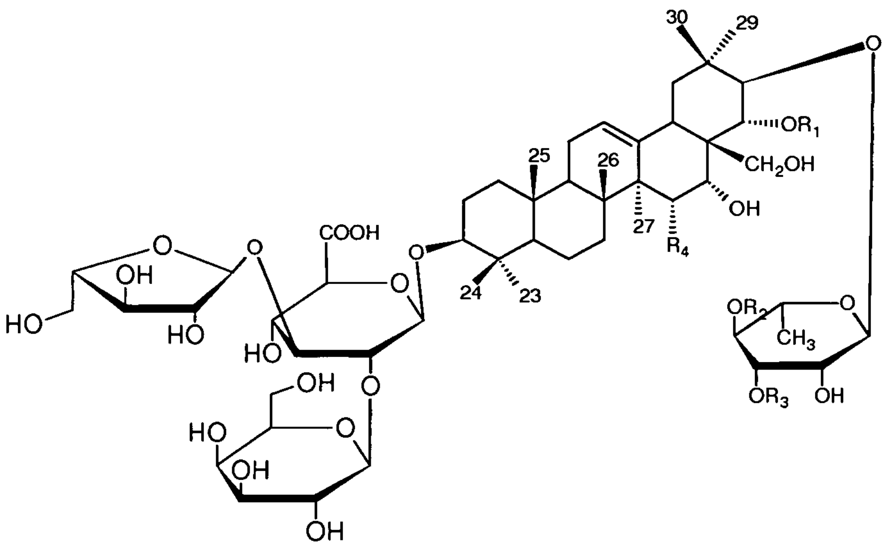

Wherein R1 =A or B or C; R2 =A or B or C; R3 =A or B or C; R4 =H or OH; A=angeloy group; B = acetyl group; C= Hydrogen

| | R1 | R2 | R3 |
|---|---|---|---|
| 1 | A | A | A |
| 2 | A | A | B |
| 3 | A | A | C |
| 4 | A | B | A |
| 5 | A | B | B |
| 6 | A | B | C |

(continued)

| | R1 | R2 | R3 |
|---|---|---|---|
| 7 | A | C | A |
| 8 | A | C | B |
| 9 | A | C | C |
| 10 | B | A | A |
| 11 | B | A | B |
| 12 | B | A | C |
| 13 | B | B | A |
| 14 | B | B | B |
| 15 | B | B | C |
| 16 | B | C | A |
| 17 | B | C | B |
| 18 | B | C | C |
| 19 | C | A | A |
| 20 | C | A | B |
| 21 | C | A | C |
| 22 | C | B | A |
| 23 | C | B | B |
| 24 | C | B | C |
| 25 | C | C | A |
| 26 | C | C | B |
| 27 | C | C | C |

Structure 4

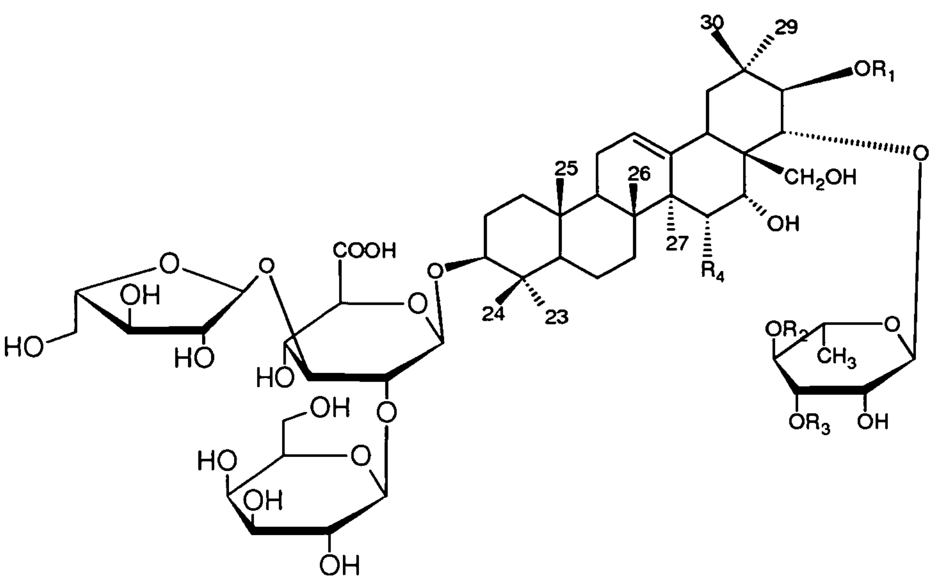

Wherein R1 =A or B or C; R2 =A or B or C; R3 =A or B or C; R4 =H or OH A=angeloy group; B = acetyl group; C= Hydrogen

| | R1 | R2 | R3 |
|---|---|---|---|
| 1 | A | A | A |
| 2 | A | A | B |
| 3 | A | A | C |
| 4 | A | B | A |
| 5 | A | B | B |
| 6 | A | B | C |
| 7 | A | C | A |
| 8 | A | C | B |
| 9 | A | C | C |
| 10 | B | A | A |
| 11 | B | A | B |
| 12 | B | A | C |
| 13 | B | B | A |
| 14 | B | B | B |
| 15 | B | B | C |
| 16 | B | C | A |
| 17 | B | C | B |
| 18 | B | C | C |
| 19 | C | A | A |
| 20 | C | A | B |
| 21 | C | A | C |
| 22 | C | B | A |
| 23 | C | B | B |
| 24 | C | B | C |
| 25 | C | C | A |
| 26 | C | C | B |
| 27 | C | C | C |

There are many components and pathways monitoring cell proliferation.
The Xanthoceras Sorbifolia compound or its derivatives work in the Wnt (Wingless-type MMTV integtation site family member) signaling pathway. The Wnt signaling pathway is evolutionarily conserved and controls many events during the embryogenesis. This pathway regulates cell morphology, proliferation, motility and as well as cell apoptosis. It also plays an important role during turmorigenesis. The Wnt pathway has also been observed as inappropriately activated in several different types of cancers in humans.
In the nucleus, the target genes for Wnt signaling are normally kept silent by an inhibitory complex of gene regulatory proteins, e.g. the Groucho corepressor protein bound to the gene regulatory protein LEF-I/TCF. In the absence of a Wnt signal some β-cartenin is bound to the cytosolic tail of cadherin proteins, and any cytosolic β-cartenin that becomes bound by the APC-axin-GSK-3β will trigger its ubiquitylation and degradation in proteasomes.The result is the decrease of intracellular amount of β-cartenin. However, when the Wnt binding to Frizzled (a seven transmembrane receptor)and LRP (Low density lipoprotein Receptor)activates Dishevelled (a cytoplasmic signaling protein) by a mechanism, this leads to the inactivation of GSK-β3 in the degradation complex by a mechanism which requires casein kinase I, as well as casein kinase II. The activity of the multiprotein complex of β-catenin-axin-adenomatous-polyposis coli (APC)-glycogen

synthase kinase (GSK)-3β, which targets β-catenin by phosphorylation for degradation by the proteasome, is then inhibited by Dsh/Dvl (Dishevelled, dsh homolog 1). This then inhibits priming of β-catenin, and indirectly prevents the GSK-3β phosphorylation of β-catenin. When stimulated by Wnt, Dvl recruits the GSK-3 binding protein, GBP, to the multiprotein complex of β-catenin-axin-adenomatous-polyposis coli (APC)-glycogen synthase kinase (GSK)-3β. GBP then titrates GSK-β from axin, and in this way, phosphorylation of β-catenin is inhibited. Then, axin is sequestrated by LRP at the cell membrane. The result of all of this is an accumulation of cytosolic β-catenin. In the nucleus, β-catenin binds to LEF-I/TCF, displaces Groucho, and acts a co-activator to stimulate the transcription of Wnt target genes.

Xanthoceras Sorbifolia compositions regulate the components related to Wnt pathways or its receptors, thereby stopping the proliferation of cancer cells.

The compound or its derivatives work in the Mitogens, Ras and a MAP (Microtubule associated protein) kinase pathway. Mitogens stimulate cell division. The binding of mitogens to cell-surface receptors leads to the activation of Ras and a MAP kinase cascade. One effect of this pathway is the increased production of the gene regulatory protein Myc. Myc increases the transcription of several genes, including the gene encoding cyclin D and a subunit of the SCF ubiquitin ligase. The resulting increase in $G_1$-Cdk and $G_1$/S-Cdk activities promotes Rb phosphyorylation and activation of the gene regulatory protein E2F, resulting in S-phase entry, in which $G_1$-Cdk activity initiates Rb phosphorylation, in turn inactivating Rb and freeing E2F to activate the transcription of S-phase genes including the genes for a $G_1$/S-cyclin(cyclin E) and S-cyclin(cyclin A). The resulting appearance of $G_1$/S-Cdk and S-Cdk further enhances Rb phosphorylation, forming a positive feedback loop, and the E2F acts back to stimulate the transcription of its own gene, forming another positive feedback loop. Myc may also promote E2F activity directly by stimulating the transcription of the E2F gene. The result is the increased transcription of genes entry into S phase. However if this pathway is overactive, it will cause cancer cell growth.

Compounds or compositions derived from the plant Xanthoceras Sorbifolia regulate the Ras-MAP kinase cascade so that the pathway is not overactive.

The compound or its derivatives work in Ras-dependent or Myc pathway. Sometimes the mutation of amino acid in Ras causes the protein to become permanently overactive, stimulating the Ras-dependent signal pathways overactive in absence of mitogenic stimulation. Similarly, mutations that cause an overexpression of Myc promote excessive cell growth, which in turn promotes the development of cancer.

Compounds or compositions derived from the plant Xanthoceras Sorbifolia regulate the components of the Ras-dependent or Myc pathway to make sure it is not overactive.

The compound or its derivatives reactivate the abnormal cell checkpoint mechanism. Inside the cell, there is a checkpoint mechanism which detects abnormal mitogenic stimulation and causes abnormally overactive cells to go into apoptosis. However this mechanism is not active in cancer cells due to mutations in the genes that encode essential components of the checkpoint responses. If the mutation happens in the checkpoint mechanism, the cancer cell will growth and divide endlessly. Compounds or compositions derived from the plant Xanthoceras Sorbifolia reactivate the checkpoint mechanism to stop the cancer cell growth.

The compound or its derivatives affect the extracellular growth signaling pathways. The extracellular growth factors that stimulate cell growth are bound to receptors on the cell surface and activate intracellular signaling pathways. It activates the enzyme PI3-kinase, which promotes protein synthesis, at least partly through the activation of eif4e and phosphorylated s6 kinase, resulting in increased mRNA translation and then a stimulation of cell growth.

Compounds or compositions derived from the plant Xanthoceras Sorbifolia regulate the components or receptor relate to extracellular growth. It binds the receptor of ovarian cancer cells so as to stop the cancer cell growth.

Compounds or compositions derived from the plant Xanthoceras Sorbifolia regulate the components relating to Ras and MAP Kinase, which ceases ovarian cancer cell growth.

The compound or its derivatives affect the intracellular mechanism. Cell division is also controlled by an intracellular mechanism that can limit cell proliferation. In normal cells, the Myc protein acts in the nucleus as a signal for cell proliferation. Large quantities of Myc can cause the cell to proliferate in excess and form a tumor.

Compounds or compositions derived from the plant Xanthoceras Sorbifolia regulate the components or receptor of the Myc cell's proliferation to stop the tumor cells from dividing.

The compound or its derivatives affect the TGF-alpha signaling pathway. TGF-alpha is produced by keratimcytes, macrophages, hepatocytes, and platelets. Its synthesis is stimulated by the infection by viruses. TGF-Alpha induces the long term proliferation of murine and chicken immature hematopoietic progenitor cell such as BFU-E without causing differentiation. It also induces the terminal differentiation of BFU-Ecell into erythrocytes. TGF-Alpha stimulates the proliferation of cultured endothelial cells. It plays an importance role in the vascularisation of tumor tissues.

Compounds or compositions derived from the plant Xanthoceras Sorbifolia regulate the components or receptor of TGF-alpha to suppress ovarian cancer and bladder cancer cell growth.

The compound or its deviative compounds affect the TGF-beta signaling pathway. TGF-beta regulates growth and proliferation of cells, blocking growth of many cell types. There are two TGF-beta receptors: Type 1 and Type 2. They are serine-threonine kinases that signal through the SMAD (Protein named after the first two identified, Sma in C.

elegans and Mad in Drosophila) family of transcriptional regulators. The TGF-beta pathway and mutation in SMADs are associated with cancer in humans.

Compounds or compositions derived from the plant Xanthoceras Sorbifolia regulate the components or receptor of TGF-beta to suppress the ovarian cancer and bladder cancer cell growth. The compound or its derivaties reactivate the cell functions which are damaged by DNA viruses. DNA tumor viruses cause cancer by interfering with cell cycle control Rb protein and the p53 protein. Mutation in p53 gene will allow cancer cells to survive and proliferate despite DNA damage. The papillomanius uses the proteins E6 and E7 to sequence the p53 and Rb respectively. This action activates mutated cells, allowing them to survive and then divide and accumulate. The accumulation of damaged cells can lead to cancer.

Compounds or compositions derived from the plant Xanthoceras Sorbifolia regulate the proteins E6 and E7 and release the proteins Rb and p53, which will prevent abnormal cells from dividing. It also regulates or reacts with the protein, causing the cancer cells to die.

The compound or its derivatives affect the p53 signaling pathway. p53 helps multi-cellular organisms cope safely with DNA damage and other stressful cellular events, stopping cell proliferation in circumstances where it would be dangerous. Cancer cells tend to contain large quantities of mutant p53 protein, suggesting that the genetic accidents they undergo or the stresses of growth in an inappropriate environment created the signals that normally activate the p53 protein. Thus, the loss of p53 activity can be extremely dangerous in relation to cancer because it allows mutant cells to continue through the cell cycle. It also allows them to escape apoptosis. So, if their DNA is damaged, some cells will die but the cells which survive will carry on dividing without pausing to repair the damage. This may cause the cells to die, or they could survive and proliferate with a corrupted genome, which could lead to loss of both tumor suppressor genes and the activation of oncogenes, for example by gene amplification. Gene amplification could enable cells to develop resistance against therapeutic drugs.

Compounds or compositions derived from the plant Xanthoceras Sorbifolia regulate the components and receptor of the p53 pathway, which stops the cancer cells from dividing.

The compound or its derivatives affect the cell suicide signaling pathway. All cells with a nucleus contain various inactive procaspases, awaiting a signal before destroying the cell. Each suicide protease is made as an inactive proenzyme called procaspase. It is usually activated by proteolytic cleavage by another member of the caspase family. Two of the cleaved fragments come together to form the active part of the caspase, and the active enzyme is thought to be a tetramer of two of these two parts. Each activated caspase molecule can cleave many procaspase molecules, which in turn activates more molecules. Through a chain reaction or cascade, this leads to the explosive action of a large number of procaspase molecules. Then, some of the activated procaspases cleave a number of key proteins in the cell, including specific cytosolic proteins and nuclear-lamins leading to the controlled death of the cell.

Activating the death receptor on the outside of the cell can also trigger inactive procaspases. For example, killer lymphocytes, can cause apoptosis by producing the protein Fas on the surface of the targeted cell. These clusters of Fas protein then recruit intracellular adaptor proteins that bind and aggregate procaspase-8 molecules. These then cleave and activate one another. The activated caspase-8 molecules then activate downstream procaspases to induce apoptosis. However in cancer cells, the signal to destroy the cell is blocked, due to gene mutation. This means that the cancer cells continue to divide, thereby causing a tumor.

Compounds or compositions derived from the plant Xanthoceras Sorbifolia unblock the suicide signals, allowing cancer cells to destroy themselves.

This invention provides a method for inhibiting tumor cell growth comprising contacting an amount of the compound, wherein R1, R2, R3, R4 are short aliphatic chain and R5 contains an oxyl group; and a pharmaceutically acceptable carrier effective to inhibit growth of said tumor cells.

This invention provides the above method, wherein R1=R2=R3=R4=CH3 and R5=OH.

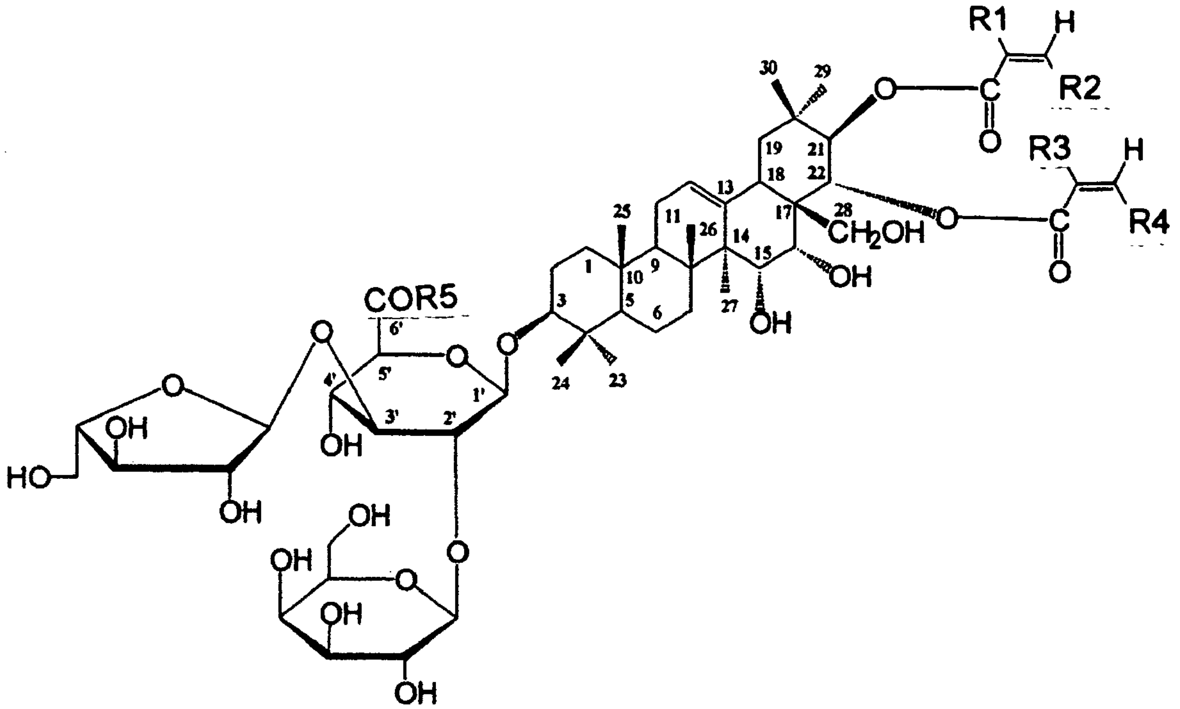

STRUCTURE 1

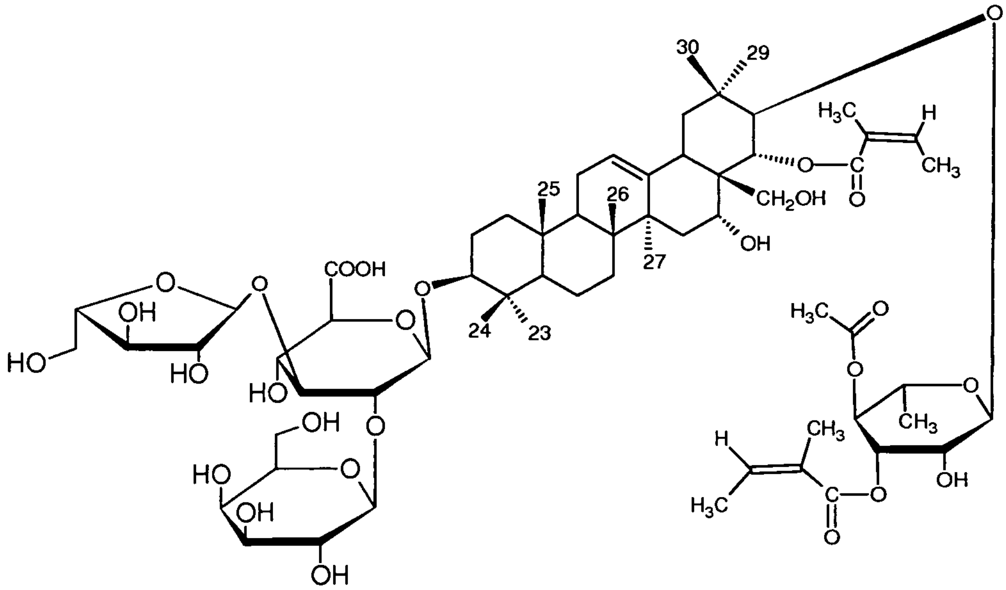

Structure 2

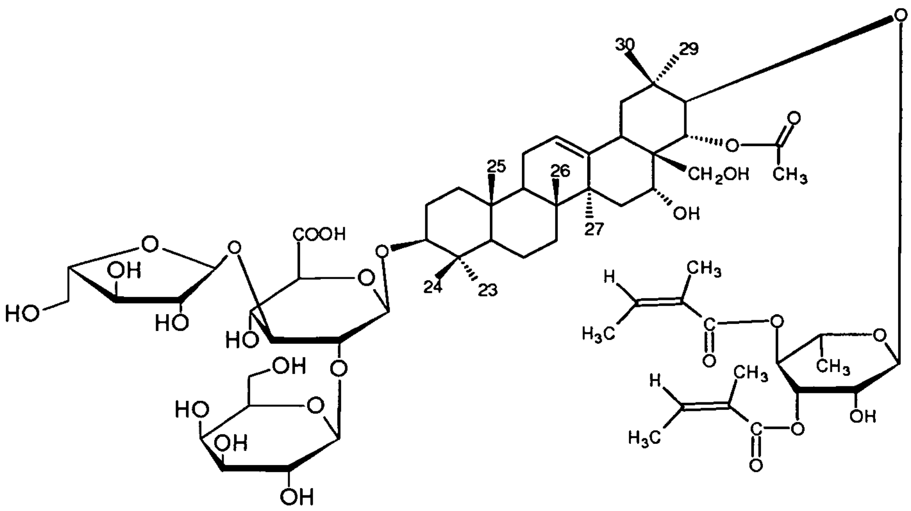

Structure 3

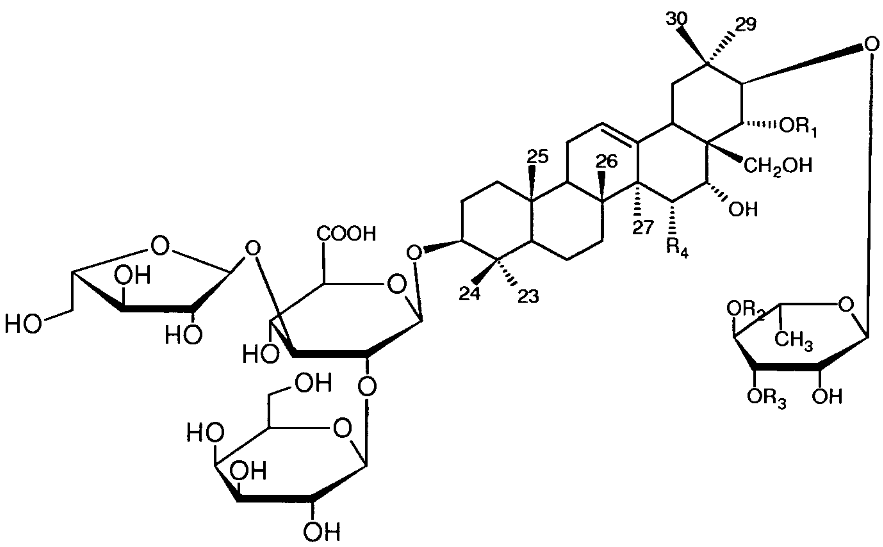

Wherein R1 =A or B or C; R2 =A or B or C; R3 =A or B or C; R4 =H or OH; A=angeloyl group; B = acetyl group; C= Hydrogen

| | R1 | R2 | R3 |
|---|---|---|---|
| 1 | A | A | A |
| 2 | A | A | B |
| 3 | A | A | C |
| 4 | A | B | A |
| 5 | A | B | B |
| 6 | A | B | C |
| 7 | A | C | A |
| 8 | A | C | B |

(continued)

| | R1 | R2 | R3 |
|---|---|---|---|
| 9 | A | C | C |
| 10 | B | A | A |
| 11 | B | A | B |
| 12 | B | A | C |
| 13 | B | B | A |
| 14 | B | B | B |
| 15 | B | B | C |
| 16 | B | C | A |
| 17 | B | C | B |
| 18 | B | C | C |
| 19 | C | A | A |
| 20 | C | A | B |
| 21 | C | A | C |
| 22 | C | B | A |
| 23 | C | B | B |
| 24 | C | B | C |
| 25 | C | C | A |
| 26 | C | C | B |
| 27 | C | C | C |

;

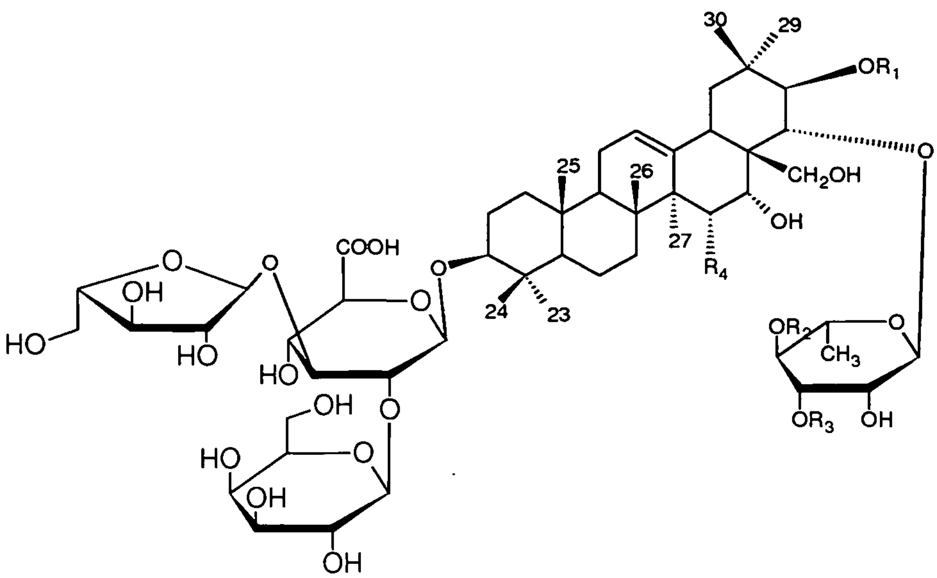

Wherein R1 =A or B or C; R2 =A or B or C; R3 =A or B or C; R4 =H or OH A=angeloyl group; B = acetyl group; C= Hydrogen

| | R1 | R2 | R3 |
|---|---|---|---|
| 1 | A | A | A |
| 2 | A | A | B |
| 3 | A | A | C |
| 4 | A | B | A |
| 5 | A | B | B |
| 6 | A | B | C |
| 7 | A | C | A |
| 8 | A | C | B |
| 9 | A | C | C |
| 10 | B | A | A |
| 11 | B | A | B |
| 12 | B | A | C |
| 13 | B | B | A |
| 14 | B | B | B |
| 15 | B | B | C |
| 16 | B | C | A |
| 17 | B | C | B |
| 18 | B | C | C |
| 19 | C | A | A |
| 20 | C | A | B |
| 21 | C | A | C |
| 22 | C | B | A |
| 23 | C | B | B |
| 24 | C | B | C |
| 25 | C | C | A |
| 26 | C | C | B |
| 27 | C | C | C |

This invention provides a method for inhibiting tumor cell growth comprising contacting an amount of the compound comprising: a sugar; a triterpene or Sapogenin; side chain at Carbon 21 and 22 or Angeloyl groups, operatively linked form the compound; and a pharmaceutically acceptable carrier.

This invention provides a method for inhibiting tumor cell growth in a subject comprising administering to the subject, wherein R1, R2, R3, R4 are short aliphatic chain and R5 contains an oxyl group; effective to inhibit growth of said tumor cells and a pharmaceutically acceptable carrier.

This invention provides a method, wherein R1=R2=R3=R4=CH3 and R5 contains an oxyl bond.

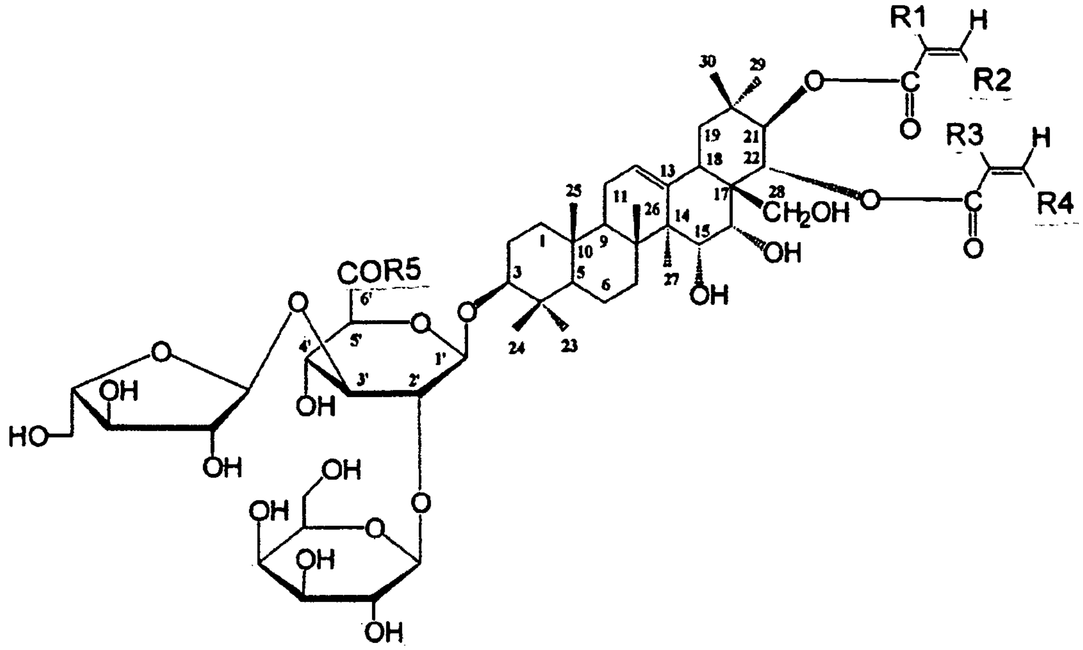

Structure 1

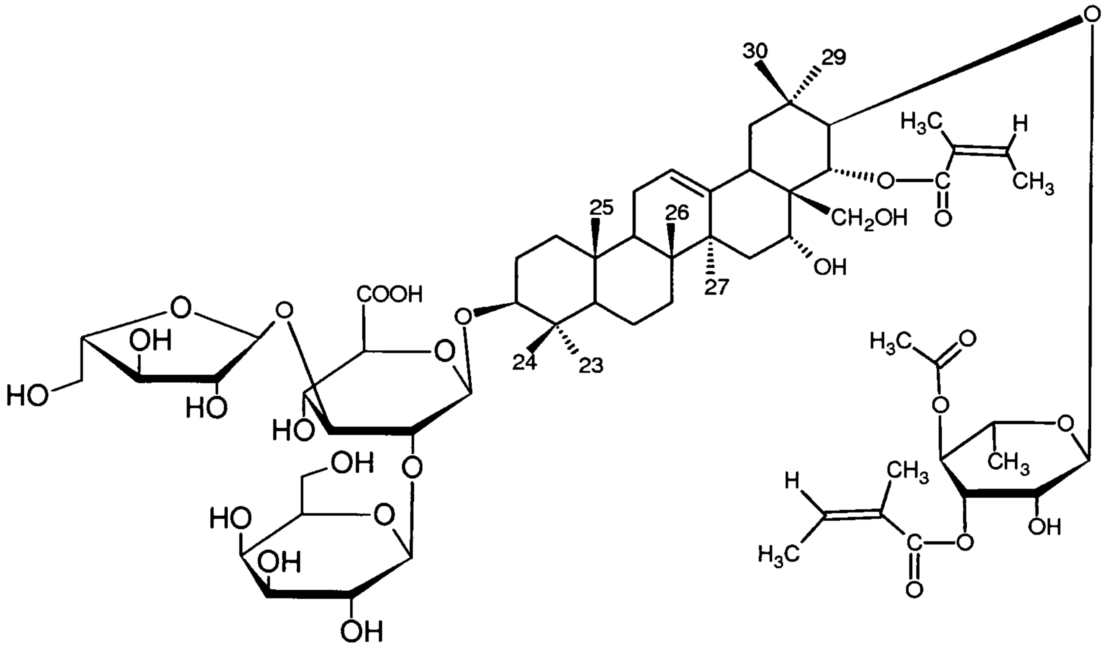

Structure 2

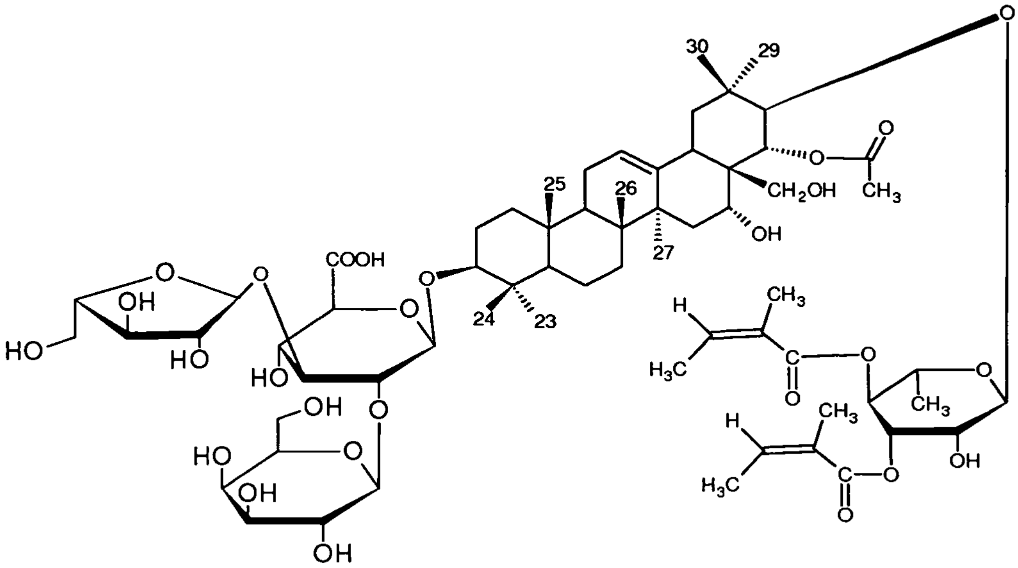

Structure 3

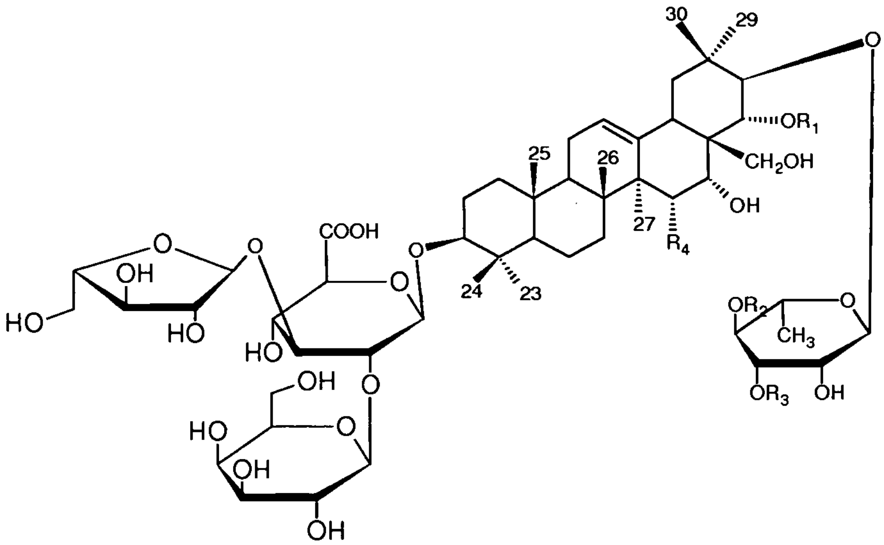

Wherein R1 =A or B or C; R2 =A or B or C; R3 =A or B or C; R4 =H or OH; A=angeloyl group; B = acetyl group; C= Hydrogen

| | R1 | R2 | R3 |
|---|---|---|---|
| 1 | A | A | A |
| 2 | A | A | B |
| 3 | A | A | C |
| 4 | A | B | A |
| 5 | A | B | B |
| 6 | A | B | C |
| 7 | A | C | A |

(continued)

| | R1 | R2 | R3 |
|---|---|---|---|
| 8 | A | C | B |
| 9 | A | C | C |
| 10 | B | A | A |
| 11 | B | A | B |
| 12 | B | A | C |
| 13 | B | B | A |
| 14 | B | B | B |
| 15 | B | B | C |
| 16 | B | C | A |
| 17 | B | C | B |
| 18 | B | C | C |
| 19 | C | A | A |
| 20 | C | A | B |
| 21 | C | A | C |
| 22 | C | B | A |
| 23 | C | B | B |
| 24 | C | B | C |
| 25 | C | C | A |
| 26 | C | C | B |
| 27 | C | C | C |

;

Structure 4

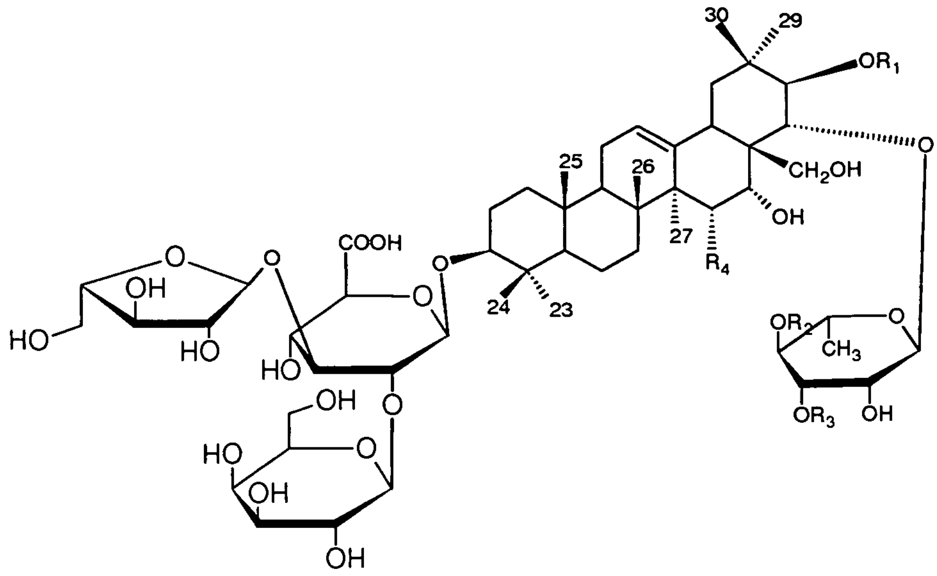

Wherein R 1 =A or B or C; R2 =A or B or C; R3 =A or B or C; R4 =H or OH A=angeloyl group; B = acetyl group; C= Hydrogen

| | R1 | R2 | R3 |
|---|---|---|---|
| 1 | A | A | A |
| 2 | A | A | B |
| 3 | A | A | C |
| 4 | A | B | A |
| 5 | A | B | B |
| 6 | A | B | C |
| 7 | A | C | A |
| 8 | A | C | B |
| 9 | A | C | C |
| 10 | B | A | A |
| 11 | B | A | B |
| 12 | B | A | C |
| 13 | B | B | A |
| 14 | B | B | B |
| 15 | B | B | C |
| 16 | B | C | A |
| 17 | B | C | B |
| 18 | B | C | C |
| 19 | C | A | A |
| 20 | C | A | B |
| 21 | C | A | C |
| 22 | C | B | A |
| 23 | C | B | B |
| 24 | C | B | C |

(continued)

| | R1 | R2 | R3 |
|---|---|---|---|
| 25 | C | C | A |
| 26 | C | C | B |
| 27 | C | C | C |

This invention provides a method for inhibiting tumor cell growth in a subject comprising administering to the subject a compound comprising: a sugar; a triterpene or Sapogenin; side chain at Carbon 21 and 22 or Angeloyl groups, operatively linked form the compound; and a pharmaceutically acceptable carrier.

This invention will be better understood from the examples which follow. However, one skilled in the art will readily appreciate that the specific methods and results discussed are merely illustrative of the invention as described more fully in the claims which follow thereafter.

**EXPERIMENTAL DETAILS**

**Experiment 1:**

**Herb Extraction**

(a) extracting Xanthoceras Sorbifolia powder of husk or branch or stem or leave or kernel or root or bark with organic solvent at ratio of 1:2 for 4-5 times for 20-35 hours for each time to form an organic extract; (b) collecting the organic extract; (c) refluxing the organic extract for 2-3 times at 80°C to form second extracts; (d) removing the organic solvent from the second extract; and (e) Drying and sterilizing the extract to form a Xanthoceras Sorbifolia extract powder.

**Experiment 2:**

**Analysis of Xanthoceras Sorbifolia extract components by HPLC chromatography**

**Methods and Materials**

HPLC

A C-18 reverse phase μbondapak column (Water P/N 27324) was equilibrated with 10% acetonitrile, 0.005% Trifluoroacetic acid (equilibration solution). An extract of Xanthoceras Sorbifolia was dissolved in equilibration solution (1 mg/ml) before being applied onto the column. 20 ug of samples was applied into column. Elution conditions: Fractions were eluted (flow rate 0.5 ml/min.) with acetonitrile (concentration gradient from 10% to 80% in 70 min) and then remains at 80% for 10 min (70-80 min). The acetonitrile then dropped to 10% (80-85 min) and remained at 10% for 25 min (85-110 min). The fractions were monitored at 207 nm with a chart speed 0.25 cm/min and OD full scale of 0.128.
Instruments: Waters Model 510 Solvent Delivery System; Waters 484 tunable Absorbance Detector; Waters 745/745B Data Module

Absorbance analysis

The absorption profile of Xanthoceras Sorbifolia extract at various wavelength was determined. An extract of Xanthoceras Sorbifolia was dissolved in 10% acetonitrile/TFA and scanned at 200-700 nm with a spectrophotometer [Spectronic Ins. Model Gene Sys2].

Results

HPLC

About 60-70 peaks can be accounted for in the profile. Among them four are major peaks, 10 are medium size and the rest are small fractions. The major peaks are labeled with a to z following increased concentration of acetonitrile elution. See Figure 7 and Figure 8A-B.

Absorption maximum

Three absorption maximum were identified for Xanthoceras Sorbifolia plant extract; 207nm, 278nm and 500nm. See Figure 9.

**Experiment 3:**

**Screening of cytotoxicity of Xanthoceras Sorbifolia extract toward cancer cells derived from different human organs by MTT assay**

**Methods and Materials**

Cells

Human cancer cell lines were obtained from American Type Culture Collection: HTB-9 (bladder), HeLa-S3 (cervix), DU145 (prostate), H460 (lung), MCF-7 (breast), K562 (leukocytes), HCT116 (colon), HepG2 (liver), U2OS (bone), T98G (brain) and OVCAR-3 (ovary). Cells were grown in culture medium (HeLa-S3, DU145, MCF-7, Hep-G2 and T98G in MEN (Earle's salts); HTB-9, H460, K562, OVCAR-3 in RPMI-1640; HCT-116, U2OS in McCoy-5A) supplemented with 10% fetal calf serum, glutamine and antibiotics in a 5% CO2 humidified incubator at 37°C.

MTT assay

The procedure for MTT assay followed the method described in (Carmichael et al., 1987) with only minor modifications. Cells were seeded into a 96-wells plate at concentrations of 10,000/well (HTB-9, HeLa, H460, HCT116, T98G, OVCAR-3), 15,000/well (DU145, MCF-7, HepG2, U2OS), or 40, 000/well (K562), for 24 hours before drug-treatment. Cells were then exposed to drugs for 48 hours (72 hours for HepG2, U2OS, and 96 hours for MCF-7). After the drug-treatment, MTT (0.5 mg/ml) was added to cultures for an hour. The formation of formazan (product of the reduction of tetrazolium by viable cells) was dissolved with DMSO and the O.D. at 490nm was measured by an ELISA reader [Dynatech. Model MR700]. The MTT level of cells before drug-treatment was also measured (TO). The % cell-growth (%G) is calculated as:

$$\%G = (TD-T0 / TC-T0) \times 100 \qquad (1)$$

where TC or TD represent O.D. readings of control or drug-treated cells.

When T0 > TD, then the cytotoxicity (LC) expressed as % of the control is calculated as:

$$\%LC = (TD-T0 / T0) \times 100.$$

Results

Among the 10 cell lines studies, their sensitivity toward Xanthoceras Sorbifolia extract can be divided into four groups (most sensitive: Ovary. Sensitive: bladder, bone, prostate, and leukocyte, marginal sensitive: liver, breast, and brain; and lease sensitive: colon, cervix, and lung) (Figure 10). Their IC50 values are listed (Table 3.1).

**Table 3.1. IC50 value of Xanthoceras Sorbifolia extract determined in different cancer cells**

| Cancer cells from different organs | IC50 determined by MTT assay (ug/ml) |
|---|---|
| Ovary (most sensitive) | 15-15 |
| Bladder (sensitive) | 45-50 |
| Bone | 40-55 |
| Prostate | 40-50 |
| Leukocyte | 45-50 |
| Liver (marginal sensitive) | 45-65 |

(continued)

| Cancer cells from different organs | IC50 determined by MTT assay (ug/ml) |
|---|---|
| Breast | 65 |
| Brain | 70-85 |
| Colon (least sensitive) | 90 |
| Cervix | 115 |
| Lung | 110 |

Among these cell line studied, it was found that low concentrations of the Xanthoceras Sorbifolia plant extract stimulate cell growth of bladder, bone and lung cells (Figures 1 to 6).

The pure fraction Y lost the growth stimulating activity but maintained the inhibition activity (Figure 10A compare with

Figure 12 and Figure 14). Preliminary results (Figure 12) indicate that the growth components resided in FPLC fractions 610 and 1116.

**Experiment 4:**

**Purification of active Xanthoceras Sorbifolia extract**

Fractionation of Xanthoceras Sorbifolia extracts components with FPLC

Methods

Column: Octadecyl functionalized silica gel; column dimension: 2cm x 28cm; equilibrated with 10% acetonitrile - 0.005% TFA. Sample loading: 1-2 ml, concentration: 100mg/ml in 10% acetonitrile/TFA.

Gradient elution: 10-80 % acetonitrile in a total volume of 500 ml.

Monitor absorption wavelength: at 254nm.

Fraction Collector: 5 ml/fractions (collect from 10% to 72% acetonitrile, total 90 fractions)

Instrument: AKTA-FPLC, P920 pump; Monitor UPC-900; Frac-900.

Results

The elution profile shows 4-5 broad fractions (Figure 11). These fractions were analyzed with HPLC. Specific components (a-z) are then keyed back in the FPLC profile and fractions.

FPLC fractions are grouped into 7 pools and analyzed for cell growth activity inbladder cells with MTT assay. It was found only one pool (#5962) contains inhibition activity (figure 12). Fractionation of #5962 with FPLC - C18 open column with 64% acetonitrile isocratic elution

Methods

Column: Octadecyl-functionalized silica gel; 50 ml; 2cm x 28cm; equilibrated with 64% acetonitrile - 0.005% TFA.
Sample loading: 0.2 ml, with concentration: 1-2 mg/ml in 65% acetonitrile/TFA.
Elution: 64% acetonitrile isocratic.
Monitor absorption wavelength: at 254nm.
Fraction Collector: 1 ml fraction (collect the first 90 fractions)
Instrument: AKTA-FPLC, P920 pump; Monitor UPC-900; Frac-900.

Results

Fraction No. 5962 was further purified with an open ODS-C18 column with isocratic 64% acetonitrile elution.

Two major fractions (X and Y) were collected (Figure 13) (PP-iso80-5). MTT assay showed that only the Y fraction has the inhibition activity (Figure 14).

**Analysis of Fraction Y with HPLC**

Methods

Column: Waters μbondapak C18 (3.9 x 300 nm).
Elution: 35% or 45% isocratic elution.
Flow rate: 0.5 ml/min; 207 nm; attenuation 0.128; chart speed: 0.25 cm/mim.

Results

On 35% isocratic analysis, the Y fraction contains 4-5 components (Y0, Y1, Y2, Y3, and Y4). Y3 and Y4 being the major components. Y1 and Y2 group together and Y3 and Y4 group together (Figure 15)(PP-HPLC-Y-iso35).
On 45% isocratic analysis, Y1, Y2 peaks merge into one peak, and Y3, Y4 merge into one peak (Figure 16). There may be a Y5 component after the Y3/4.
Final isolation of active Y component with preparative HPLC

Methods

Column: A preparative HPLC column (Waters Delta Pak C18-300A) ;
Elution: 45% acetonitrile isocratic with flow rate of 1 ml/min. Monitor at 207nm;

Peak fractions were collected and lyophilized.

Results Under these conditions:

Y1 and Y2 are well separated from each other and they are collected individually. Y3/4 and Y5 are not separated (Figure 17) (PP-HPLC-Y-iso45). Further analysis of the ascending and the descending portion of Y3/4 peak materials with NMR analysis showed a same profile which indicated that Y3/4 is a homogenous component (Figure 18) (PP-3-4 proton NMR). Therefore the fraction is designated as Y fraction. Rerun of the collected Y-fraction showed a single peak in HPLC with a C18 reverse phase column (Figure 19) (PP-HPLC-pure-Y).
Appearance and solubility. The pure Y is amorphous white powder, soluble in aqueous alcohol (methanol, ethanol), 50% acetonitrile and 100% pyridine.

MTT analysis of Y fraction indicated that:

(a) it has activity against ovarian cancer cells (OCAR-3) with IC50 of 1 ug/ml) which is 10-15 times more potent than the unpurified material (Figure 20a and 20b); and (b) it maintains its selectivity against ovarian cancer cells versus cervical cancer cells (HeLa)(Figure 21)(compare with HeLa cells not so active).

**Experiment 5**

Determination of the chemical structure of active Xanthoceras Sorbifolia extract

**Methods**

***NMR analysis***

The pure Y fraction of Xanthoceras Sorbifolia extract and other samples are subjected NMR analysis. Samples were dissolved in pyridine-D5 with 0.05% v/v TMS. All NMR spectra were acquired using a Bruker Avance 600 MHz

NMR spectrometer with a QXI probe ($^1H/^{13}C/^{15}N/^{31}P$) at 298 K. The numbers of scans for 1D $^1H$ spectra were 16 to 128, depending on the sample concentration. 2D HMQC spectra were recorded with spectral widths of 6000 × 24,000 Hz and data points of 2024 × 256 for $t_2$ and $t_1$ dimensions, respectively. The numbers of scans were 4 to 128. 2D HMBC were acquired with spectral widths of 6000 × 30, 000 Hz and data points of 2024 × 512 for $t_2$ and $t_1$ dimensions, respectively. The numbers of scans were 64. The 2D data were zero-filled in t1 dimension to double the data points, multiplied by cosine-square-bell window functions in both t1 and t2 dimensions, and Fourier-transformed using software XWIN-NMR. The final real matrix sizes of these 2D spectra are 2048×256 and 2048×512 data points (F2×F1) for HMQC and HMBC, respectively.

***Mass spectral analysis***

The mass of samples was analyzed by (A) MALDI-TOF Mass Spectrometry and by (B) ESI-MS Mass spectrometry.

(A) Samples for MALDI-TOF were first dissolved in acetonitrile, then mixed with the matrix CHCA (Alpha-cyano-4-hydroxycinnamic acid, 10mg CHCA/mL in 50:50 water/acetonitrile and 0.1% TFA in final concentration). The sample was dissolved completely in acetonitrile and stay dissolved after mixing with the matrix. The molecular weight was determined by the high resolution mass spectroscope analysis with standards.

(B) For ESI, the sample was analyzed with LCQ DECA XP Plus machine made by Thermo Finnigan. It is ionized with ESI source and the solvent for the compound is acetonitrile.

Results

The profile of the proton NMR is presented in Figure 22. (Proton-NMR-7-20) The chemical shift of the proton NMR of Y is listed in Table 5.1 below.

**Table 5.1. Chemical Shift of the Proton NMR of Y Fraction of Xanthoceras Sorbifolia Extract**

| # | ADDRESS | FREQUENCY | | INTENSITY |
|---|---|---|---|---|
| | | [Hz] | [PPM] | |
| 1 | 5031.9 | 5309.524 | 8.8468 | 0.46 |
| 2 | 5430.0 | 5222.264 | 8.7015 | 134.66 |
| 3 | 5840.9 | 5132.173 | 8.5513 | 0.48 |
| 4 | 8204.9 | 4613.901 | 7.6878 | 0.26 |
| 5 | 8568.1 | 4534.263 | 7.5551 | 88.28 |
| 6 | 8943.7 | 4451.919 | 7.4179 | 0.33 |
| 7 | 9209.7 | 4393.602 | 7.3207 | 0.80 |
| 8 | 9575.2 | 4313.488 | 7.1872 | 180.00 |
| 9 | 9952.1 | 4230.846 | 7.0495 | 0.87 |
| 10 | 10277.2 | 4159.585 | 6.9308 | 1.02 |
| 11 | 10886.8 | 4025.922 | 6.7081 | 5.68 |
| 12 | 10933.6 | 4015.677 | 6.6910 | 6.19 |
| 13 | 11939.8 | 3795.084 | 6.3235 | 6.26 |
| 14 | 11986.7 | 3784.803 | 6.3063 | 5.85 |
| 15 | 12576.0 | 3655.587 | 6.0910 | 1.47 |
| 16 | 12728.0 | 3622.280 | 6.0355 | 6.92 |
| 17 | 12880.5 | 3588.844 | 5.9798 | 1.87 |
| 18 | 12914.2 | 3581.450 | 5.9675 | 4.48 |
| 19 | 12946.5 | 3574.366 | 5.9557 | 4.59 |
| 20 | 12979.5 | 3567.130 | 5.9436 | 2.00 |
| 21 | 13382.6 | 3478.754 | 5.7964 | 2.41 |
| 22 | 13415.6 | 3471.539 | 5.7844 | 5.03 |
| 23 | 13447.3 | 3464.568 | 5.7727 | 5.15 |
| 24 | 13479.1 | 3457.598 | 5.7611 | 2.66 |
| 25 | 14218.8 | 3295.432 | 5.4909 | 14.00 |
| 26 | 14655.9 | 3199.603 | 5.3312 | 28.97 |

(continued)

| # | ADDRESS | FREQUENCY [Hz] | [PPM] | INTENSITY |
|---|---|---|---|---|
| 27 | 14691.2 | 3191.875 | 5.3184 | 27.63 |
| 28 | 15715.3 | 2967.359 | 4.9443 | 5.64 |
| 29 | 15826.3 | 2943.028 | 4.9037 | 4.82 |
| 30 | 15860.6 | 2935.504 | 4.8912 | 5.15 |
| 31 | 16047.0 | 2894.632 | 4.8231 | 4.20 |
| 32 | 16149.6 | 2872.131 | 4.7856 | 3.79 |
| 33 | 16171.9 | 2867.242 | 4.7775 | 4.42 |
| 34 | 16774.0 | 2735.249 | 4.5575 | 5.92 |
| 35 | 16856.4 | 2717.187 | 4.5274 | 4.26 |
| 36 | 16883.5 | 2711.235 | 4.5175 | 4.56 |
| 37 | 16903.5 | 2706.854 | 4.5102 | 5.89 |
| 38 | 16930.6 | 2700.914 | 4.5003 | 4.68 |
| 39 | 17021.2 | 2681.046 | 4.4672 | 2.99 |
| 40 | 17056.9 | 2673.228 | 4.4542 | 8.41 |
| 41 | 17099.1 | 2663.974 | 4.4388 | 8.87 |
| 42 | 17135.6 | 2655.976 | 4.4254 | 8.11 |
| 43 | 17200.4 | 2641.771 | 4.4018 | 1.88 |
| 44 | 17232.6 | 2634.706 | 4.3900 | 2.90 |
| 45 | 17271.0 | 2626.288 | 4.3760 | 4.33 |
| 46 | 17308.8 | 2618.001 | 4.3622 | 2.85 |
| 47 | 17401.8 | 2597.607 | 4.3282 | 0.83 |
| 48 | 17459.1 | 2585.043 | 4.3073 | 3.41 |
| 49 | 17512.6 | 2573.328 | 4.2877 | 4.04 |
| 50 | 17596.4 | 2554.947 | 4.2571 | 0.89 |
| 51 | 17628.2 | 2547.968 | 4.2455 | 0.80 |
| 52 | 17666.8 | 2539.518 | 4.2314 | 1.30 |
| 53 | 17723.0 | 2527.184 | 4.2109 | 6.71 |
| 54 | 17745.9 | 2522.172 | 4.2025 | 7.98 |
| 55 | 17791.2 | 2512.248 | 4.1860 | 2.28 |
| 56 | 17874.1 | 2494.071 | 4.1557 | 2.90 |
| 57 | 17895.0 | 2489.477 | 4.1480 | 3.09 |
| 58 | 17927.1 | 2482.454 | 4.1363 | 2.64 |
| 59 | 17948.8 | 2477.683 | 4.1284 | 2.51 |
| 60 | 18034.1 | 2458.993 | 4.0972 | 3.27 |
| 61 | 18064.6 | 2452.313 | 4.0861 | 2.96 |
| 62 | 18076.5 | 2449.702 | 4.0817 | 2.96 |
| 63 | 18118.3 | 2440.538 | 4.0665 | 0.72 |
| 64 | 18156.4 | 2432.180 | 4.0526 | 0.89 |
| 65 | 18196.3 | 2423.432 | 4.0380 | 0.44 |
| 66 | 18432.1 | 2371.735 | 3.9518 | 2.34 |
| 67 | 18459.7 | 2365.684 | 3.9418 | 4.30 |
| 68 | 18487.4 | 2359.600 | 3.9316 | 2.24 |
| 69 | 18962.4 | 2255.466 | 3.7581 | 3.86 |
| 70 | 19011.0 | 2244.816 | 3.7404 | 4.38 |
| 71 | 19669.3 | 2100.485 | 3.4999 | 4.39 |
| 72 | 19717.6 | 2089.898 | 3.4822 | 4.16 |
| 73 | 20365.2 | 1947.920 | 3.2457 | 2.42 |
| 74 | 20401.1 | 1940.054 | 3.2326 | 2.35 |
| 75 | 20418.9 | 1936.161 | 3.2261 | 2.38 |

(continued)

| # | ADDRESS | FREQUENCY [Hz] | [PPM] | INTENSITY |
|---|---|---|---|---|
| 76 | 20751.3 | 1863.290 | 3.1047 | 0.70 |
| 77 | 20815.8 | 1849.152 | 3.0811 | 3.20 |
| 78 | 20857.9 | 1839.919 | 3.0657 | 11.24 |
| 79 | 20924.8 | 1825.236 | 3.0412 | 1.13 |
| 80 | 23431.3 | 1275.730 | 2.1257 | 3.95 |
| 81 | 23491.3 | 1262.573 | 2.1037 | 5.36 |
| 82 | 23538.7 | 1252.187 | 2.0864 | 15.56 |
| 83 | 23570.5 | 1245.203 | 2.0748 | 15.89 |
| 84 | 23696.1 | 1217.673 | 2.0289 | 2.98 |
| 85 | 23770.1 | 1201.463 | 2.0019 | 27.17 |
| 86 | 23827.6 | 1188.849 | 1.9809 | 1.36 |
| 87 | 23887.4 | 1175.734 | 1.9590 | 15.22 |
| 88 | 23919.5 | 1168.700 | 1.9473 | 15.28 |
| 89 | 24010.2 | 1148.811 | 1.9142 | 0.49 |
| 90 | 24104.2 | 1128.207 | 1.8798 | 1.77 |
| 91 | 24197.7 | 1107.716 | 1.8457 | 28.22 |
| 92 | 24263.8 | 1093.228 | 1.8216 | 3.27 |
| 93 | 24321.8 | 1080.503 | 1.8004 | 2.93 |
| 94 | 24373.5 | 1069.166 | 1.7815 | 2.83 |
| 95 | 24497.0 | 1042.094 | 1.7364 | 26.10 |
| 96 | 24598.4 | 1019.871 | 1.6993 | 3.17 |
| 97 | 24626.1 | 1013.793 | 1.6892 | 3.14 |
| 98 | 24676.1 | 1002.826 | 1.6709 | 1.84 |
| 99 | 24931.2 | 946.900 | 1.5777 | 2.14 |
| 100 | 24983.0 | 935.533 | 1.5588 | 3.02 |
| 101 | 25226.8 | 882.103 | 1.4698 | 0.25 |
| 102 | 25370.4 | 850.608 | 1.4173 | 4.11 |
| 103 | 25412.0 | 841.492 | 1.4021 | 4.88 |
| 104 | 25499.7 | 822.270 | 1.3701 | 4.07 |
| 105 | 25556.8 | 809.746 | 1.3492 | 2.62 |
| 106 | 25639.9 | 791.527 | 1.3189 | 31.95 |
| 107 | 25717.9 | 774.418 | 1.2904 | 2.84 |
| 108 | 25790.4 | 758.539 | 1.2639 | 22.85 |
| 109 | 26011.7 | 710.018 | 1.1830 | 4.66 |
| 110 | 26070.7 | 697.082 | 1.1615 | 24.95 |
| 111 | 26249.2 | 657.953 | 1.0963 | 31.39 |
| 112 | 26536.4 | 594.981 | 0.9914 | 25.26 |
| 113 | 26610.9 | 578.657 | 0.9642 | 0.97 |
| 114 | 26914.0 | 512.196 | 0.8534 | 2.04 |
| 115 | 27012.2 | 490.676 | 0.8176 | 25.88 |
| 116 | 27118.1 | 467.463 | 0.7789 | 3.69 |
| 117 | 27226.4 | 443.715 | 0.7393 | 1.07 |
| 118 | 28513.4 | 161.554 | 0.2692 | 0.89 |
| 119 | 28539.8 | 155.777 | 0.2596 | 1.54 |

2D NMR data are presented in following figures: Figure 23 (PP-2D-NMR-Y-HMQC); Figure 24 (PP-2D-NMR-Y-HMBC); and the chemical shift data are listed in Tables 5.2 (HMQC-peak-shift) and Table 5.3 (HMBC-peak-shift) below.

**Table 5.2. Chemical Shift Data of 2D NMR chemical shift of HMQC analysis of Y**

EXPNO=5, PROCNO=1, F1PLO=144.360ppm, F1PHI=10.797ppm, F2PLO=7.966ppm, F2PHI=0.417ppm, MI=1.00cm, MAXI=10000.00cm, PC=1.400

| # | ADDRESS | FREQUENCY | | INTENSITY |
|---|---|---|---|---|
| | Row | [Hz]F1 | [PPM]F1 | |
| | Col | [Hz]F2 | [PPM]F2 | |
| **1** | 148 | 20698.986 | 137.1608 | |
| | 384 | 3574.377 | 5.9557 | 2 |
| **2** | 152 | 20564.195 | 136.2676 | |
| | 401 | 3471.337 | 5.784 | 2.67 |
| **3** | 157 | 20465.209 | 135.6117 | |
| | 220 | 4533.779 | 7.5543 | 45.24 |
| **4** | 223 | 18893.424 | 125.1963 | |
| | 431 | 3295.261 | 5.4906 | 6.22 |
| **5** | 234 | 18649.311 | 123.5787 | |
| | 258 | 4311.82 | 7.1845 | 100 |
| **6** | 315 | 16736.119 | 110.9011 | |
| | 376 | 3620.289 | 6.0322 | 7.49 |
| **7** | 353 | 15834.069 | 104.9237 | |
| | 493 | 2934.55 | 4.8896 | 3.98 |
| **8** | 355 | 15778.398 | 104.5548 | |
| | 449 | 3192.387 | 5.3192 | 2.99 |
| **9** | 355 | 15778.398 | 104.5548 | |
| | 492 | 2936.414 | 4.8927 | 1.18 |
| **10** | 451 | 13524.788 | 89.6213 | |

| # | ADDRESS | FREQUENCY | | INTENSITY |
|---|---|---|---|---|
| | row | [Hz]F1 | [PPM]F1 | |
| | col | [Hz]F2 | [PPM]F2 | |
| **40** | 670 | 8349.676 | 55.3287 | |
| | 911 | 480.283 | 0.8003 | 2.14 |
| **41** | 670 | 8349.676 | 55.3287 | |
| | 913 | 465.738 | 0.776 | 2.18 |
| **42** | 726 | 7038.86 | 46.6427 | |
| | 679 | 1839.659 | 3.0653 | 2.51 |
| **43** | 726 | 7038.86 | 46.6427 | |
| | 819 | 1016.627 | 1.6939 | 1.42 |
| **44** | 726 | 7038.86 | 46.6427 | |
| | 848 | 848.511 | 1.4138 | 2.49 |
| **45** | 764 | 6151.769 | 40.7644 | |
| | 679 | 1841.145 | 3.0678 | 6.4 |
| **46** | 764 | 6151.769 | 40.7644 | |
| | 682 | 1821.592 | 3.0352 | 1.04 |
| **47** | 777 | 5836.727 | 38.6768 | |
| | 850 | 837.869 | 1.3961 | 2.15 |
| **48** | 777 | 5836.727 | 38.6768 | |
| | 853 | 818.861 | 1.3644 | 2.05 |
| **49** | 777 | 5836.727 | 38.6768 | |
| | 907 | 500.256 | 0.8335 | 1.64 |

(continued)

| # | ADDRESS | FREQUENCY | | INTENSITY |
|---|---|---|---|---|
| | Row | [Hz]F1 | [PPM]F1 | |
| | Col | [Hz]F2 | [PPM]F2 | |
| | 660 | 1951.827 | 3.2522 | 2.41 |
| **11** | 451 | 13524.788 | 89.6213 | |
| | 663 | 1934.401 | 3.2231 | 2.6 |
| **12** | 473 | 12994.274 | 86.1059 | |
| | 563 | 2520.987 | 4.2005 | 1.93 |
| **13** | 479 | 12861.933 | 85.229 | |
| | 500 | 2891.933 | 4.8186 | 3.96 |
| **14** | 491 | 12583.008 | 83.3807 | |
| | 487 | 2967.012 | 4.9437 | 6.95 |
| **15** | 523 | 11826.204 | 78.3658 | |
| | 307 | 4025.464 | 6.7073 | 3.92 |
| **16** | 523 | 11826.204 | 78.3658 | |
| | 309 | 4011.801 | 6.6846 | 4.78 |
| **17** | 523 | 11826.204 | 78.3658 | |
| | 545 | 2627.492 | 4.378 | 1.99 |
| **18** | 529 | 11690.02 | 77.4633 | |
| | 504 | 2866.164 | 4.7757 | 4.6 |
| **19** | 532 | 11624.016 | 77.026 | |
| | 530 | 2713.96 | 4.5221 | 2.32 |
| **20** | 532 | 11624.016 | 77.026 | |
| | 532 | 2703.563 | 4.5047 | 2.51 |

| # | ADDRESS | FREQUENCY | | INTENSITY |
|---|---|---|---|---|
| | row | [Hz]F1 | [PPM]F1 | |
| | col | [Hz]F2 | [PPM]F2 | |
| **50** | 791 | 5512.022 | 36.5251 | |
| | 775 | 1277.34 | 2.1283 | 2.38 |
| **51** | 791 | 5512.022 | 36.5251 | |
| | 778 | 1258.929 | 2.0977 | 1.38 |
| **52** | 791 | 5512.022 | 36.5251 | |
| | 785 | 1218.892 | 2.0309 | 1.38 |
| **53** | 791 | 5512.022 | 36.5251 | |
| | 788 | 1201.847 | 2.0025 | 1.09 |
| **54** | 837 | 4417.038 | 29.2693 | |
| | 881 | 655.973 | 1.093 | 23.74 |
| **55** | 848 | 4174.679 | 27.6633 | |
| | 864 | 757.206 | 1.2617 | 20.53 |
| **56** | 848 | 4174.679 | 27.6633 | |
| | 872 | 709.06 | 1.1815 | 3.17 |
| **57** | 856 | 3984.149 | 26.4008 | |
| | 774 | 1281.339 | 2.135 | 1.66 |
| **58** | 856 | 3984.149 | 26.4008 | |
| | 778 | 1259.751 | 2.099 | 1.59 |
| **59** | 856 | 3984.149 | 26.4008 | |
| | 804 | 1107.062 | 1.8446 | 1.02 |

(continued)

| # | ADDRESS Row Col | FREQUENCY [Hz]F1 [Hz]F2 | [PPM]F1 [PPM]F2 | INTENSITY |
|---|---|---|---|---|
| 21 | 535 | 11536.339 | 76.445 | |
| | 590 | 2363.678 | 3.9384 | 3.34 |
| 22 | 545 | 11299.475 | 74.8754 | |
| | 573 | 2461.387 | 4.1012 | 2.35 |
| 23 | 545 | 11299.475 | 74.8754 | |
| | 576 | 2447.179 | 4.0775 | 2.1 |
| 24 | 555 | 11063.554 | 73.3121 | |
| | 541 | 2653.693 | 4.4216 | 6.62 |
| 25 | 567 | 10795.113 | 71.5333 | |
| | 537 | 2673.042 | 4.4539 | 1.84 |
| 26 | 567 | 10795.113 | 71.5333 | |
| | 539 | 2662.683 | 4.4366 | 2.82 |
| 27 | 567 | 10795.113 | 71.5333 | |
| | 541 | 2650.933 | 4.417 | 1.72 |
| 28 | 579 | 10495.725 | 69.5494 | |
| | 527 | 2734.037 | 4.5555 | 5.06 |
| 29 | 594 | 10156.363 | 67.3006 | |
| | 563 | 2523.651 | 4.205 | 7.18 |
| 30 | 622 | 9486.037 | 62.8588 | |
| | 608 | 2256.69 | 3.7601 | 2.91 |

| # | ADDRESS row col | FREQUENCY [Hz]F1 [Hz]F2 | [PPM]F1 [PPM]F2 | INTENSITY |
|---|---|---|---|---|
| 60 | 856 | 3984.149 | 26.4008 | |
| | 807 | 1089.219 | 1.8149 | 1.21 |
| 61 | 873 | 3578.068 | 23.7099 | |
| | 799 | 1138.137 | 1.8964 | 1.68 |
| 62 | 873 | 3578.068 | 23.7099 | |
| | 814 | 1046.185 | 1.7432 | 1.26 |
| 63 | 891 | 3142.837 | 20.8259 | |
| | 788 | 1200.655 | 2.0006 | 13.95 |
| 64 | 891 | 3142.837 | 20.8259 | |
| | 804 | 1107.046 | 1.8446 | 28.24 |
| 65 | 894 | 3086.147 | 20.4502 | |
| | 788 | 1200.275 | 1.9999 | 3.08 |
| 66 | 894 | 3086.147 | 20.4502 | |
| | 804 | 1106.803 | 1.8442 | 1.17 |
| 67 | 894 | 3086.147 | 20.4502 | |
| | 815 | 1041.758 | 1.7358 | 27.19 |
| 68 | 894 | 3086.147 | 20.4502 | |
| | 858 | 789.804 | 1.316 | 1.16 |
| 69 | 897 | 3015.337 | 19.981 | |
| | 858 | 790.292 | 1.3168 | 33.47 |
| 70 | 906 | 2802.854 | 18.573 | |

(continued)

| # | ADDRESS Row Col | FREQUENCY [Hz]F1 [Hz]F2 | [PPM]F1 [PPM]F2 | INTENSITY | # | ADDRESS row col | FREQUENCY [Hz]F1 [Hz]F2 | [PPM]F1 [PPM]F2 | INTENSITY |
|---|---|---|---|---|---|---|---|---|---|
| **31** | 622 | 9486.037 | 62.8588 | | | 830 | 953.652 | 1.589 | 1.35 |
| | 611 | 2241.336 | 3.7346 | 2.67 | | | | | |
| | | | | | **71** | 906 | 2802.854 | 18.573 | |
| **32** | 622 | 9486.037 | 62.8588 | | | 834 | 931.208 | 1.5516 | 1.89 |
| | 635 | 2100.199 | 3.4994 | 2.96 | | | | | |
| | | | | | **72** | 914 | 2613.995 | 17.3215 | |
| **33** | 622 | 9486.037 | 62.8588 | | | 892 | 592.663 | 0.9875 | 12.71 |
| | 637 | 2086.756 | 3.477 | 3.42 | | | | | |
| | | | | | **73** | 919 | 2490.082 | 16.5004 | |
| **34** | 627 | 9381.439 | 62.1656 | | | 875 | 693.445 | 1.1554 | 8.9 |
| | 552 | 2586.967 | 4.3105 | 3.58 | | | | | |
| | | | | | **74** | 919 | 2490.082 | 16.5004 | |
| **35** | 627 | 9381.439 | 62.1656 | | | 880 | 660.343 | 1.1003 | 1.76 |
| | 555 | 2568.334 | 4.2794 | 3.42 | | | | | |
| | | | | | **75** | 925 | 2342.84 | 15.5247 | |
| **36** | 627 | 9381.439 | 62.1656 | | | 778 | 1258.345 | 2.0967 | 5.45 |
| | 568 | 2494.546 | 4.1565 | 2.54 | | | | | |
| | | | | | **76** | 925 | 2342.84 | 15.5247 | |
| **37** | 627 | 9381.439 | 62.1656 | | | 782 | 1237.122 | 2.0613 | 5.31 |
| | 571 | 2474.559 | 4.1232 | 2.51 | | | | | |
| | | | | | | 925 | 2342.84 | 15.5247 | |
| **38** | 630 | 9297.809 | 61.6115 | | **77** | 791 | 1183.41 | 1.9718 | 2.33 |
| | 531 | 2709.734 | 4.515 | 2.61 | | | | | |
| | | | | | **78** | 925 | 2342.84 | 15.5247 | |
| **39** | 630 | 9297.809 | 61.6115 | | | 795 | 1159.732 | 1.9324 | 2.38 |
| | 539 | 2660.734 | 4.4334 | 2.66 | | | | | |
| | | | | | **79** | 925 | 2342.84 | 15.5247 | |
| | | | | | | 907 | 503.26 | 0.8385 | 6.27 |
| | | | | | **80** | 925 | 2342.84 | 15.5247 | |
| | | | | | | 912 | 474.699 | 0.791 | 7.15 |

**Table 5.3. Chemical Shift Data of 2D NMR chemical shift of HMBC analysis of Y**

EXPNO=6, PROCNO=1 F1PL0=178.339ppm, F1PHI=10.721ppm, F2PLO=6.881ppm, F2PHI=0.573ppm MI=1.00cm, MAXI=10000.00cm, PC=1.400

| # | ADDRESS | FREQUENCY | | INTENSITY | # | ADDRESS | FREQUENCY | | INTENSITY |
|---|---|---|---|---|---|---|---|---|---|
| | Row | [Hz/F1] | [PPM/F1] | | | Row | [Hz/F1] | [PPM/F1] | |
| | Col | [Hz/F2] | [PPM/F2] | | | Col | [Hz/F2] | [PPM/F2] | |
| **1** | 123 | 26590.75 | 176.2058 | | **181** | 814 | 6218.377 | 41.2066 | |
| | 895 | 573.276 | 0.9552 | 3.11 | | 880 | 659.072 | 1.0982 | 1.66 |
| **2** | 145 | 25939.373 | 171.8894 | | **182** | 814 | 6218.377 | 41.2066 | |
| | 531 | 2710.686 | 4.5166 | 2.92 | | 891 | 594.287 | 0.9902 | 49.75 |
| **3** | 145 | 25939.373 | 171.8894 | | **183** | 814 | 6218.377 | 41.2066 | |
| | 539 | 2662.033 | 4.4355 | 2.54 | | 895 | 572.724 | 0.9543 | 2.16 |
| **4** | 166 | 25312.006 | 167.7321 | | **184** | 814 | 6218.377 | 41.2066 | |
| | 308 | 4016.437 | 6.6923 | 3.45 | | 909 | 489.811 | 0.8161 | 1.62 |
| **5** | 166 | 25312.006 | 167.7321 | | **185** | 823 | 5950.421 | 39.431 | |
| | 346 | 3793.847 | 6.3214 | 14.9 | | 662 | 1940.805 | 3.2338 | 1.07 |
| **6** | 166 | 25312.006 | 167.7321 | | **186** | 823 | 5950.421 | 39.431 | |
| | 348 | 3785.322 | 6.3072 | 12.47 | | 776 | 1271.011 | 2.1178 | 1.62 |
| **7** | 166 | 25312.006 | 167.7321 | | | | | | |
| | 385 | 3564.443 | 5.9392 | 1.47 | **187** | 823 | 5950.421 | 39.431 | |
| | | | | | | 804 | 1107.587 | 1.8455 | 2.29 |
| **8** | 166 | 25312.006 | 167.7321 | | | | | | |
| | 400 | 3477.948 | 5.795 | 4.16 | **188** | 823 | 5950.421 | 39.431 | |
| | | | | | | 831 | 947.726 | 1.5791 | 1.04 |
| **9** | 166 | 25312.006 | 167.7321 | | | | | | |
| | 403 | 3458.552 | 5.7627 | 3.96 | **189** | 823 | 5950.421 | 39.431 | |
| | | | | | | 858 | 790.52 | 1.3172 | 1.45 |
| **10** | 166 | 25312.006 | 167.7321 | | | | | | |

(continued)

| # | ADDRESS | FREQUENCY | | INTENSITY | # | ADDRESS | FREQUENCY | | INTENSITY |
|---|---|---|---|---|---|---|---|---|---|
| | 781 | 1243.983 | 2.0728 | 1.17 | **190** | 823 | 5950.421 | 39.431 | |
| | | | | | | 864 | 757.09 | 1.2615 | 54.14 |
| **11** | 166 | 25312.006 | 167.7321 | | | | | | |
| | 788 | 1200.447 | 2.0002 | 10.44 | **191** | 823 | 5950.421 | 39.431 | |
| | | | | | | 874 | 696.048 | 1.1598 | 72.55 |
| **12** | 166 | 25312.006 | 167.7321 | | | | | | |
| | 793 | 1171.992 | 1.9528 | 3.35 | **192** | 823 | 5950.421 | 39.431 | |
| | | | | | | 880 | 658.503 | 1.0972 | 9.62 |
| **13** | 166 | 25312.006 | 167.7321 | | | | | | |
| | 815 | 1041.336 | 1.7351 | 33.24 | **193** | 823 | 5950.421 | 39.431 | |
| | | | | | | 891 | 594.53 | 0.9906 | 2.75 |
| **14** | 290 | 21640.068 | 143.3997 | | | | | | |
| | 679 | 1839.97 | 3.0658 | 5.67 | **194** | 823 | 5950.421 | 39.431 | |
| | | | | | | 911 | 479.432 | 0.7988 | 7.32 |
| **15** | 290 | 21640.068 | 143.3997 | | | | | | |
| | 788 | 1199.609 | 1.9988 | 1.03 | **195** | 823 | 5950.421 | 39.431 | |
| | | | | | | 913 | 465.776 | 0.7761 | 7.03 |
| **16** | 290 | 21640.068 | 143.3997 | | | | | | |
| | 804 | 1107.222 | 1.8449 | 33.9 | **196** | 827 | 5834.797 | 38.6648 | |
| | | | | | | 804 | 1106.687 | 1.844 | 2.69 |
| **17** | 290 | 21640.068 | 143.3997 | | | | | | |
| | 813 | 1053.918 | 1.7561 | 2.3 | **197** | 827 | 5834.797 | 38.6648 | |
| | | | | | | 819 | 1017.803 | 1.6959 | 1.66 |
| **18** | 290 | 21640.068 | 143.3997 | | | | | | |
| | 848 | 848.155 | 1.4132 | 1.25 | **198** | 827 | 5834.797 | 38.6648 | |
| | | | | | | 822 | 1001.708 | 1.6691 | 1.68 |
| **19** | 322 | 20697.354 | 137.1527 | | | | | | |
| | 780 | 1246.505 | 2.077 | 31.46 | **199** | 827 | 5834.797 | 38.6648 | |
| | | | | | | 841 | 889.985 | 1.4829 | 1.01 |
| **20** | 322 | 20697.354 | 137.1527 | | | | | | |
| | 788 | 1200.075 | 1.9996 | 44.6 | **200** | 827 | 5834.797 | 38.6648 | |
| | | | | | | 858 | 789.621 | 1.3157 | 1.33 |
| **21** | 322 | 20697.354 | 137.1527 | | | | | | |

(continued)

| # | ADDRESS | FREQUENCY | | INTENSITY | # | ADDRESS | FREQUENCY | | INTENSITY |
|---|---|---|---|---|---|---|---|---|---|
| | 793 | 1170.377 | 1.9501 | 3.79 | **201** | 827 | 5834.797 | 38.6648 | |
| | | | | | | 864 | 758.164 | 1.2633 | 3.94 |
| **22** | 322 | 20697.354 | 137.1527 | | | | | | |
| | 815 | 1040.86 | 1.7343 | 4.31 | **202** | 827 | 5834.797 | 38.6648 | |
| | | | | | | 874 | 696.137 | 1.1599 | 6.09 |
| **23** | 327 | 20566.367 | 136.2847 | | | | | | |
| | 780 | 1246.629 | 2.0772 | 3.06 | **203** | 827 | 5834.797 | 38.6648 | |
| | | | | | | 881 | 656.905 | 1.0945 | 2.43 |
| **24** | 327 | 20566.367 | 136.2847 | | | | | | |
| | 788 | 1201.192 | 2.0015 | 5.78 | **204** | 827 | 5834.797 | 38.6648 | |
| | | | | | | 892 | 593.606 | 0.9891 | 1.9 |
| **25** | 327 | 20566.367 | 136.2847 | | | | | | |
| | 793 | 1170.748 | 1.9507 | 47.25 | **205** | 827 | 5834.797 | 38.6648 | |
| | | | | | | 909 | 489.245 | 0.8152 | 51.13 |
| **26** | 327 | 20566.367 | 136.2847 | | | | | | |
| | 815 | 1041.067 | 1.7346 | 58.19 | **206** | 830 | 5743.329 | 38.0586 | |
| | | | | | | 804 | 1108.031 | 1.8462 | 1.4 |
| **27** | 365 | 19434.006 | 128.7811 | | | | | | |
| | 780 | 1245.861 | 2.0759 | 16.02 | **207** | 830 | 5743.329 | 38.0586 | |
| | | | | | | 858 | 790.551 | 1.3172 | 1.95 |
| **28** | 365 | 19434.006 | 128.7811 | | | | | | |
| | 788 | 1200.442 | 2.0002 | 33.73 | **208** | 830 | 5743.329 | 38.0586 | |
| | | | | | | 864 | 757.091 | 1.2615 | 2.85 |
| **29** | 365 | 19434.006 | 128.7811 | | | | | | |
| | 793 | 1171.204 | 1.9515 | 46.7 | **209** | 830 | 5743.329 | 38.0586 | |
| | | | | | | 874 | 696.052 | 1.1598 | 3.54 |
| **30** | 365 | 19434.006 | 128.7811 | | | | | | |
| | 815 | 1040.94 | 1.7344 | 80.6 | **210** | 830 | 5743.329 | 38.0586 | |
| | | | | | | 881 | 655.62 | 1.0924 | 2.41 |
| **31** | 384 | 18893.113 | 125.1968 | | | | | | |
| | 679 | 1839.547 | 3.0651 | 9.8 | **211** | 830 | 5743.329 | 38.0586 | |
| | | | | | | 891 | 594.762 | 0.991 | 3.06 |
| **32** | 384 | 18893.113 | 125.1968 | | | | | | |

(continued)

| # | ADDRESS | FREQUENCY | | INTENSITY | # | ADDRESS | FREQUENCY | | INTENSITY |
|---|---|---|---|---|---|---|---|---|---|
| | 788 | 1200.729 | 2.0007 | 2.34 | **212** | 830 | 5743.329 | 38.0586 | |
| | | | | | | 909 | 489.474 | 0.8156 | 10.76 |
| **33** | 384 | 18893.113 | 125.1968 | | | | | | |
| | 801 | 1124.942 | 1.8744 | 1.93 | **213** | 834 | 5623.896 | 37.2672 | |
| | | | | | | 804 | 1107.433 | 1.8452 | 1.25 |
| **34** | 384 | 18893.113 | 125.1968 | | | | | | |
| | 803 | 1113.96 | 1.8561 | 1.36 | **214** | 834 | 5623.896 | 37.2672 | |
| | | | | | | 822 | 1002.039 | 1.6696 | 1.43 |
| **35** | 384 | 18893.113 | 125.1968 | | | | | | |
| | 809 | 1077.556 | 1.7954 | 3.32 | **215** | 834 | 5623.896 | 37.2672 | |
| | | | | | | 833 | 939.62 | 1.5656 | 1.01 |
| **36** | 384 | 18893.113 | 125.1968 | | | | | | |
| | 813 | 1051.694 | 1.7524 | 3.14 | **216** | 834 | 5623.896 | 37.2672 | |
| | | | | | | 858 | 790.603 | 1.3173 | 4.03 |
| **37** | 384 | 18893.113 | 125.1968 | | | | | | |
| | 848 | 847.499 | 1.4121 | 1.45 | **217** | 834 | 5623.896 | 37.2672 | |
| | | | | | | 864 | 756.768 | 1.2609 | 1.89 |
| **38** | 457 | 16738.236 | 110.9173 | | | | | | |
| | 361 | 3707.331 | 6.1772 | 6.44 | **218** | 834 | 5623.896 | 37.2672 | |
| | | | | | | 874 | 696.444 | 1.1604 | 2.15 |
| **39** | 457 | 16738.236 | 110.9173 | | | | | | |
| | 390 | 3534.169 | 5.8887 | 6.75 | **219** | 834 | 5623.896 | 37.2672 | |
| | | | | | | 881 | 656.011 | 1.0931 | 4.39 |
| **40** | 457 | 16738.236 | 110.9173 | | | | | | |
| | 486 | 2972.803 | 4.9534 | 1.24 | **220** | 834 | 5623.896 | 37.2672 | |
| | | | | | | 891 | 594.776 | 0.991 | 4.19 |
| **41** | 457 | 16738.236 | 110.9173 | | | | | | |
| | 488 | 2962.223 | 4.9357 | 1.4 | **221** | 834 | 5623.896 | 37.2672 | |
| | | | | | | 909 | 489.272 | 0.8152 | 10.53 |
| **42** | 457 | 16738.236 | 110.9173 | | | | | | |
| | 563 | 2520.559 | 4.1998 | 12.21 | **222** | 837 | 5533.835 | 36.6704 | |
| | | | | | | 777 | 1268.681 | 2.1139 | 1.72 |
| **43** | 488 | 15822.76 | 104.8508 | | | | | | |

(continued)

| # | ADDRESS | FREQUENCY | | INTENSITY | # | ADDRESS | FREQUENCY | | INTENSITY |
|---|---|---|---|---|---|---|---|---|---|
| | 531 | 2712.055 | 4.5189 | 1.39 | **223** | 837 | 5533.835 | 36.6704 | |
| | | | | | | 804 | 1108.282 | 1.8466 | 1.01 |
| **44** | 488 | 15822.76 | 104.8508 | | | | | | |
| | 538 | 2668.887 | 4.447 | 4.68 | **224** | 837 | 5533.835 | 36.6704 | |
| | | | | | | 811 | 1067.12 | 1.7781 | 1.7 |
| **45** | 488 | 15822.76 | 104.8508 | | | | | | |
| | 545 | 2627.709 | 4.3783 | 14.61 | **225** | 837 | 5533.835 | 36.6704 | |
| | | | | | | 819 | 1017.407 | 1.6952 | 4.37 |
| **46** | 488 | 15822.76 | 104.8508 | | | | | | |
| | 660 | 1952.474 | 3.2533 | 2.34 | **226** | 837 | 5533.835 | 36.6704 | |
| | | | | | | 822 | 1001.73 | 1.6691 | 4.67 |
| **47** | 488 | 15822.76 | 104.8508 | | | | | | |
| | 662 | 1941.101 | 3.2343 | 3.52 | **227** | 837 | 5533.835 | 36.6704 | |
| | | | | | | 832 | 943.762 | 1.5725 | 2.78 |
| **48** | 488 | 15822.76 | 104.8508 | | | | | | |
| | 664 | 1928.93 | 3.214 | 2.41 | **228** | 837 | 5533.835 | 36.6704 | |
| | | | | | | 849 | 841.934 | 1.4028 | 2.09 |
| **49** | 489 | 15777.47 | 104.5507 | | | | | | |
| | 538 | 2669.101 | 4.4473 | 12.69 | **229** | 837 | 5533.835 | 36.6704 | |
| | | | | | | 858 | 790.63 | 1.3174 | 8.57 |
| **50** | 489 | 15777.47 | 104.5507 | | | | | | |
| | 546 | 2622.941 | 4.3704 | 6.06 | **230** | 837 | 5533.835 | 36.6704 | |
| | | | | | | 864 | 757.963 | 1.2629 | 2.1 |
| **51** | 489 | 15777.47 | 104.5507 | | | | | | |
| | 590 | 2363.695 | 3.9384 | 3.08 | **231** | 837 | 5533.835 | 36.6704 | |
| | | | | | | 874 | 695.245 | 1.1584 | 1.83 |
| **52** | 489 | 15777.47 | 104.5507 | | | | | | |
| | 660 | 1953.443 | 3.2549 | 2.02 | **232** | 837 | 5533.835 | 36.6704 | |
| | | | | | | 881 | 656.351 | 1.0936 | 11.03 |
| **53** | 489 | 15777.47 | 104.5507 | | | | | | |
| | 662 | 1940.913 | 3.234 | 1.47 | **233** | 837 | 5533.835 | 36.6704 | |
| | | | | | | 891 | 594.692 | 0.9909 | 25.74 |
| **54** | 566 | 13527.92 | 89.6439 | | | | | | |

(continued)

| # | ADDRESS | FREQUENCY | | INTENSITY | # | ADDRESS | FREQUENCY | | INTENSITY |
|---|---|---|---|---|---|---|---|---|---|
| | 492 | 2938.198 | 4.8957 | 11.15 | **234** | 837 | 5533.835 | 36.6704 | |
| | | | | | | 909 | 489.139 | 0.815 | 53.53 |
| **55** | 566 | 13527.92 | 89.6439 | | | | | | |
| | 805 | 1101.852 | 1.8359 | 1.93 | **235** | 837 | 5533.835 | 36.6704 | |
| | | | | | | 913 | 467.893 | 0.7796 | 6.55 |
| **56** | 566 | 13527.92 | 89.6439 | | | | | | |
| | 851 | 830.552 | 1.3839 | 5.09 | **236** | 840 | 5450.419 | 36.1177 | |
| | | | | | | 307 | 4023.448 | 6.704 | 5.35 |
| **57** | 566 | 13527.92 | 89.6439 | | | | | | |
| | 864 | 757.374 | 1.262 | 53.64 | **237** | 840 | 5450.419 | 36.1177 | |
| | | | | | | 309 | 4013.866 | 6.688 | 5.05 |
| **58** | 566 | 13527.92 | 89.6439 | | | | | | |
| | 874 | 696.11 | 1.1599 | 39.08 | **238** | 840 | 5450.419 | 36.1177 | |
| | | | | | | 346 | 3794.546 | 6.3226 | 1.25 |
| **59** | 566 | 13527.92 | 89.6439 | | | | | | |
| | 880 | 658.836 | 1.0978 | 4.62 | **239** | 840 | 5450.419 | 36.1177 | |
| | | | | | | 679 | 1839.242 | 3.0646 | 3.88 |
| **60** | 566 | 13527.92 | 89.6439 | | | | | | |
| | 911 | 480.432 | 0.8005 | 1.38 | **240** | 840 | 5450.419 | 36.1177 | |
| | | | | | | 849 | 842.83 | 1.4043 | 6.34 |
| **61** | 566 | 13527.92 | 89.6439 | | | | | | |
| | 913 | 466.506 | 0.7773 | 1.4 | **241** | 840 | 5450.419 | 36.1177 | |
| | | | | | | 858 | 790.237 | 1.3167 | 88.69 |
| **62** | 584 | 12992.842 | 86.0981 | | | | | | |
| | 376 | 3621.487 | 6.0342 | 5.29 | **242** | 840 | 5450.419 | 36.1177 | |
| | | | | | | 862 | 767.125 | 1.2782 | 1.79 |
| **63** | 584 | 12992.842 | 86.0981 | | | | | | |
| | 531 | 2708.564 | 4.5131 | 1.17 | **243** | 840 | 5450.419 | 36.1177 | |
| | | | | | | 864 | 755.041 | 1.2581 | 1.99 |
| **64** | 584 | 12992.842 | 86.0981 | | | | | | |
| | 539 | 2662.648 | 4.4366 | 4.18 | **244** | 840 | 5450.419 | 36.1177 | |
| | | | | | | 874 | 695.441 | 1.1588 | 2.24 |

(continued)

| # | ADDRESS | FREQUENCY | | INTENSITY | # | ADDRESS | FREQUENCY | | INTENSITY |
|---|---|---|---|---|---|---|---|---|---|
| **65** | 584 | 12992.842 | 86.0981 | | **245** | 840 | 5450.419 | 36.1177 | |
| | 545 | 2625.947 | 4.3754 | 6.92 | | 881 | 656.06 | 1.0931 | 84.89 |
| **66** | 584 | 12992.842 | 86.0981 | | **246** | 840 | 5450.419 | 36.1177 | |
| | 864 | 757.04 | 1.2614 | 1.36 | | 891 | 594.675 | 0.9909 | 7.64 |
| **67** | 588 | 12860.941 | 85.2241 | | **247** | 840 | 5450.419 | 36.1177 | |
| | 370 | 3655.044 | 6.0901 | 2.76 | | 909 | 489.3 | 0.8153 | 8.88 |
| **68** | 588 | 12860.941 | 85.2241 | | **248** | 875 | 4418.238 | 29.2778 | |
| | 376 | 3621.609 | 6.0344 | 10.88 | | 307 | 4022.778 | 6.7028 | 8.21 |
| **51** | 489 | 15777.47 | 104.5507 | | | | | | |
| **69** | 588 | 12860.941 | 85.2241 | | **249** | 875 | 4418.238 | 29.2778 | |
| | 554 | 2574.294 | 4.2893 | 1.21 | | 309 | 4014.385 | 6.6889 | 7.83 |
| **70** | 588 | 12860.941 | 85.2241 | | **250** | 875 | 4418.238 | 29.2778 | |
| | 864 | 756.227 | 1.26 | 1.05 | | 679 | 1841.576 | 3.0685 | 1.85 |
| **71** | 598 | 12585.614 | 83.3996 | | **251** | 875 | 4418.238 | 29.2778 | |
| | 376 | 3621.268 | 6.0338 | 1.92 | | 849 | 844.669 | 1.4074 | 1.65 |
| **72** | 598 | 12585.614 | 83.3996 | | **252** | 875 | 4418.238 | 29.2778 | |
| | 475 | 3038.901 | 5.0635 | 1.23 | | 858 | 790.492 | 1.3171 | 100 |
| **73** | 598 | 12585.614 | 83.3996 | | **253** | 875 | 4418.238 | 29.2778 | |
| | 500 | 2890.984 | 4.817 | 1.6 | | 863 | 763.811 | 1.2727 | 3.72 |
| **74** | 598 | 12585.614 | 83.3996 | | **254** | 875 | 4418.238 | 29.2778 | |
| | 505 | 2863.233 | 4.7708 | 3.39 | | 870 | 718.885 | 1.1978 | 22.51 |
| **75** | 598 | 12585.614 | 83.3996 | | **255** | 875 | 4418.238 | 29.2778 | |
| | 881 | 655.156 | 1.0916 | 1.11 | | 875 | 691.903 | 1.1529 | 4.9 |

(continued)

| # | ADDRESS | FREQUENCY | | INTENSITY | # | ADDRESS | FREQUENCY | | INTENSITY |
|---|---|---|---|---|---|---|---|---|---|
| **76** | 623 | 11822.784 | 78.3447 | | **256** | 875 | 4418.238 | 29.2778 | |
| | 295 | 4093.818 | 6.8212 | 2.3 | | 878 | 670.147 | 1.1166 | 1.92 |
| **77** | 623 | 11822.784 | 78.3447 | | **257** | 875 | 4418.238 | 29.2778 | |
| | 321 | 3940.803 | 6.5663 | 2.25 | | 882 | 648.47 | 1.0805 | 1.77 |
| **78** | 623 | 11822.784 | 78.3447 | | **258** | 875 | 4418.238 | 29.2778 | |
| | 348 | 3785.12 | 6.3069 | 18.28 | | 892 | 593.301 | 0.9886 | 19.66 |
| **79** | 623 | 11822.784 | 78.3447 | | **259** | 883 | 4176.082 | 27.6732 | |
| | 434 | 3277.04 | 5.4603 | 1.13 | | 662 | 1941.3 | 3.2346 | 1.71 |
| **80** | 623 | 11822.784 | 78.3447 | | **260** | 883 | 4176.082 | 27.6732 | |
| | 448 | 3194.918 | 5.3234 | 7.26 | | 853 | 819.336 | 1.3652 | 15.64 |
| **81** | 623 | 11822.784 | 78.3447 | | **261** | 883 | 4176.082 | 27.6732 | |
| | 563 | 2521.171 | 4.2008 | 7.36 | | 857 | 794.801 | 1.3243 | 4.59 |
| **82** | 623 | 11822.784 | 78.3447 | | **262** | 883 | 4176.082 | 27.6732 | |
| | 845 | 867.271 | 1.4451 | 1.26 | | 863 | 763.374 | 1.272 | 1.7 |
| **83** | 623 | 11822.784 | 78.3447 | | **263** | 883 | 4176.082 | 27.6732 | |
| | 849 | 844.669 | 1.4074 | 10.09 | | 874 | 696.058 | 1.1598 | 89.76 |
| **84** | 623 | 11822.784 | 78.3447 | | **264** | 883 | 4176.082 | 27.6732 | |
| | 858 | 790.448 | 1.3171 | 26.63 | | 878 | 671.861 | 1.1195 | 2.96 |
| **85** | 623 | 11822.784 | 78.3447 | | **265** | 883 | 4176.082 | 27.6732 | |
| | 881 | 656.434 | 1.0938 | 51.16 | | 881 | 657.198 | 1.095 | 7.96 |
| **86** | 630 | 11619.674 | 76.9987 | | **266** | 883 | 4176.082 | 27.6732 | |
| | 348 | 3785.085 | 6.3068 | 1.21 | | 911 | 479.487 | 0.7989 | 2.54 |

(continued)

| # | ADDRESS | FREQUENCY | | INTENSITY | # | ADDRESS | FREQUENCY | | INTENSITY |
|---|---|---|---|---|---|---|---|---|---|
| **87** | 630 | 11619.674 | 76.9987 | | **267** | 883 | 4176.082 | 27.6732 | |
| | 539 | 2661.936 | 4.4354 | 6.49 | | 913 | 466.645 | 0.7775 | 2.27 |
| **88** | 630 | 11619.674 | 76.9987 | | **268** | 903 | 3588.775 | 23.7813 | |
| | 552 | 2584.607 | 4.3065 | 1.49 | | 431 | 3296.117 | 5.4921 | 2.85 |
| **89** | 630 | 11619.674 | 76.9987 | | **269** | 903 | 3588.775 | 23.7813 | |
| | 858 | 790.978 | 1.3179 | 2.74 | | 791 | 1182.756 | 1.9707 | 1.05 |
| **90** | 630 | 11619.674 | 76.9987 | | **270** | 903 | 3588.775 | 23.7813 | |
| | 881 | 655.977 | 1.093 | 5.21 | | 801 | 1127.886 | 1.8793 | 1.45 |
| **91** | 641 | 11304.499 | 74.9102 | | **271** | 903 | 3588.775 | 23.7813 | |
| | 307 | 4021.328 | 6.7004 | 1.03 | | 804 | 1108.54 | 1.8471 | 1.43 |
| **92** | 641 | 11304.499 | 74.9102 | | **272** | 903 | 3588.775 | 23.7813 | |
| | 526 | 2736.013 | 4.5588 | 4.14 | | 811 | 1064.128 | 1.7731 | 1.37 |
| **93** | 641 | 11304.499 | 74.9102 | | **273** | 903 | 3588.775 | 23.7813 | |
| | 538 | 2670.608 | 4.4498 | 9.8 | | 821 | 1006.1 | 1.6764 | 3.82 |
| **94** | 641 | 11304.499 | 74.9102 | | **274** | 903 | 3588.775 | 23.7813 | |
| | 881 | 656.537 | 1.0939 | 1.61 | | 874 | 696.649 | 1.1608 | 2.42 |
| **95** | 650 | 11028.925 | 73.0841 | | **275** | 903 | 3588.775 | 23.7813 | |
| | 308 | 4015.948 | 6.6915 | 16.79 | | 881 | 655.757 | 1.0926 | 2.43 |
| **96** | 650 | 11028.925 | 73.0841 | | **276** | 917 | 3174.632 | 21.037 | |
| | 346 | 3793.459 | 6.3207 | 13.94 | | 307 | 4021.419 | 6.7006 | 1.44 |
| **97** | 650 | 11028.925 | 73.0841 | | **277** | 917 | 3174.632 | 21.037 | |
| | 526 | 2736.761 | 4.5601 | 7.46 | | 384 | 3571.435 | 5.9508 | 2.36 |
| **98** | 650 | 11028.925 | 73.0841 | | **278** | 917 | 3174.632 | 21.037 | |

(continued)

| # | ADDRESS | FREQUENCY | | INTENSITY | # | ADDRESS | FREQUENCY | | INTENSITY |
|---|---|---|---|---|---|---|---|---|---|
| | 574 | 2454.41 | 4.0896 | 4.7 | | 403 | 3463.331 | 5.7707 | 1.11 |
| **99** | 650 | 11028.925 | 73.0841 | | **279** | 917 | 3174.632 | 21.037 | |
| | 610 | 2245.856 | 3.7421 | 11.08 | | 561 | 2531.85 | 4.2186 | 5.21 |
| **100** | 650 | 11028.925 | 73.0841 | | **280** | 917 | 3174.632 | 21.037 | |
| | 637 | 2088.895 | 3.4806 | 5.03 | | 564 | 2517.7 | 4.195 | 5.22 |
| **101** | 650 | 11028.925 | 73.0841 | | **281** | 917 | 3174.632 | 21.037 | |
| | 679 | 1839.51 | 3.065 | 8.83 | | 778 | 1262.748 | 2.104 | 3.52 |
| **102** | 650 | 11028.925 | 73.0841 | | **282** | 917 | 3174.632 | 21.037 | |
| | 848 | 850.226 | 1.4167 | 1.52 | | 793 | 1170.055 | 1.9496 | 22.51 |
| **103** | 650 | 11028.925 | 73.0841 | | **283** | 917 | 3174.632 | 21.037 | |
| | 881 | 656.986 | 1.0947 | 1.8 | | 800 | 1133.275 | 1.8883 | 2.77 |
| **104** | 658 | 10790.329 | 71.503 | | **284** | 917 | 3174.632 | 21.037 | |
| | 308 | 4016.477 | 6.6923 | 1.26 | | 805 | 1100.419 | 1.8335 | 3.08 |
| **105** | 658 | 10790.329 | 71.503 | | **285** | 917 | 3174.632 | 21.037 | |
| | 531 | 2708.84 | 4.5135 | 2.58 | | 809 | 1079.427 | 1.7986 | 2.91 |
| **106** | 658 | 10790.329 | 71.503 | | **286** | 917 | 3174.632 | 21.037 | |
| | 564 | 2518.203 | 4.1959 | 5.49 | | 815 | 1043.118 | 1.7381 | 24.13 |
| **107** | 658 | 10790.329 | 71.503 | | **287** | 917 | 3174.632 | 21.037 | |
| | 609 | 2253.841 | 3.7554 | 1.07 | | 827 | 975.02 | 1.6246 | 2.05 |
| **108** | 658 | 10790.329 | 71.503 | | **288** | 917 | 3174.632 | 21.037 | |
| | 881 | 655.677 | 1.0925 | 1.26 | | 848 | 851.242 | 1.4184 | 1.6 |
| **109** | 668 | 10496.015 | 69.5527 | | **289** | 917 | 3174.632 | 21.037 | |

(continued)

| # | ADDRESS | FREQUENCY | | INTENSITY | # | ADDRESS | FREQUENCY | | INTENSITY |
|---|---|---|---|---|---|---|---|---|---|
| | 530 | 2715.37 | 4.5244 | 1.73 | | 864 | 757.267 | 1.2618 | 1.67 |
| **110** | 668 | 10496.015 | 69.5527 | | **290** | 917 | 3174.632 | 21.037 | |
| | 533 | 2699.76 | 4.4984 | 1.85 | | 869 | 727.306 | 1.2119 | 1.8 |
| **111** | 668 | 10496.015 | 69.5527 | | **291** | 917 | 3174.632 | 21.037 | |
| | 540 | 2658.708 | 4.43 | 3.19 | | 881 | 656.407 | 1.0937 | 6.11 |
| **112** | 668 | 10496.015 | 69.5527 | | **292** | 922 | 3013.427 | 19.9687 | |
| | 590 | 2363.392 | 3.9379 | 4.52 | | 309 | 4014.743 | 6.6895 | 13.55 |
| **113** | 668 | 10496.015 | 69.5527 | | **293** | 922 | 3013.427 | 19.9687 | |
| | 804 | 1107.602 | 1.8455 | 1 | | 401 | 3472.374 | 5.7857 | 2.11 |
| **114** | 668 | 10496.015 | 69.5527 | | **294** | 922 | 3013.427 | 19.9687 | |
| | 881 | 656.508 | 1.0939 | 1.01 | | 679 | 1841.875 | 3.069 | 7.26 |
| **115** | 680 | 10155.364 | 67.2954 | | **295** | 922 | 3013.427 | 19.9687 | |
| | 541 | 2651.054 | 4.4172 | 2.15 | | 777 | 1263.952 | 2.106 | 1.96 |
| **116** | 680 | 10155.364 | 67.2954 | | **296** | 922 | 3013.427 | 19.9687 | |
| | 804 | 1107.228 | 1.8449 | 32.23 | | 794 | 1165.725 | 1.9424 | 1.57 |
| **117** | 680 | 10155.364 | 67.2954 | | **297** | 922 | 3013.427 | 19.9687 | |
| | 881 | 656.545 | 1.0939 | 1 | | 799 | 1137.94 | 1.8961 | 2.13 |
| **118** | 703 | 9486.47 | 62.8629 | | **298** | 922 | 3013.427 | 19.9687 | |
| | 346 | 3793.169 | 6.3203 | 9.4 | | 804 | 1104.456 | 1.8403 | 3.32 |
| **119** | 703 | 9486.47 | 62.8629 | | **299** | 922 | 3013.427 | 19.9687 | |
| | 348 | 3784.896 | 6.3065 | 9.63 | | 815 | 1043.07 | 1.738 | 1.92 |
| **120** | 703 | 9486.47 | 62.8629 | | **300** | 922 | 3013.427 | 19.9687 | |
| | 679 | 1839.346 | 3.0648 | 3.08 | | 826 | 975.688 | 1.6257 | 3.29 |

(continued)

| # | ADDRESS | FREQUENCY | | INTENSITY | # | ADDRESS | FREQUENCY | | INTENSITY |
|---|---|---|---|---|---|---|---|---|---|
| **121** | 703 | 9486.47 | 62.8629 | | **301** | 922 | 3013.427 | 19.9687 | |
| | 863 | 758.265 | 1.2634 | 1.07 | | 847 | 852.676 | 1.4207 | 19.79 |
| **122** | 706 | 9376.976 | 62.1373 | | **302** | 922 | 3013.427 | 19.9687 | |
| | 347 | 3790.748 | 6.3162 | 1.61 | | 863 | 759.553 | 1.2656 | 3.39 |
| **123** | 706 | 9376.976 | 62.1373 | | **303** | 922 | 3013.427 | 19.9687 | |
| | 505 | 2862.456 | 4.7695 | 2.79 | | 869 | 726.647 | 1.2108 | 19.23 |
| **124** | 706 | 9376.976 | 62.1373 | | **304** | 922 | 3013.427 | 19.9687 | |
| | 881 | 655.399 | 1.092 | 1.01 | | 881 | 656.158 | 1.0933 | 72.09 |
| **125** | 709 | 9296.042 | 61.601 | | **305** | 929 | 2803.19 | 18.5756 | |
| | 590 | 2363.684 | 3.9384 | 7.53 | | 308 | 4021.188 | 6.7002 | 1.12 |
| **126** | 741 | 8346.307 | 55.3075 | | **306** | 929 | 2803.19 | 18.5756 | |
| | 777 | 1264.802 | 2.1074 | 3.77 | | 823 | 997.155 | 1.6615 | 1.03 |
| **127** | 741 | 8346.307 | 55.3075 | | **307** | 929 | 2803.19 | 18.5756 | |
| | 852 | 825.908 | 1.3761 | 3.3 | | 847 | 852.409 | 1.4203 | 2.46 |
| **128** | 741 | 8346.307 | 55.3075 | | **308** | 929 | 2803.19 | 18.5756 | |
| | 864 | 757.401 | 1.262 | 35.15 | | 864 | 756.417 | 1.2604 | 2.78 |
| **129** | 741 | 8346.307 | 55.3075 | | **309** | 929 | 2803.19 | 18.5756 | |
| | 874 | 696.341 | 1.1603 | 25.71 | | 869 | 728.727 | 1.2142 | 2.15 |
| **130** | 741 | 8346.307 | 55.3075 | | **310** | 929 | 2803.19 | 18.5756 | |
| | 881 | 658.199 | 1.0967 | 3.92 | | 881 | 657.345 | 1.0953 | 5.08 |
| **131** | 741 | 8346.307 | 55.3075 | | **311** | 929 | 2803.19 | 18.5756 | |
| | 891 | 595.647 | 0.9925 | 1.15 | | 902 | 531.583 | 0.8857 | 1.25 |

(continued)

| # | ADDRESS | FREQUENCY | | INTENSITY | # | ADDRESS | FREQUENCY | | INTENSITY |
|---|---|---|---|---|---|---|---|---|---|
| **132** | 741 | 8346.307 | 55.3075 | | **312** | 929 | 2803.19 | 18.5756 | |
| | 909 | 489.518 | 0.8156 | 23.33 | | 911 | 478.022 | 0.7965 | 4.88 |
| **133** | 781 | 7163.191 | 47.4675 | | **313** | 929 | 2803.19 | 18.5756 | |
| | 346 | 3793.181 | 6.3203 | 1.69 | | 913 | 466.494 | 0.7773 | 5.42 |
| **134** | 781 | 7163.191 | 47.4675 | | **314** | 936 | 2611.793 | 17.3073 | |
| | 431 | 3294.477 | 5.4893 | 5.3 | | 777 | 1266.005 | 2.1094 | 1.92 |
| **135** | 781 | 7163.191 | 47.4675 | | **315** | 936 | 2611.793 | 17.3073 | |
| | 539 | 2661.476 | 4.4346 | 3.1 | | 783 | 1228.491 | 2.0469 | 2.67 |
| **136** | 781 | 7163.191 | 47.4675 | | **316** | 936 | 2611.793 | 17.3073 | |
| | 541 | 2647.966 | 4.4121 | 3.42 | | 786 | 1211.785 | 2.0191 | 2.98 |
| **137** | 781 | 7163.191 | 47.4675 | | **317** | 936 | 2611.793 | 17.3073 | |
| | 564 | 2517.984 | 4.1955 | 1.14 | | 819 | 1018.301 | 1.6967 | 5.9 |
| **138** | 781 | 7163.191 | 47.4675 | | **318** | 936 | 2611.793 | 17.3073 | |
| | 679 | 1839.442 | 3.0649 | 3.64 | | 822 | 1001.86 | 1.6693 | 7.01 |
| **139** | 781 | 7163.191 | 47.4675 | | **319** | 936 | 2611.793 | 17.3073 | |
| | 804 | 1107.164 | 1.8448 | 37.94 | | 864 | 757.418 | 1.262 | 6.44 |
| **140** | 781 | 7163.191 | 47.4675 | | **320** | 936 | 2611.793 | 17.3073 | |
| | 822 | 1002.093 | 1.6697 | 1.62 | | 869 | 725.753 | 1.2093 | 1.48 |
| **141** | 781 | 7163.191 | 47.4675 | | | | | | |
| | 849 | 845.058 | 1.4081 | 2.98 | **321** | 936 | 2611.793 | 17.3073 | |
| | | | | | | 872 | 707.264 | 1.1785 | 1.56 |
| **142** | 781 | 7163.191 | 47.4675 | | | | | | |
| | 858 | 790.203 | 1.3167 | 4.12 | **322** | 936 | 2611.793 | 17.3073 | |
| | | | | | | 881 | 654.782 | 1.091 | 17.83 |

(continued)

| # | ADDRESS | FREQUENCY | | INTENSITY | # | ADDRESS | FREQUENCY | | INTENSITY |
|---|---|---|---|---|---|---|---|---|---|
| **143** | 781 | 7163.191 | 47.4675 | | | | | | |
| | 874 | 695.441 | 1.1588 | 1.07 | **323** | 936 | 2611.793 | 17.3073 | |
| | | | | | | 902 | 531.793 | 0.8861 | 19.86 |
| **144** | 781 | 7163.191 | 47.4675 | | | | | | |
| | 881 | 656.256 | 1.0935 | 6.01 | **324** | 936 | 2611.793 | 17.3073 | |
| | | | | | | 909 | 490.194 | 0.8168 | 2.94 |
| **145** | 781 | 7163.191 | 47.4675 | | | | | | |
| | 891 | 594.695 | 0.9909 | 39.6 | **325** | 936 | 2611.793 | 17.3073 | |
| | | | | | | 920 | 427.649 | 0.7126 | 1.52 |
| **146** | 781 | 7163.191 | 47.4675 | | | | | | |
| | 909 | 489.613 | 0.8158 | 3.9 | **326** | 940 | 2489.586 | 16.4974 | |
| | | | | | | 662 | 1941.819 | 3.2355 | 2.46 |
| **147** | 786 | 7041.051 | 46.6581 | | | | | | |
| | 432 | 3290.762 | 5.4831 | 2.39 | **327** | 940 | 2489.586 | 16.4974 | |
| | | | | | | 664 | 1929.172 | 3.2144 | 1.76 |
| **148** | 786 | 7041.051 | 46.6581 | | | | | | |
| | 669 | 1898.191 | 3.1628 | 1.21 | **328** | 940 | 2489.586 | 16.4974 | |
| | | | | | | 822 | 1001.01 | 1.6679 | 1.2 |
| **149** | 786 | 7041.051 | 46.6581 | | | | | | |
| | 677 | 1850.653 | 3.0836 | 2.37 | **329** | 940 | 2489.586 | 16.4974 | |
| | | | | | | 864 | 757.021 | 1.2614 | 66 |
| **150** | 786 | 7041.051 | 46.6581 | | | | | | |
| | 680 | 1835.948 | 3.0591 | 2.84 | **330** | 940 | 2489.586 | 16.4974 | |
| | | | | | | 871 | 712.622 | 1.1874 | 10.06 |
| **151** | 786 | 7041.051 | 46.6581 | | | | | | |
| | 682 | 1821.518 | 3.0351 | 2.66 | **331** | 940 | 2489.586 | 16.4974 | |
| | | | | | | 880 | 660.618 | 1.1007 | 2.75 |
| **152** | 786 | 7041.051 | 46.6581 | | | | | | |
| | 689 | 1782.432 | 2.9699 | 2.02 | **332** | 940 | 2489.586 | 16.4974 | |
| | | | | | | 885 | 632.387 | 1.0537 | 18.76 |
| **153** | 786 | 7041.051 | 46.6581 | | | | | | |
| | 776 | 1274.214 | 2.1231 | 2.05 | **333** | 940 | 2489.586 | 16.4974 | |
| | | | | | | 891 | 594.244 | 0.9901 | 3.19 |

(continued)

| # | ADDRESS | FREQUENCY | | INTENSITY | # | ADDRESS | FREQUENCY | | INTENSITY |
|---|---|---|---|---|---|---|---|---|---|
| **154** | 786 | 7041.051 | 46.6581 | | | | | | |
| | 800 | 1129.576 | 1.8821 | 1.51 | **334** | 940 | 2489.586 | 16.4974 | |
| | | | | | | 899 | 548.142 | 0.9133 | 1.83 |
| **155** | 786 | 7041.051 | 46.6581 | | | | | | |
| | 804 | 1106.939 | 1.8444 | 6.33 | **335** | 940 | 2489.586 | 16.4974 | |
| | | | | | | 911 | 478.615 | 0.7975 | 8.03 |
| **156** | 786 | 7041.051 | 46.6581 | | | | | | |
| | 808 | 1082.787 | 1.8042 | 3.27 | **336** | 940 | 2489.586 | 16.4974 | |
| | | | | | | 913 | 466.429 | 0.7772 | 10.1 |
| **157** | 786 | 7041.051 | 46.6581 | | | | | | |
| | 811 | 1065.178 | 1.7748 | 3.51 | **337** | 940 | 2489.586 | 16.4974 | |
| | | | | | | 920 | 426.462 | 0.7106 | 1.57 |
| **158** | 786 | 7041.051 | 46.6581 | | | | | | |
| | 829 | 959.508 | 1.5988 | 3.1 | **338** | 945 | 2348.374 | 15.5617 | |
| | | | | | | 383 | 3576.612 | 5.9594 | 2.25 |
| **159** | 786 | 7041.051 | 46.6581 | | | | | | |
| | 838 | 906.837 | 1.511 | 1.42 | **339** | 945 | 2348.374 | 15.5617 | |
| | | | | | | 402 | 3464.839 | 5.7732 | 1.77 |
| **160** | 786 | 7041.051 | 46.6581 | | | | | | |
| | 848 | 847.28 | 1.4118 | 1.15 | **340** | 945 | 2348.374 | 15.5617 | |
| | | | | | | 769 | 1311.156 | 2.1847 | 9.92 |
| **161** | 786 | 7041.051 | 46.6581 | | | | | | |
| | 858 | 790.53 | 1.3172 | 49.82 | **341** | 945 | 2348.374 | 15.5617 | |
| | | | | | | 783 | 1233.208 | 2.0548 | 4.9 |
| **162** | 786 | 7041.051 | 46.6581 | | | | | | |
| | 874 | 696.77 | 1.161 | 1.14 | **342** | 945 | 2348.374 | 15.5617 | |
| | | | | | | 791 | 1184.039 | 1.9729 | 8.41 |
| **163** | 786 | 7041.051 | 46.6581 | | | | | | |
| | 881 | 656.443 | 1.0938 | 70.13 | **343** | 945 | 2348.374 | 15.5617 | |
| | | | | | | 804 | 1106.429 | 1.8436 | 5.06 |
| **164** | 786 | 7041.051 | 46.6581 | | | | | | |
| | 891 | 594.532 | 0.9906 | 18.99 | **344** | 945 | 2348.374 | 15.5617 | |
| | | | | | | 819 | 1018.365 | 1.6968 | 3.91 |

(continued)

| # | ADDRESS | FREQUENCY | | INTENSITY | # | ADDRESS | FREQUENCY | | INTENSITY |
|---|---|---|---|---|---|---|---|---|---|
| **165** | 786 | 7041.051 | 46.6581 | | | | | | |
| | 909 | 489.363 | 0.8154 | 21.1 | **345** | 945 | 2348.374 | 15.5617 | |
| | | | | | | 822 | 1001.834 | 1.6693 | 5.15 |
| **166** | 814 | 6218.377 | 41.2066 | | | | | | |
| | 431 | 3293.885 | 5.4883 | 1.45 | **346** | 945 | 2348.374 | 15.5617 | |
| | | | | | | 864 | 756.741 | 1.2609 | 6.6 |
| **167** | 814 | 6218.377 | 41.2066 | | | | | | |
| | 561 | 2531.574 | 4.2182 | 3.03 | **347** | 945 | 2348.374 | 15.5617 | |
| | | | | | | 872 | 710.417 | 1.1837 | 1.2 |
| **168** | 814 | 6218.377 | 41.2066 | | | | | | |
| | 564 | 2517.867 | 4.1953 | 3.26 | **348** | 945 | 2348.374 | 15.5617 | |
| **169** | 814 | 6218.377 | 41.2066 | | | 881 | 655.039 | 1.0914 | 2.79 |
| | 635 | 2096.944 | 3.494 | 2.64 | **349** | 945 | 2348.374 | 15.5617 | |
| | | | | | | 885 | 632.486 | 1.0539 | 2.24 |
| **170** | 814 | 6218.377 | 41.2066 | | | | | | |
| | 678 | 1846.079 | 3.076 | 2.51 | **350** | 945 | 2348.374 | 15.5617 | |
| | | | | | | 899 | 550.147 | 0.9167 | 19.61 |
| **171** | 814 | 6218.377 | 41.2066 | | | | | | |
| | 776 | 1274.484 | 2.1236 | 1.75 | **351** | 945 | 2348.374 | 15.5617 | |
| | | | | | | 906 | 510.518 | 0.8506 | 3.78 |
| **172** | 814 | 6218.377 | 41.2066 | | | | | | |
| | 783 | 1228.144 | 2.0464 | 1.41 | **352** | 945 | 2348.374 | 15.5617 | |
| | | | | | | 911 | 480.301 | 0.8003 | 8.74 |
| **173** | 814 | 6218.377 | 41.2066 | | | | | | |
| | 786 | 1211.726 | 2.019 | 1.51 | **353** | 945 | 2348.374 | 15.5617 | |
| | | | | | | 914 | 464.362 | 0.7737 | 8.49 |
| **174** | 814 | 6218.377 | 41.2066 | | | | | | |
| | 799 | 1136.142 | 1.8931 | 1.43 | **354** | 945 | 2348.374 | 15.5617 | |
| | | | | | | 920 | 426.538 | 0.7107 | 18.76 |
| **175** | 814 | 6218.377 | 41.2066 | | | | | | |
| | 804 | 1107.204 | 1.8448 | 67.13 | | | | | |
| **176** | 814 | 6218.377 | 41.2066 | | | | | | |

(continued)

| # | ADDRESS | FREQUENCY | | INTENSITY | # | ADDRESS | FREQUENCY | | INTENSITY |
|---|---|---|---|---|---|---|---|---|---|
| | 819 | 1018.301 | 1.6967 | 4.37 | | | | | |
| **177** | 814 | 6218.377 | 41.2066 | | | | | | |
| | 822 | 1001.489 | 1.6687 | 4.96 | | | | | |
| **178** | 814 | 6218.377 | 41.2066 | | | | | | |
| | 833 | 939.695 | 1.5657 | 3.66 | | | | | |
| **179** | 814 | 6218.377 | 41.2066 | | | | | | |
| | 864 | 756.271 | 1.2601 | 2.02 | | | | | |
| **180** | 814 | 6218.377 | 41.2066 | | | | | | |
| | 874 | 695.963 | 1.1596 | 2.98 | | | | | |

Table 5.4 (Y-structure and 2D NMR chemical shift data) shows the assignment of functional groups derived from the chemical shifts of proton and carbon-13 of compound Y. Based on these data and analysis, the structure of compound Y is assigned and shown below.

Compound Y

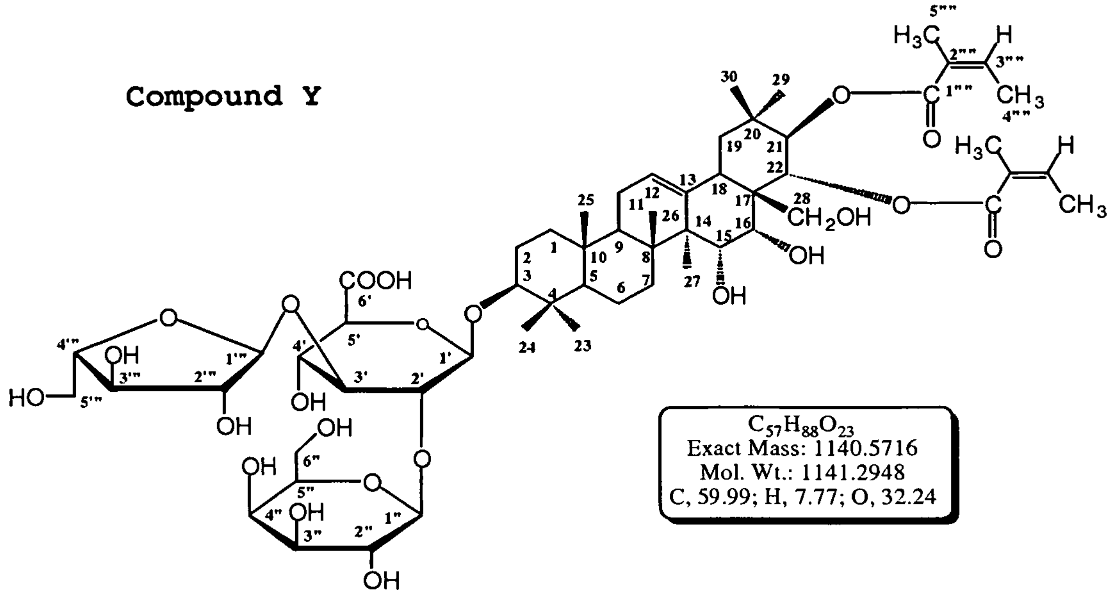


Structure 1

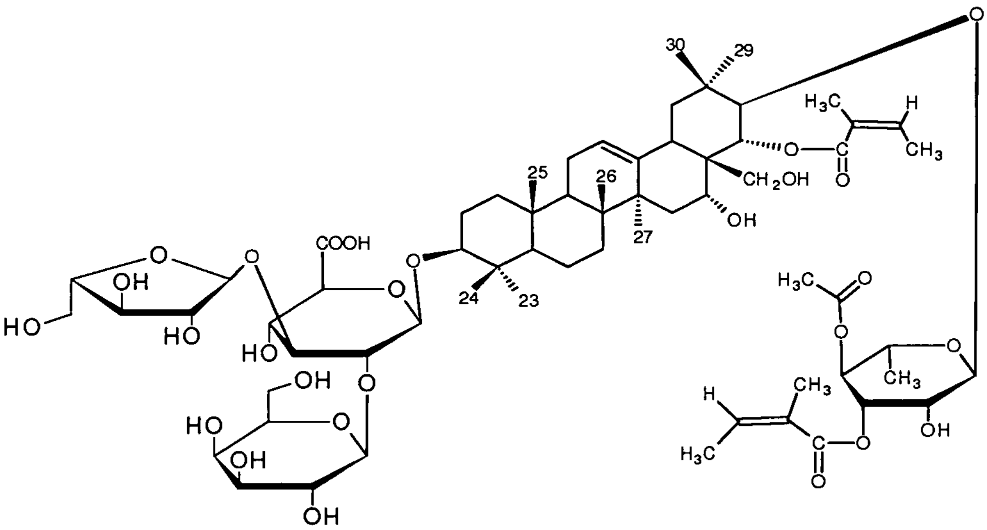

(continued)

Structure 2

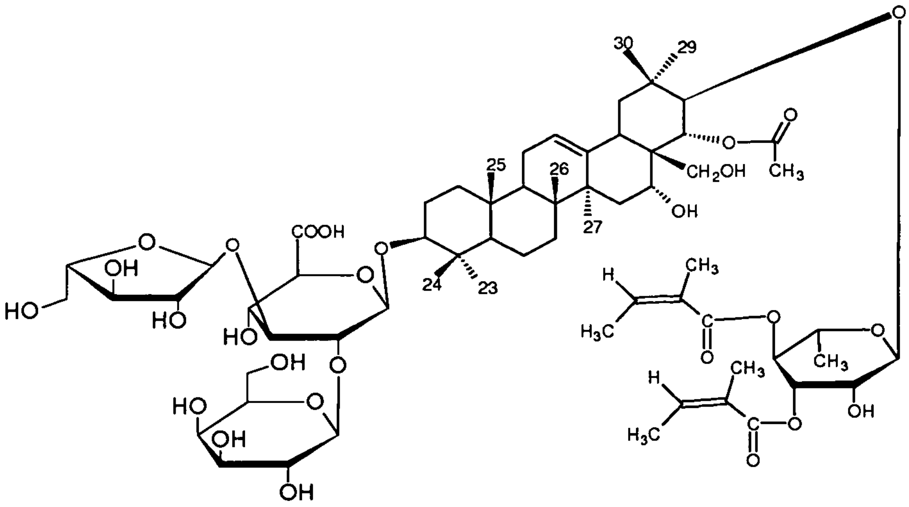

Structure 3

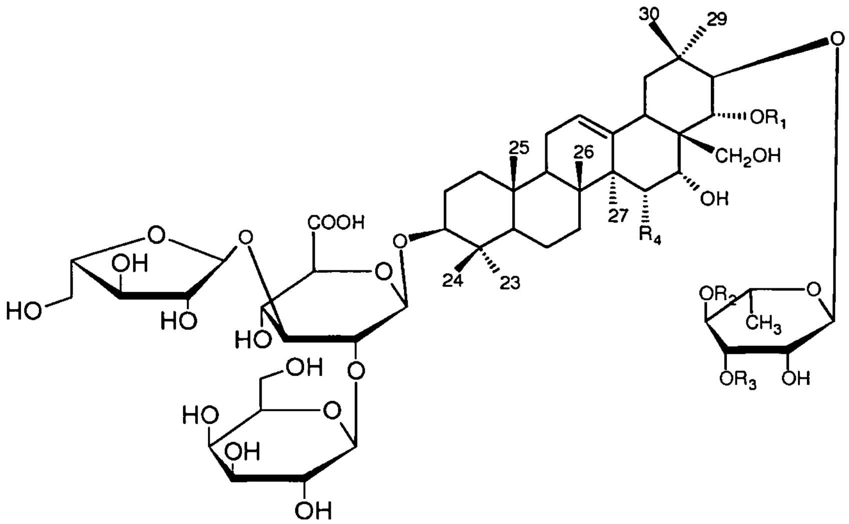

Wherein R 1 =A or B or C; R2 =A or B or C; R3 =A or B or C; R4 =H or OH; A=angeloyl group; B = acetyl group; C= Hydrogen

| | R1 | R2 | R3 |
|---|---|---|---|
| 1 | A | A | A |
| 2 | A | A | B |
| 3 | A | A | C |
| 4 | A | B | A |
| 5 | A | B | B |
| 6 | A | B | C |
| 7 | A | C | A |
| 8 | A | C | B |
| 9 | A | C | C |
| 10 | B | A | A |

(continued)

| | R1 | R2 | R3 |
|---|---|---|---|
| 11 | B | A | B |
| 12 | B | A | C |
| 12 | B | B | A |
| 14 | B | B | B |
| 15 | B | B | C |
| 16 | B | C | A |
| 17 | B | C | B |
| 18 | B | C | C |
| 19 | C | A | A |
| 20 | C | A | B |
| 21 | C | A | C |
| 22 | C | B | A |
| 23 | C | B | B |
| 24 | C | B | C |
| 25 | C | C | A |
| 26 | C | C | B |
| 27 | C | C | C |

;

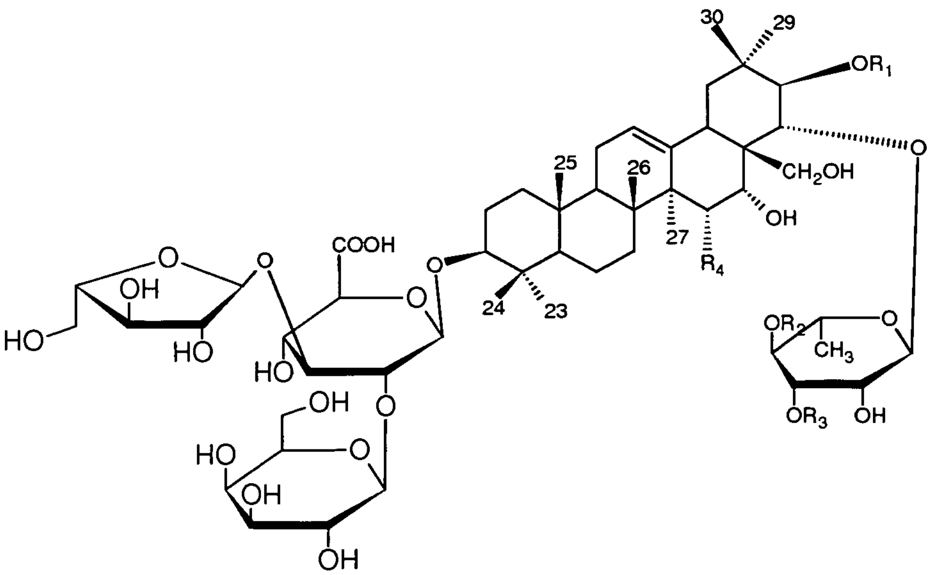

Wherein R1 =A or B or C; R2 =A or B or C; R3 =A or B or C; R4 =H or OH A=angeloyl group; B = acetyl group; C= Hydrogen

| | R1 | R2 | R3 |
|---|---|---|---|
| 1 | A | A | A |

(continued)

| | R1 | R2 | R3 |
|---|---|---|---|
| 2 | A | A | B |
| 3 | A | A | C |
| 4 | A | B | A |
| 5 | A | B | B |
| 6 | A | B | C |
| 7 | A | C | A |
| 8 | A | C | B |
| 9 | A | C | C |
| 10 | B | A | A |
| 11 | B | A | B |
| 12 | B | A | C |
| 13 | B | B | A |
| 14 | B | B | B |
| 15 | B | B | C |
| 16 | B | C | A |
| 17 | B | C | B |
| 18 | B | C | C |
| 19 | C | A | A |
| 20 | C | A | B |
| 21 | C | A | C |
| 22 | C | B | A |
| 23 | C | B | B |
| 24 | C | B | C |
| 25 | C | C | A |
| 26 | C | C | B |
| 27 | C | C | C |

**Structure of Compound Y:**

The chemical name of compound Y is:

3-O-[β-D-galactopyranosyl(1→2)]-α-L-arabinofuranosyl(1→3)-β-D-glucuronopyranosyl-21,22-O-diangeloyl-3β, 15α, 160α, 21β, 22α, 28-hexahydroxyolean-12-ene

**Table 5.4. $^{13}$C and $^{1}$H NMR data for compound Y (in Pyridine-$d_5$)[a]**

| Position | C | H | Key HMBC correlations |
|---|---|---|---|
| 1 | 38.7 | 0.83, 1.40 | C-3, C-5, C-9 |
| 2 | 26.4 | 1.81, 2.14 | - |
| 3 | 89.6 | 3.25, 1H, dd, 12.0/4.0 Hz | C-23, C-24, GlcA C-1' |
| 4 | 39.4 | - | - |

(continued)

| Position | C | H | Key HMBC correlations |
|---|---|---|---|
| 5 | 55.3 | 0.78 | - |
| 6 | 18.5 | 1.55, 1.59 | C-8, C-10 |
| 7 | 36.5 | 2.00, 2.10 | C-5, C-9 |
| 8 | 41.2 | - | - |
| 9 | 47.0 | 3.06 | C-7, C-8, C-12, C-14, C-26 |
| 10 | 37.2 | - | - |
| 11 | 23.7 | 1.74, 1.89 | - |
| 12 | 125.2 | 5.49, 1H, br s | C-9, C-11, C-14, C-18 |
| 13 | 143.4 | - | - |
| 14 | 47.5 | - | - |
| 15 | 67.3 | 4.21 | C-8, C-27 |
| 16 | 73.6 | 4.45 | C-14, C-15, C-18 |
| 17 | 48.3 | - | - |
| 18 | 40.8 | 3.07 | C-12, C-13, C-14, C-16, C-19, C-20, C-28, |
| 19 | 46.8 | 1.41, 1.69 | - |
| 20 | 36.2 | - | - |
| 21 | 79.3 | 6.71, 1H, d, 10 Hz | C-20, C-22, C-29, C-30, 21-*O*-Ang C-1"" |
| 22 | 73.5 | 6.32, 1H, d, 10 Hz | C-16, C-17, C-21, C-28, 22-*O*-Ang C-1"" |
| 23 | 27.7 | 1.26, 3H, s | C-3, C-4, C-5, C-24 |
| 24 | 16.5 | 1.16, 3H, s | C-3, C-4, C-5, C-23 |
| 25 | 16.0 | 0.81, 3H, s | C-1, C-5, C-9, C-10 |
| 26 | 17.3 | 0.99, 3H, s | C-7, C-8, C-9, C-14 |
| 27 | 21.0 | 1.85, 3H, s | C-8, C-13, C-14, C-15 |
| 28 | 62.9 | 3.50, 1H, d, 11.0 Hz, 3.76, 1H, d, 11.0 Hz, | C-16, C-17, C-18, C-22 |
| 29 | 29.2 | 1.09, 3H, s | C-19, C-20, C-21, C-30 |
| 30 | 20.0 | 1.32, 3H, s | C-19, C-20, C-21, C-29 |
| GlcA | | | |
| 1’ | 104.9 | 4.89, 1H, d, 7.8 Hz | C-3 |
| 2’ | 79.1 | 4.38 | GlcA C-1’, C-3’, Gal C-1" |
| 3’ | 86.1 | 4.20 | GlcA C-2’, C-4’, Ara C-1’” |
| 4’ | 71.5 | 4.42 | GlcA C-3’, C-5’, C-6’ |
| 5’ | 78.0 | 4.52 | GlcA C-4’, C-6’ |
| 6’ | 171.9 | - | - |
| Gal | | | |
| 1" | 104.6 | 5.32, 1H, d, 7.7 Hz | GlcA C-2’ |
| 2" | 73.6 | 4.42 | Gal C-1", C-3" |
| 3" | 74.9 | 4.10 | Gal C-2" |
| 4" | 69.5 | 4.56 | Gal C-2", C-3" |

(continued)

| Position | C | H | Key HMBC correlations |
|---|---|---|---|
| 5" | 76.4 | 3.94 | Gal C-4", C-6" |
| 6" | 61.6 | 4.43, 4.52 | Gal C-4", C-5" |
| Ara-f | | | |
| 1"' | 110.6 | 6.03. 1H, br s | GlcA C-3', Ara C-2"', C-4''' |
| 2''' | 83.4 | 4.94 | Ara C-3"' |
| 3"' | 78.3 | 4.78 | Ara C-2''' |
| 4''' | 85.2 | 4.82 | Ara C-5''' |
| 5''' | 62.2 | 4.12, 4.28 | Ara C-3''' |
| 21-*O*-Ang | | | |
| 1"" | 167.7 | - | - |
| 2"" | 129.6 | - | - |
| 3"" | 137.2 | 5.96, 1H, dq, 7.0/1.5 Hz | Ang C-1"", C-4"", C-5"" |
| 4"" | 15.5 | 2.10, 3H, dq, 7.0/1.5 | Ang C-2"", C-3"" |
| | | Hz | |
| 5"" | 20.8 | 2.00, 3H, s | Ang-1"", C-2"", C-3"" |
| 22-*O*-Ang | | | |
| 1"" | 167.9 | - | - |
| 2"" | 129.8 | - | - |
| 3"" | 136.3 | 5.78, 1H, dq, 7.0/1.5 Hz | Ang C-1"", C-4"", C-5"" |
| 4"" | 15.5 | 1.93, 3H, dq, 7.0/1.5 Hz | Ang C-2"", C-3"" |
| 5"" | 20.5 | 1.74, 3H, s | Ang C-1"", C-2"", C-3"" |
| [a] The data were assigned based on HMQC and HMBC correlations. | | | |

Figure 25 shows the mass spectrum of Y as determined by MALDI-TOF and ESI-MS techniques.

## Conclusion

Based on the chemical shift analysis, the active compound Y isolated from extract of Xanthoceras Sorbifolia is a triterpenoid saponins with three sugars and biangeloyl groups attached to the backbone. The formula of Y is C57H88O23, and the chemical name of Y is:

3-O-[β-D-galactopyranosyl(1→2)]-α-L-arabinofuranosyl(1→3)-β-D-glucuronopyranosyl-21,22-O-diangeloyl-3β, 15α, 16α, 21β, 22α, 28-hexahydroxyolean-12-ene

## Claims

**1.** A compound for use in a method of treating ovarian cancer, having the chemical formula Y:

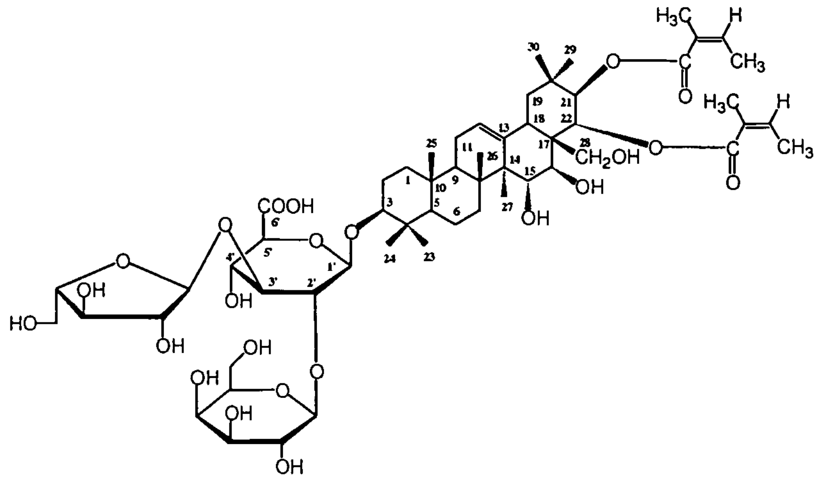

(Y)

which is designated 3-*O*-[β-D-galactopyranosyl(1→2)]-α-L-arabinofuranosyl(1→3)-β-D-glucuronopyranosyl-21,22-*O*-diangeloyl-3β, 15α, 16α, 21β, 22α, 28-hexahydroxyolean-12-ene, or a salt thereof.

**2.** The compound of claim 1 for use according to claim 1, isolated from a biological source, including *Xanthoceras sorbifolia.*

**3.** The compound of claim 2 for use according to claim 1, wherein the biological source is the husk, branch, fruit-stem, leaf, kernel, root, stem, or bark of *Xanthoceras Sorbifolia.*

**4.** The compound of claim 1 for use according to claim 1, wherein the compound is obtainable by a method comprising the steps of:

(a) Extracting *Xanthoceras sorbifolia* powder with one or more organic solvent to obtain an organic extract;
(b) Collecting the organic extract;
(c) Refluxing the organic extract with an organic solvent to obtain a second extract;
(d) Removing the organic solvent from the second extract;
(e) Drying and sterilizing the second extract to form a *Xanthoceras Sorbifolia* extract powder;
(f) Fractionating the extract powder to obtain one or more components of the extract powder;
(g) Identifying the bioactive components of the extract powder;
(h) Purifying one or more bioactive components of the extract powder with FPLC to obtain one or more fraction of the bioactive component; and
(i) Isolating the compound with preparative HPLC.

**5.** The compound of claim 4 for use according to claim 1, wherein the powders are made from the husk, branch, stem, leave, kernel, root, bark or seed shell of *Xanthoceras Sorbifolia.*

**6.** The compound of claim 4 for use according to claim 1, wherein the organic solvent is ethanol, methanol, ether, chloroform or acetone.

**7.** The compound of claim 4 for use according to claim 1, wherein the ratio of *Xanthoceras sorbifolia* powder to the organic solvent in step (a) is 1:2.

**8.** The compound of claim 4 for use according to claim 1, wherein the extraction step in (a) is performed 4-5 times for 20-35 hours each time.

**9.** The compound of claim 4 for use according to claim 1, wherein step (c) is carried out at 80°C.

**10.** The compound of claim 4 for use according to claim 1, wherein the refluxing step (c) is performed 2-3 times.

**11.** A composition for use in a method of treating ovarian cancer comprising an effective amount of the compound of any one of claims 1-10.

12. A pharmaceutical composition for use in a method of treating ovarian cancer comprising an effective amount of the compound of any one of claims 1-10, and a pharmaceutically acceptable carrier.

## Patentansprüche

1. Verbindung zur Verwendung in einem Verfahren zur Behandlung von Eierstockkrebs mit der folgenden chemischen Formel Y:

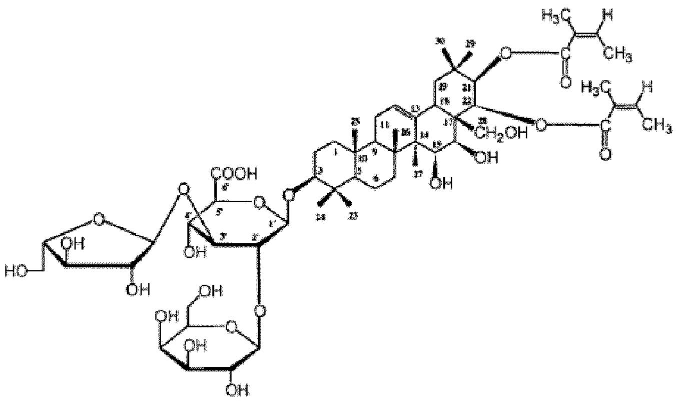

(Y)

die als 3-O-[β-D-Galactopyranosyl(1->2)]-α-L-arabinofuranosyl(1->3)-β-D-glucuronopyranosyl-21,22-O-diangeloyl-3β, 15α, 16α, 21β, 22α, 28-hexahydroxyolean-12-en bezeichnet wird, oder ein Salz davon.

2. Verbindung nach Anspruch 1 zur Verwendung nach Anspruch 1, isoliert aus einer biologischen Quelle, darin eingeschlossen Xanthoceras sorbifolia.

3. Verbindung nach Anspruch 2 zur Verwendung nach Anspruch 1, wobei die biologische Quelle die Schale, der Zweig, der Fruchtstiel, das Blatt, der Kern, die Wurzel, der Stamm oder die Rinde von Xanthoceras sorbifolia ist.

4. Verbindung nach Anspruch 1 zur Verwendung nach Anspruch 1, wobei die Verbindung durch ein Verfahren erhalten werden kann, das die folgenden Schritte umfasst:

(a) Extrahieren von Xanthoceras sorbifolia-Pulver mit einem oder mit mehreren organischen Lösemitteln, um einen organischen Extrakt zu erhalten;
(b) Sammeln des organischen Extrakts;
(c) Refluxieren des organischen Extrakts mit einem organischen Lösemittel, um einen zweiten Extrakt zu erhalten;
(d) Entfernen des organischen Lösemittels vom zweiten Extrakt;
(e) Trocknen und Sterilisieren des zweiten Extrakts, um ein Xanthoceras Sorbifolia-Extraktpulver zu bilden;
(f) Fraktionieren des Extraktpulvers, um eine oder mehrere Komponenten des Extraktpulvers zu erhalten;
(g) Identifizieren der bioaktiven Komponenten des Extraktpulvers;
(h) Reinigen einer oder mehrerer bioaktiver Komponenten des Extraktpulvers mit FPLC, um eine oder mehrere Fraktionen der bioaktiven Komponente zu erhalten; und
(i) Isolieren der Komponente mit präparativer HPLC.

5. Verbindung nach Anspruch 4 zur Verwendung nach Anspruch 1, wobei die Pulver aus der Schale, dem Zweig, dem Stamm, dem Blatt, dem Kern, der Wurzel, der Rinde oder der Samenschale von Xanthoceras sorbifolia hergestellt sind.

6. Verbindung nach Anspruch 4 zur Verwendung nach Anspruch 1, wobei das organische Lösemittel Ethanol, Methanol, Ether, Chloroform oder Aceton ist.

7. Verbindung nach Anspruch 4 zur Verwendung nach Anspruch 1, wobei das Verhältnis von Xanthoceras sorbifolia-Pulver zum organischen Lösemittel in Schritt (a) 1:2 ist.

8. Verbindung nach Anspruch 4 zur Verwendung nach Anspruch 1, wobei der Schritt des Extrahierens (a) 4 - 5 Mal während jeweils 20 - 35 Stunden durchgeführt wird.

9. Verbindung nach Anspruch 4 zur Verwendung nach Anspruch 1, wobei Schritt (c) bei 80 °C ausgeführt wird.

10. Verbindung nach Anspruch 4 zur Verwendung nach Anspruch 1, wobei der Schritt des Refluxierens (c) 2 - 3 Mal durchgeführt wird.

11. Zusammensetzung zur Verwendung bei einem Verfahren zur Behandlung von Eierstockkrebs, umfassend eine wirksame Menge der Verbindung eines der Ansprüche 1 - 10.

12. Pharmazeutische Zusammensetzung zur Verwendung bei einem Verfahren zur Behandlung von Eierstockkrebs, umfassend eine wirksame Menge der Verbindung nach einem der Ansprüche 1 - 10 und einen pharmazeutisch akzeptablen Trägerstoff.

**Revendications**

1. Composé pour son utilisation dans un procédé de traitement du cancer de l'ovaire, ayant la formule chimique Y :

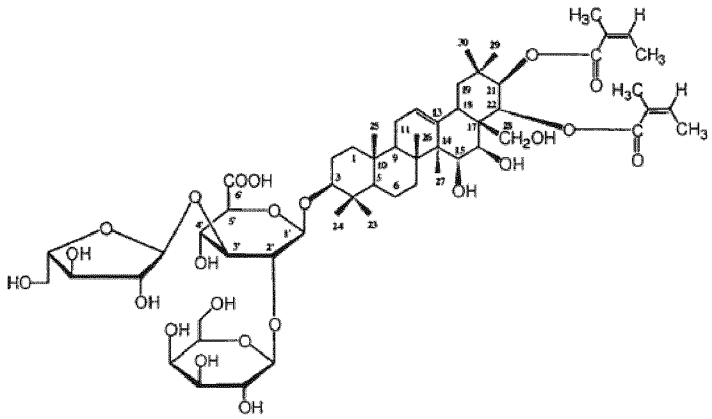

(Y)

qui est nommé 3-*O*-[β-D-galactopyranosyl(1→2)]-α-L-arabinofuranosyl(1→3)-β-D-glucuronopyranosyl-21,22-*O*-diangéloyl-3β, 15α, 16α, 21β, 22α, 28-hexahydroxyoléan-12-ène, ou un sel de celui-ci.

2. Composé selon la revendication 1 pour son utilisation selon la revendication 1, isolé d'une source biologique comprenant *Xanthoceras sorbifolia.*

3. Composé selon la revendication 2 pour son utilisation selon la revendication 1, dans lequel la source biologique est la capsule, la branche, le pédoncule, la feuille, la graine, la racine, la tige ou l'écorce de *Xanthoceras Sorbifolia.*

4. Composé selon la revendication 1 pour son utilisation selon la revendication 1, dans lequel le composé peut être obtenu par un procédé comprenant les étapes suivantes :

(a) extraction de poudre de *Xanthoceras Sorbifolia* avec un ou plusieurs solvants organiques pour obtenir un extrait organique ;
(b) recueil de l'extrait organique ;
(c) chauffage à reflux de l'extrait organique avec un solvant organique pour obtenir un deuxième extrait ;
(d) retrait du solvant organique du deuxième extrait ;
(e) séchage et stérilistion du deuxième extrait pour former une poudre d'extrait de *Xanthoceras Sorbifolia* ;
(f) fractionnement de la poudre d'extrait pour obtenir un ou plusieurs composants de la poudre d'extrait ;
(g) identification des composants bioactifs de la poudre d'extrait ;
(h) purification d'un ou plusieurs composants bioactifs de la poudre d'extrait avec une FPLC pour obtenir une ou plusieurs fractions du composant bioactif ; et
(i) isolement du composé avec une HPLC préparatoire.

5. Composé selon la revendication 4 pour son utilisation selon la revendication 1, dans lequel les poudres sont produites à partir de la capsule, de la branche, de la tige, de la feuille, de la graine, de la racine, de l'écorce ou de l'enveloppe de graine de *Xanthoceras Sorbifolia.*

6. Composé selon la revendication 4 pour son utilisation selon la revendication 1, dans lequel le solvant organique est l'éthanol, le méthanol, l'éther, le chloroforme ou l'acétone.

7. Composé selon la revendication 4 pour son utilisation selon la revendication 1, dans lequel le rapport de poudre de *Xanthoceras Sorbifolia* au solvant organique dans l'étape (a) est de 1 : 2.

8. Composé selon la revendication 4 pour son utilisation selon la revendication 1, dans lequel l'étape d'extraction du point (a) est réalisée 4 à 5 fois pendant 20 à 25 heures chaque fois.

9. Composé selon la revendication 4 pour son utilisation selon la revendication 1, dans lequel l'étape (c) est réalisée à 80 °C.

10. Composé selon la revendication 4 pour son utilisation selon la revendication 1, dans lequel l'étape de chauffage à reflux (c) est réalisée 2 à 3 fois.

11. Composition pour son utilisation dans un procédé de traitement du cancer de l'ovaire, comprenant une quantité efficace du composé selon l'une quelconque des revendications 1 à 10.

12. Composition pharmaceutique pour son utilisation dans un procédé de traitement du cancer de l'ovaire comprenant une quantité efficace du composé selon les revendications 1 à 10 et un véhicule pharmaceutiquement acceptable.

Figure 1

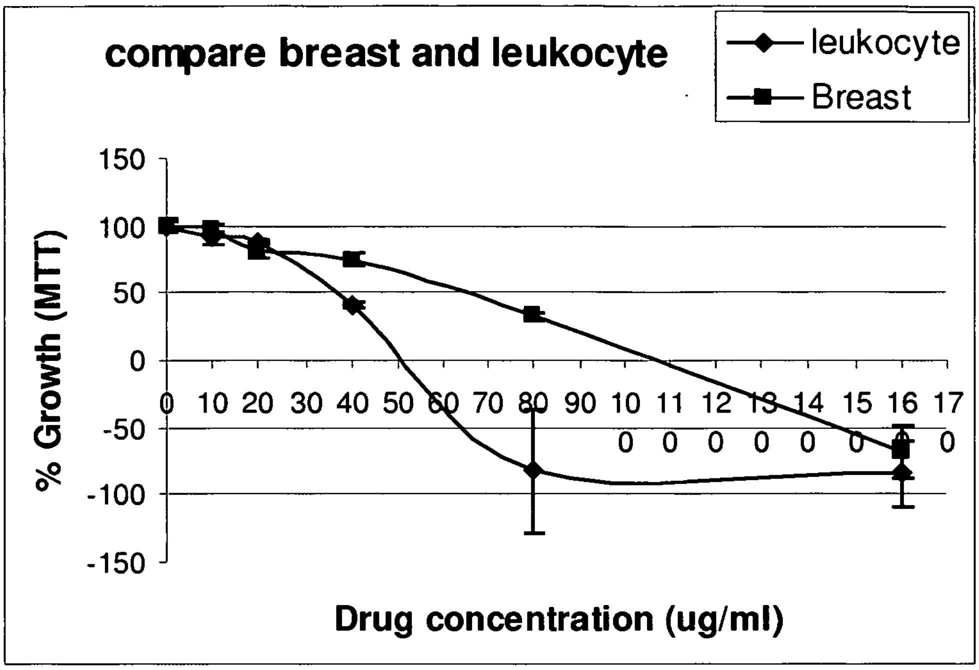


Figure 2

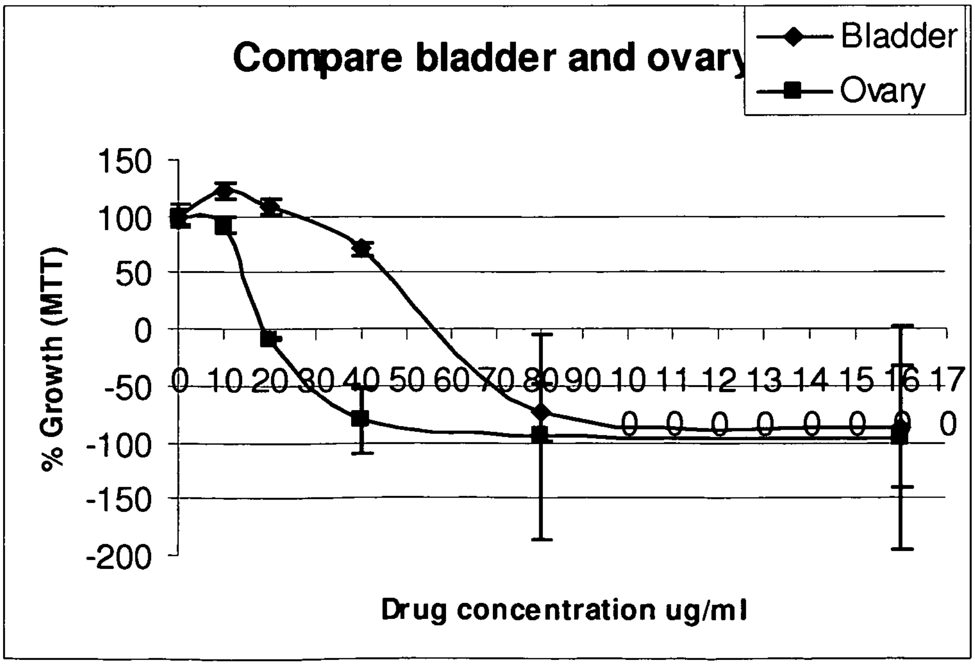

**Figure 3**

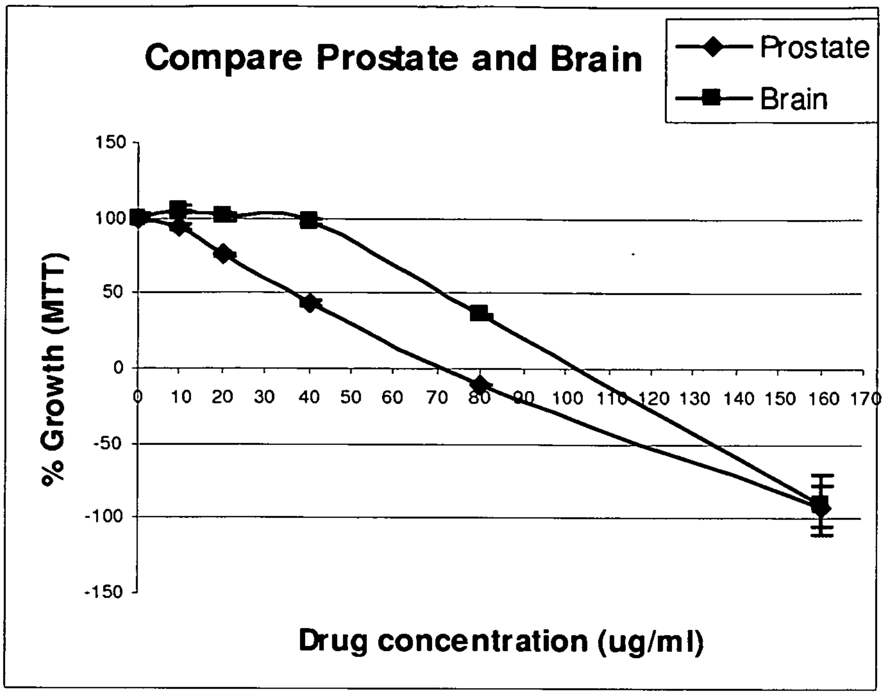


**Figure 4**

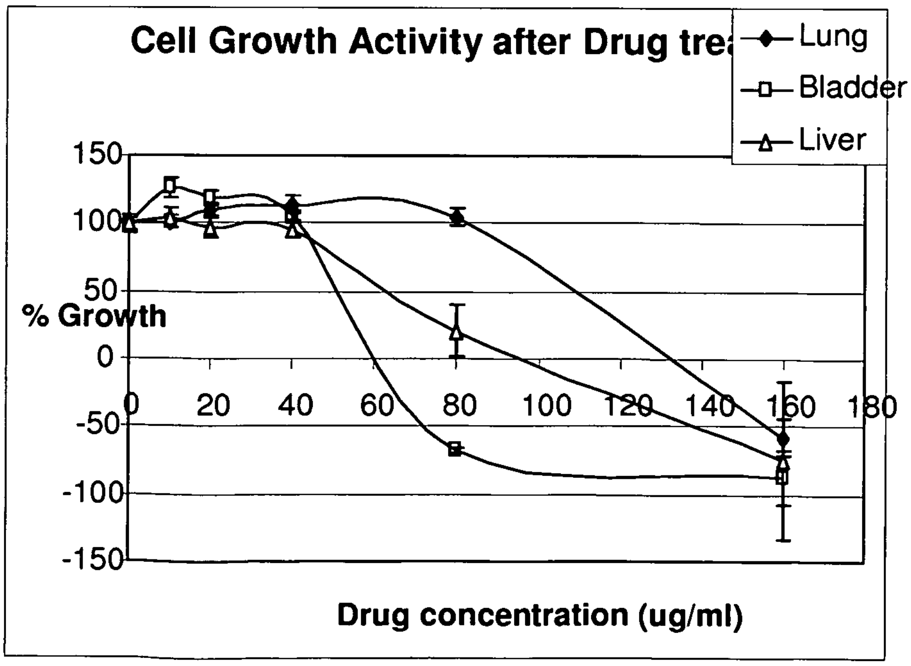

**Figure 5**

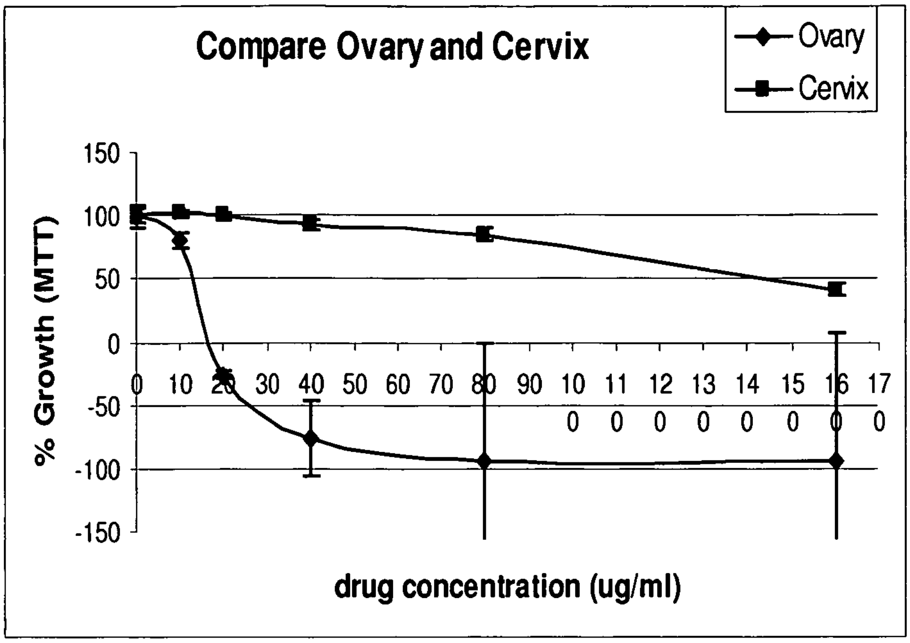


**Figure 6**

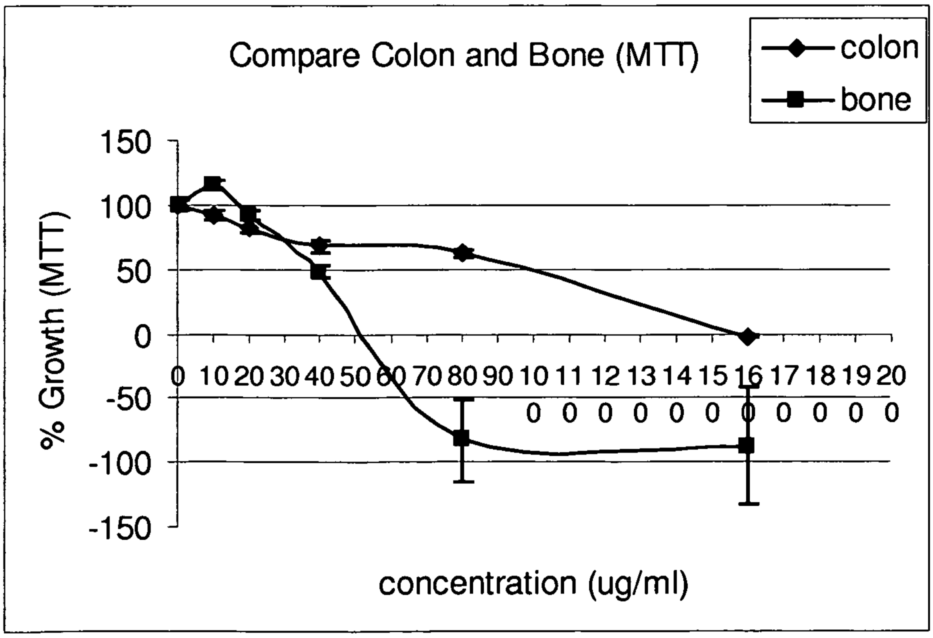

Figure 7

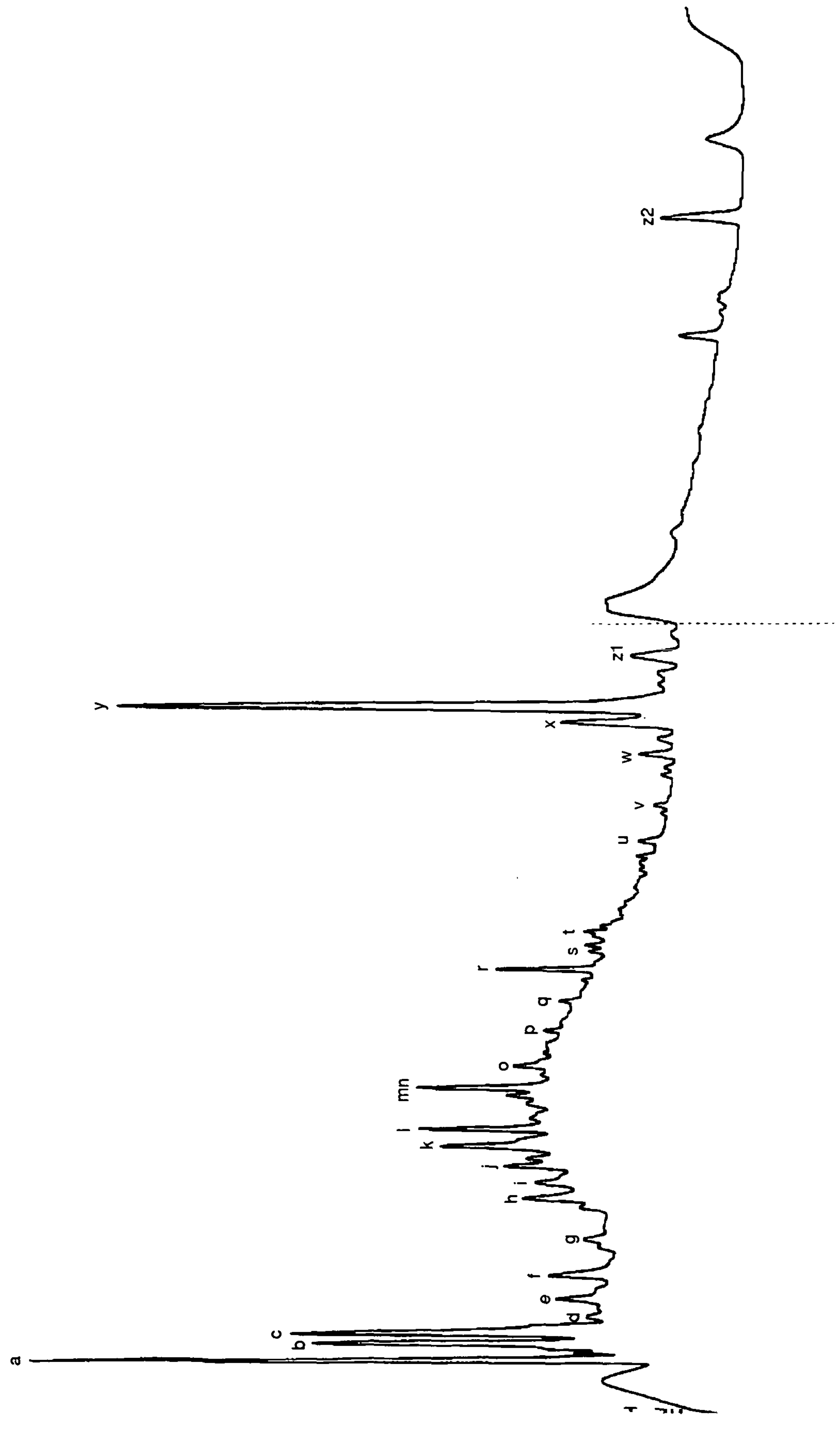

Figure 8A-B

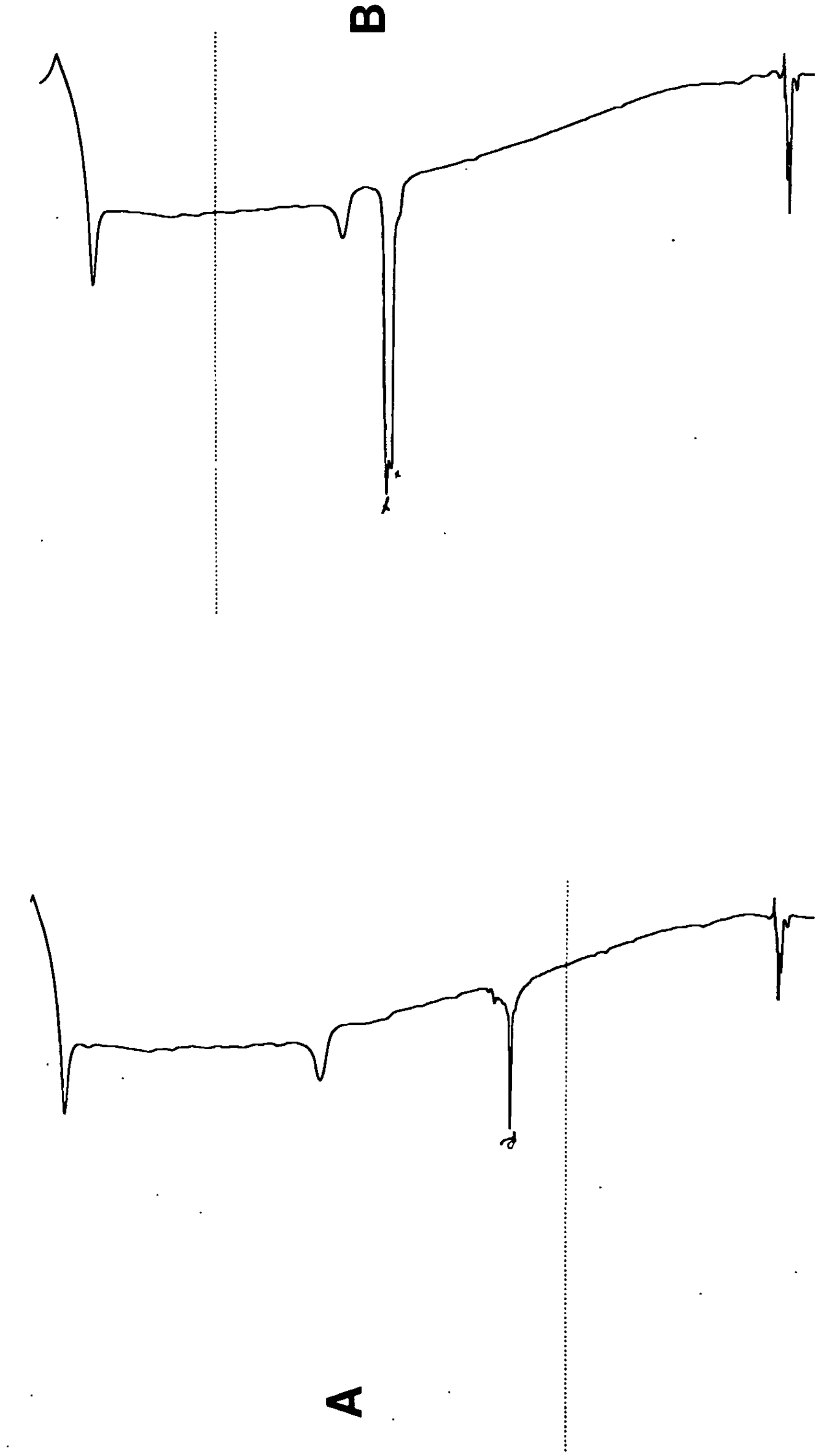

Figure 9

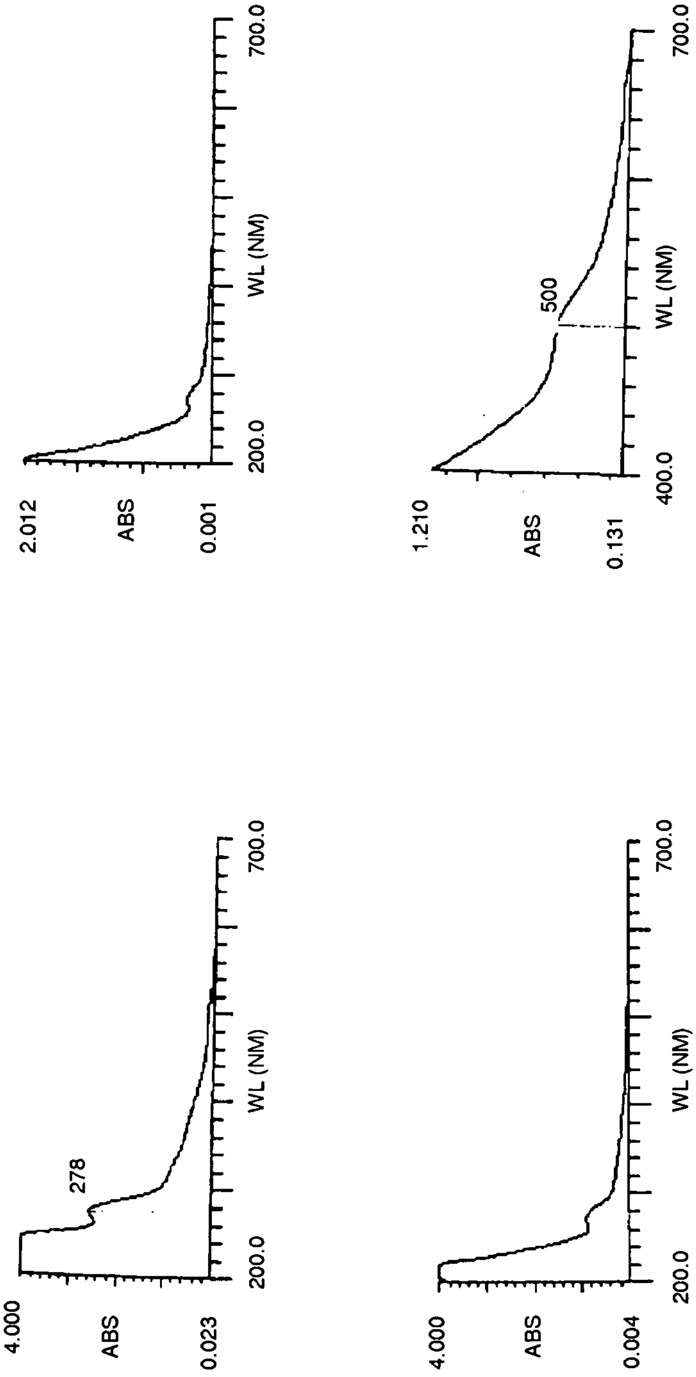
2.012
ABS
0.001
200.0
WL (NM)
700.0
1.210
ABS
0.131
400.0
500
WL (NM)
700.0
4.000
ABS
0.023
200.0
278
WL (NM)
700.0
4.000
ABS
0.004
200.0
WL (NM)
700.0

Figure 10A

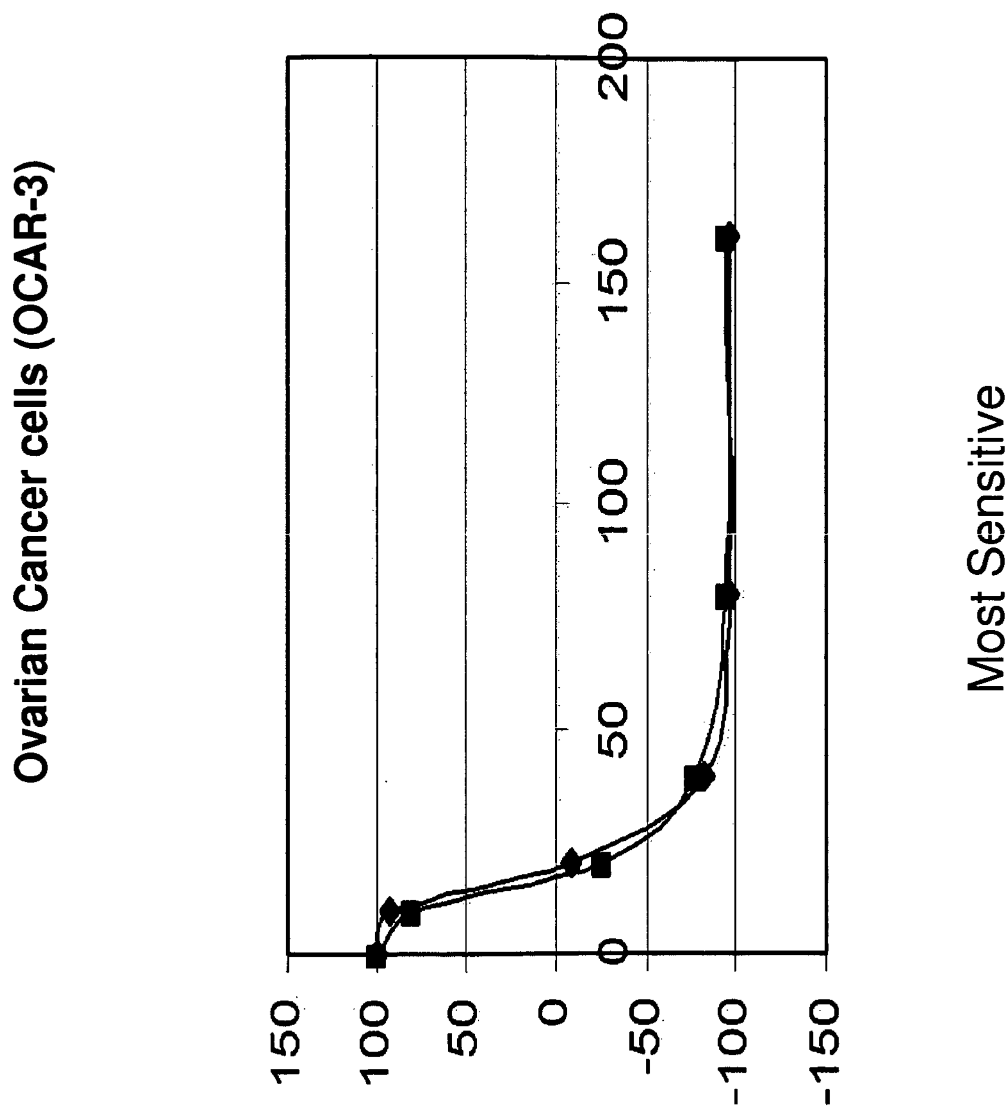
Ovarian Cancer cells (OCAR-3)
150
100
50
0
-50
-100
-150
0
50
100
150
200
Most Sensitive

Figure 10B

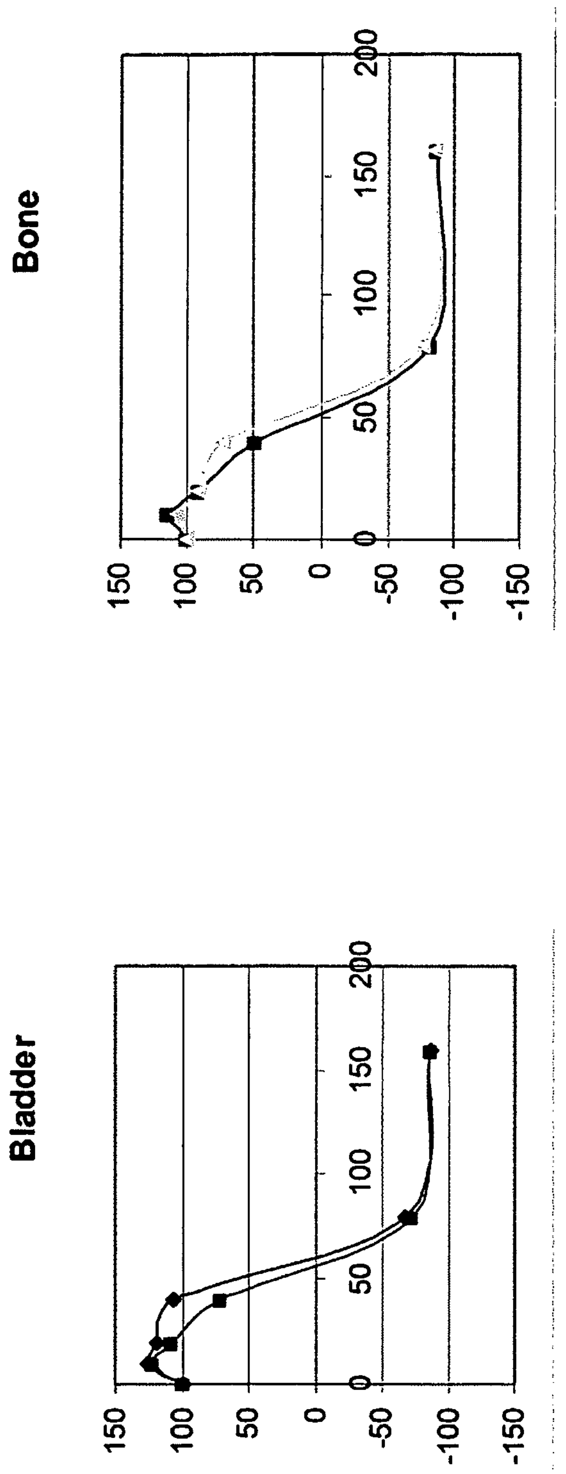


Sensitive

Figure 10C

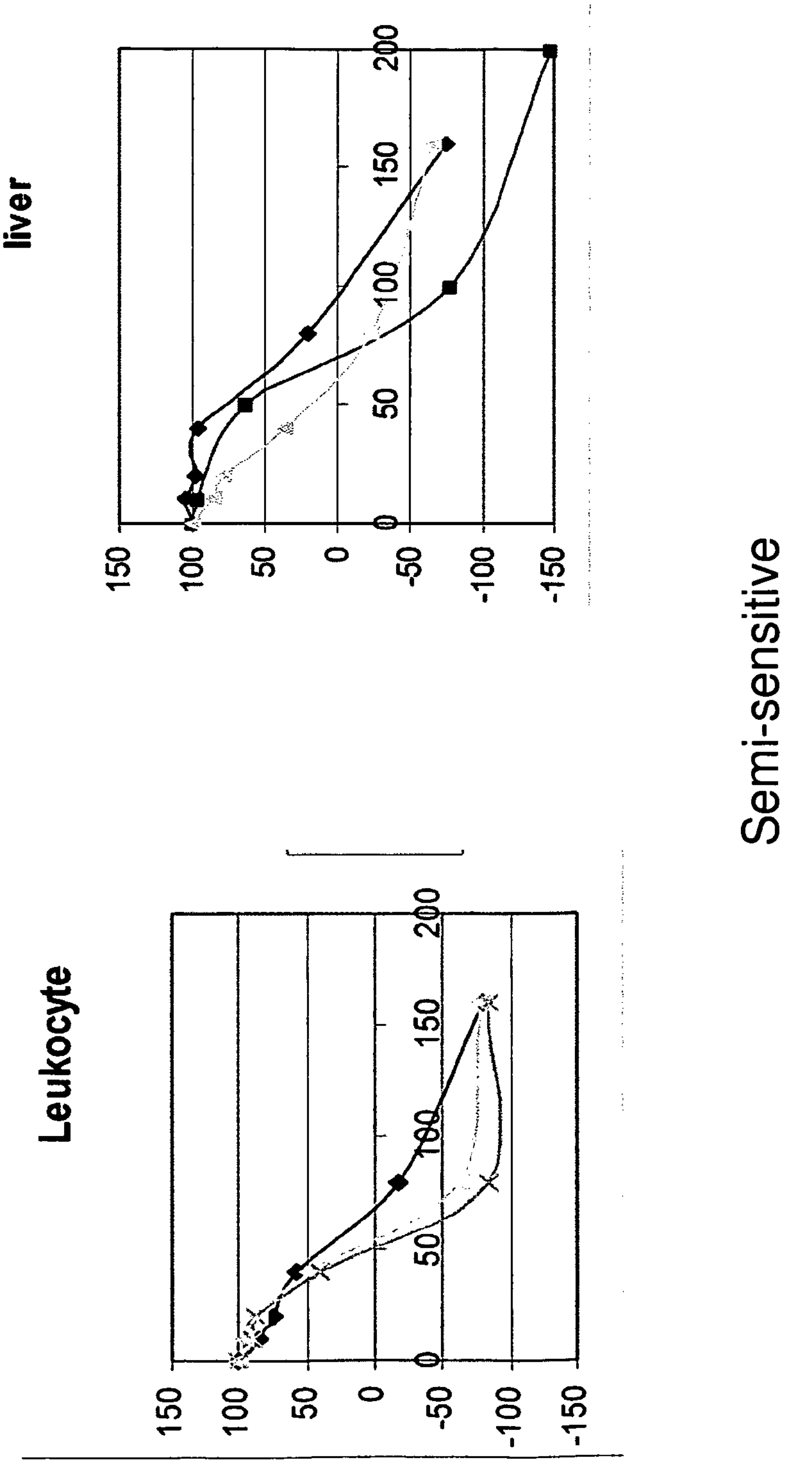
liver
Leukocyte
Semi-sensitive
150
100
50
0
-50
-100
-150
0
50
100
150
200

Figure 10D

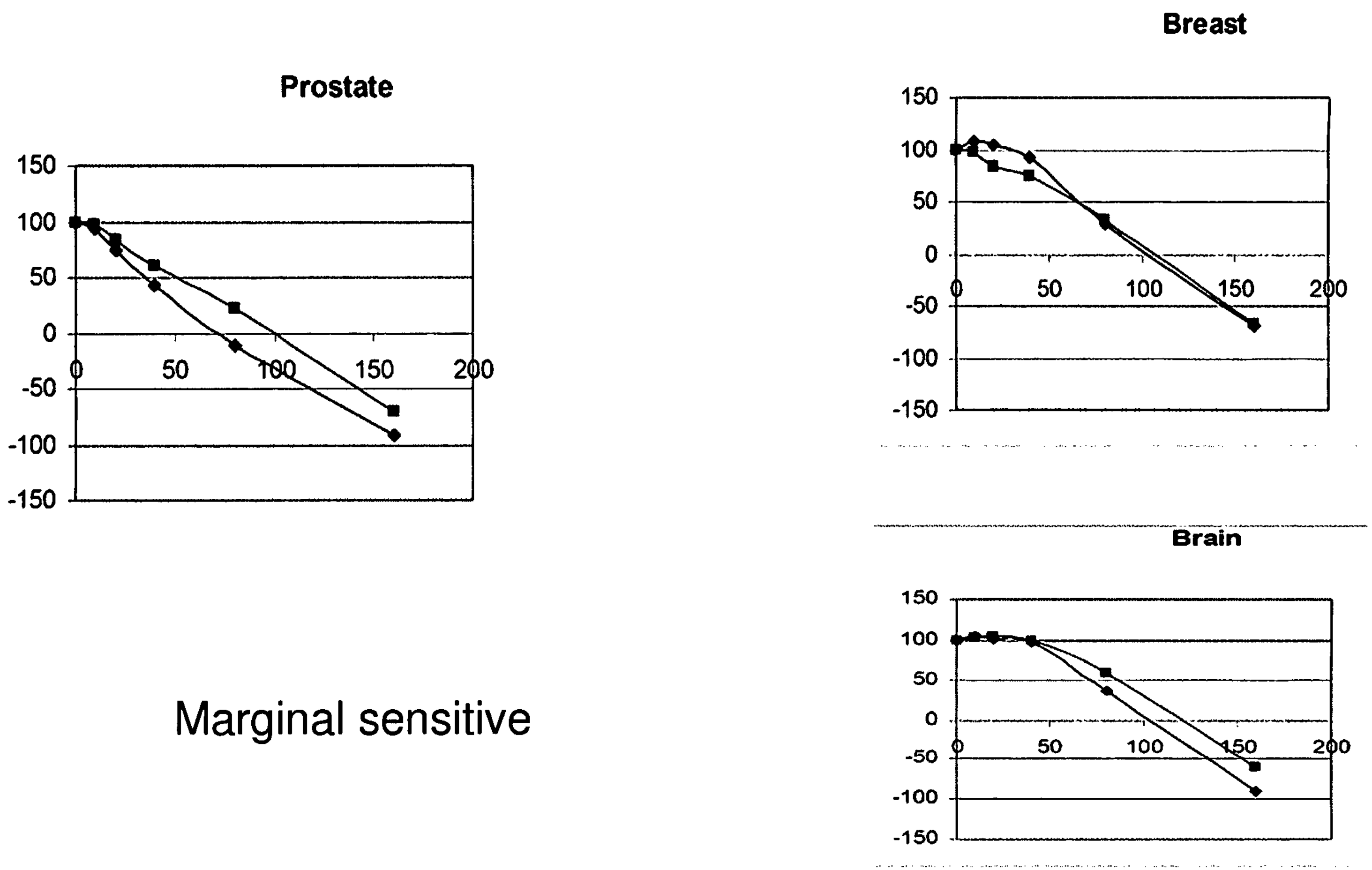


Marginal sensitive

Figure 10E

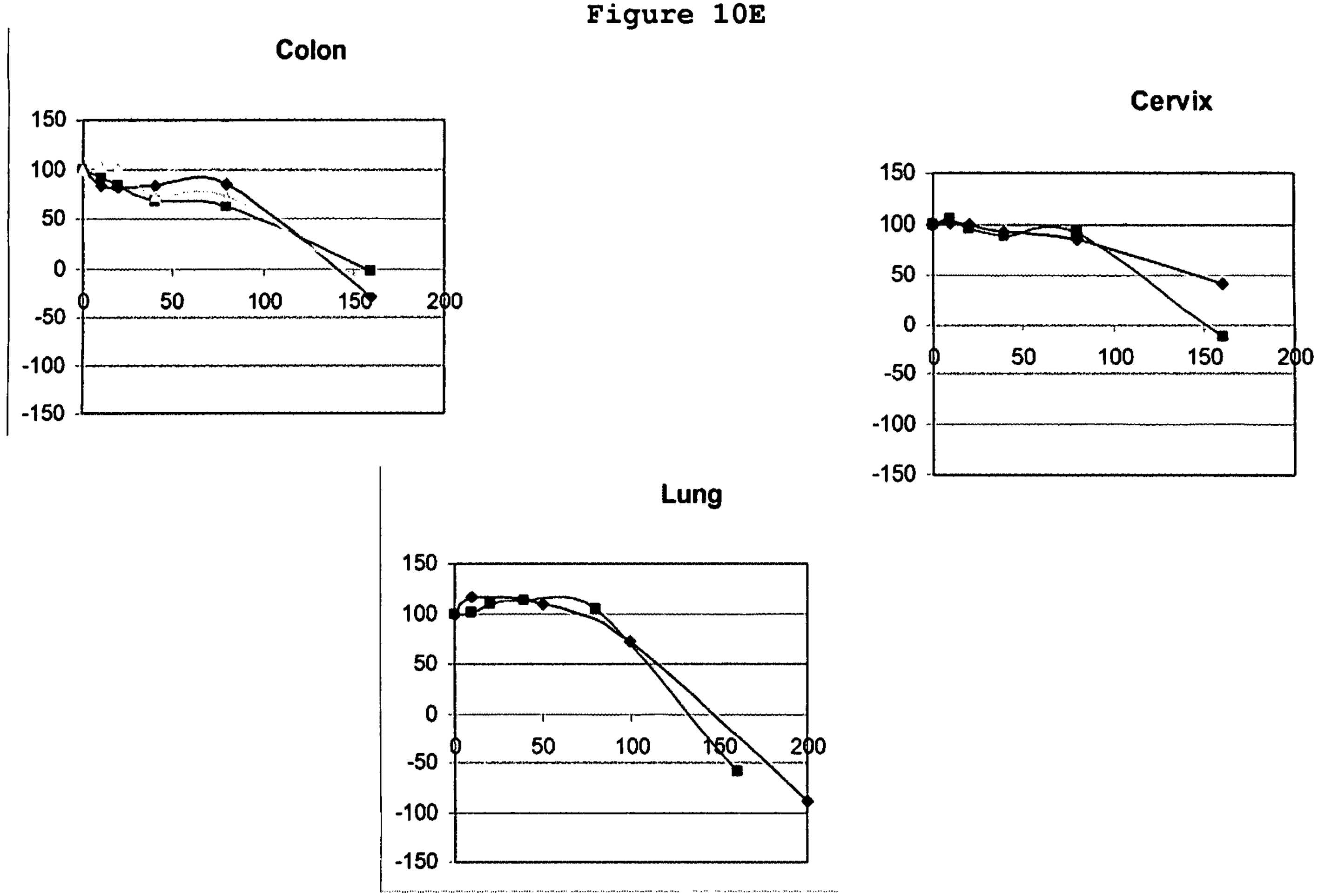


Least sensitive

Figure 11

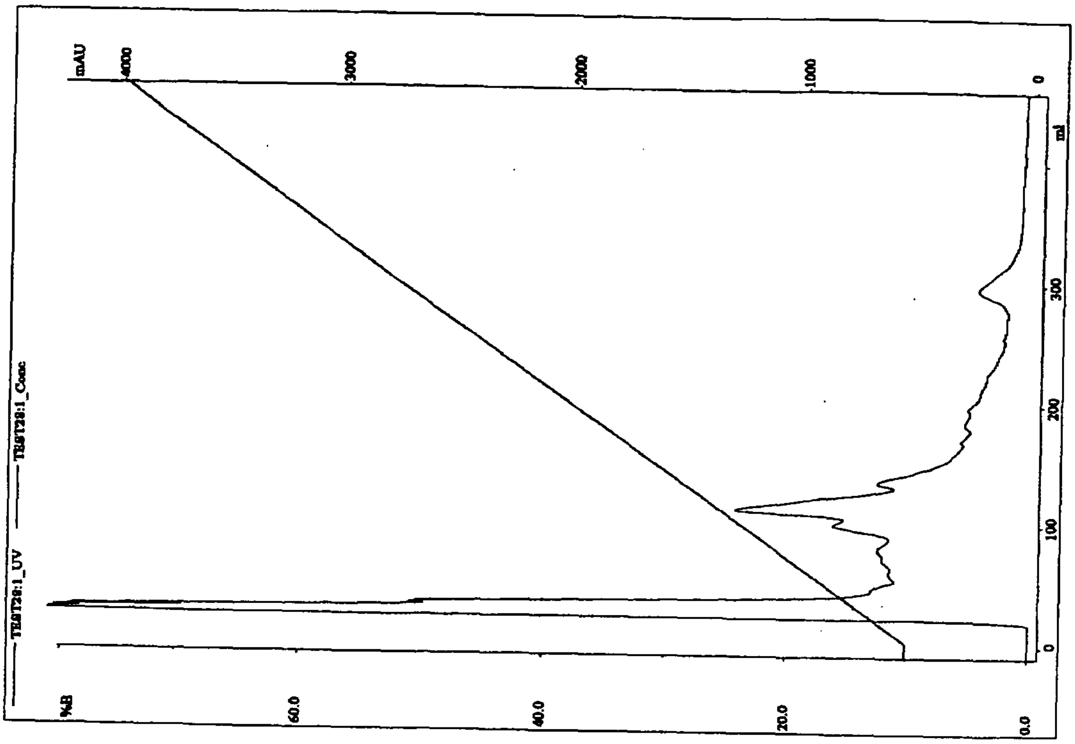
TEST28:1_UV
TEST28:1_Conc
%B
60.0
40.0
20.0
0.0
mAU
4000
3000
2000
1000
0
ml
0
100
200
300

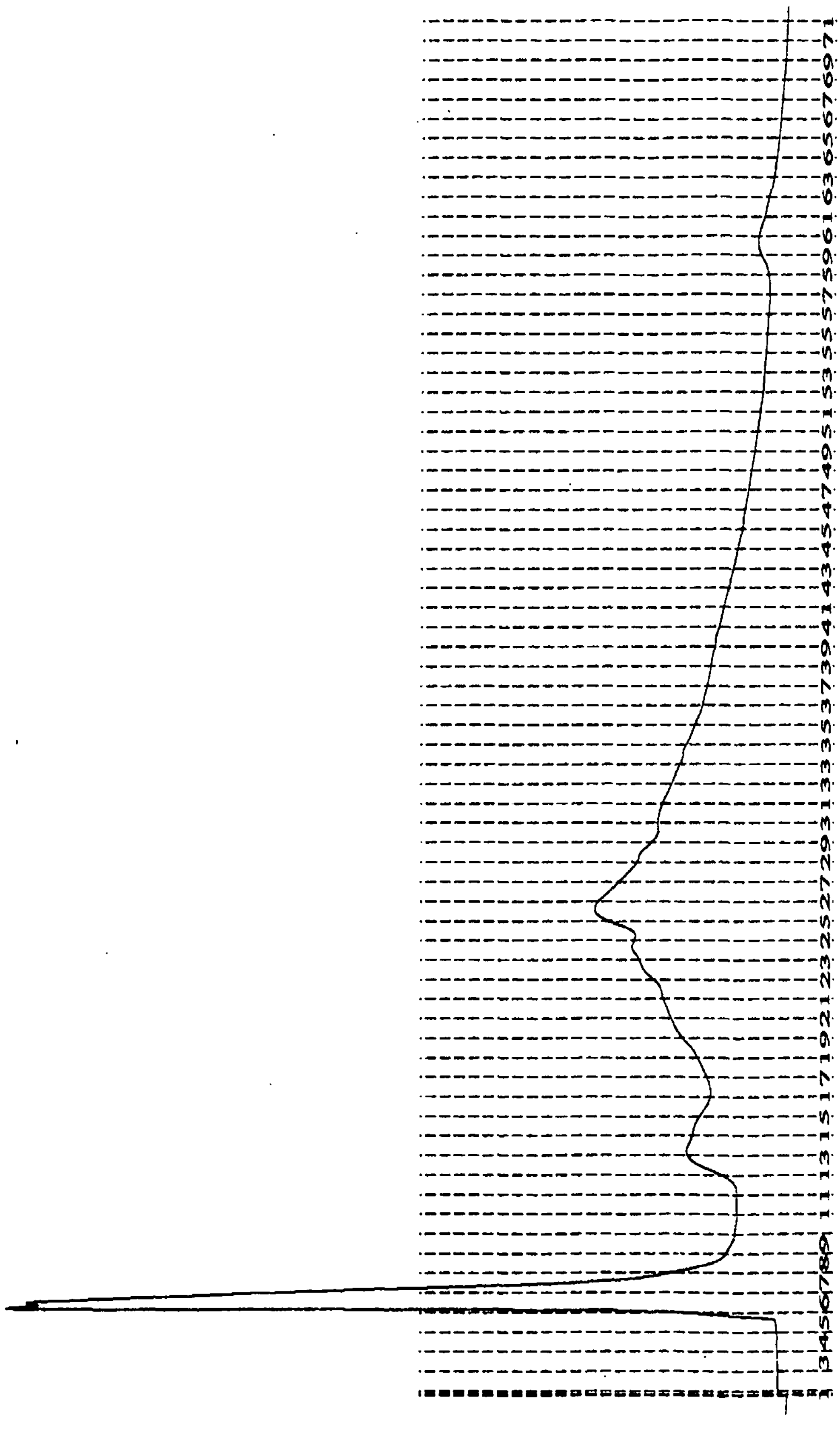

Figure 12A

Figure 12B

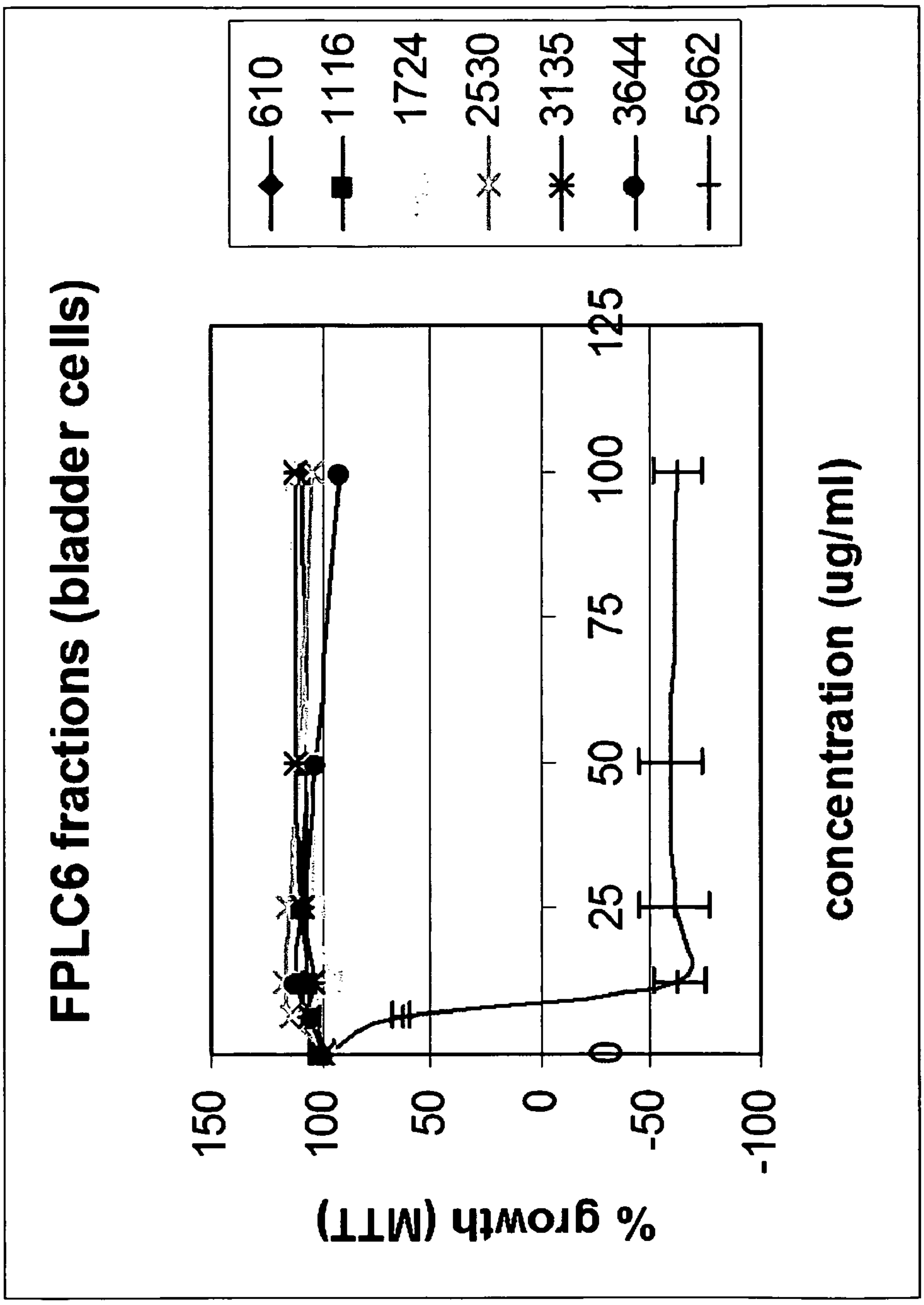
FPLC6 fractions (bladder cells)
% growth (MTT)
150
100
50
0
-50
-100
0
25
50
75
100
125
concentration (ug/ml)
610
1116
1724
2530
3135
3644
5962

Figure 13

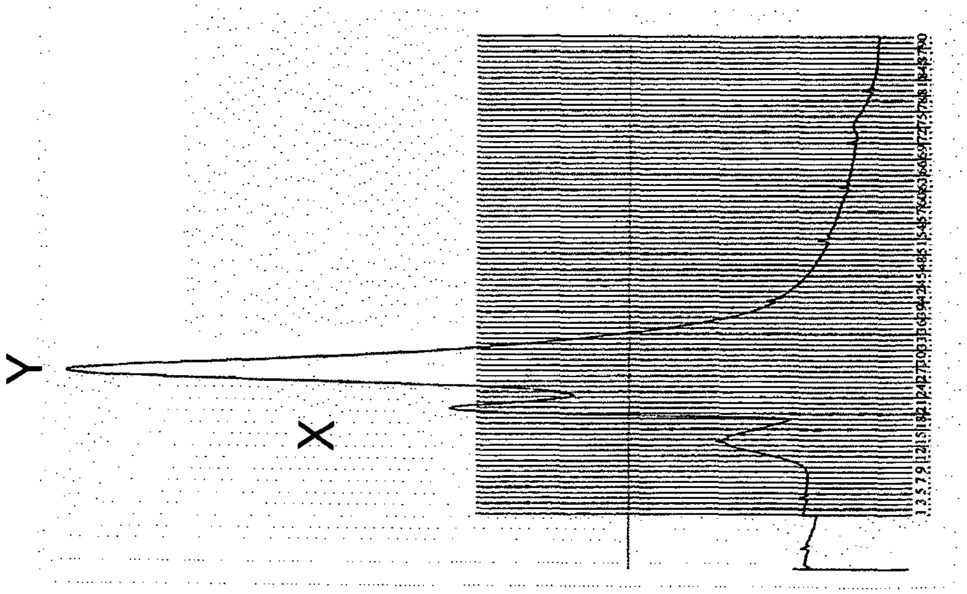

Figure 14

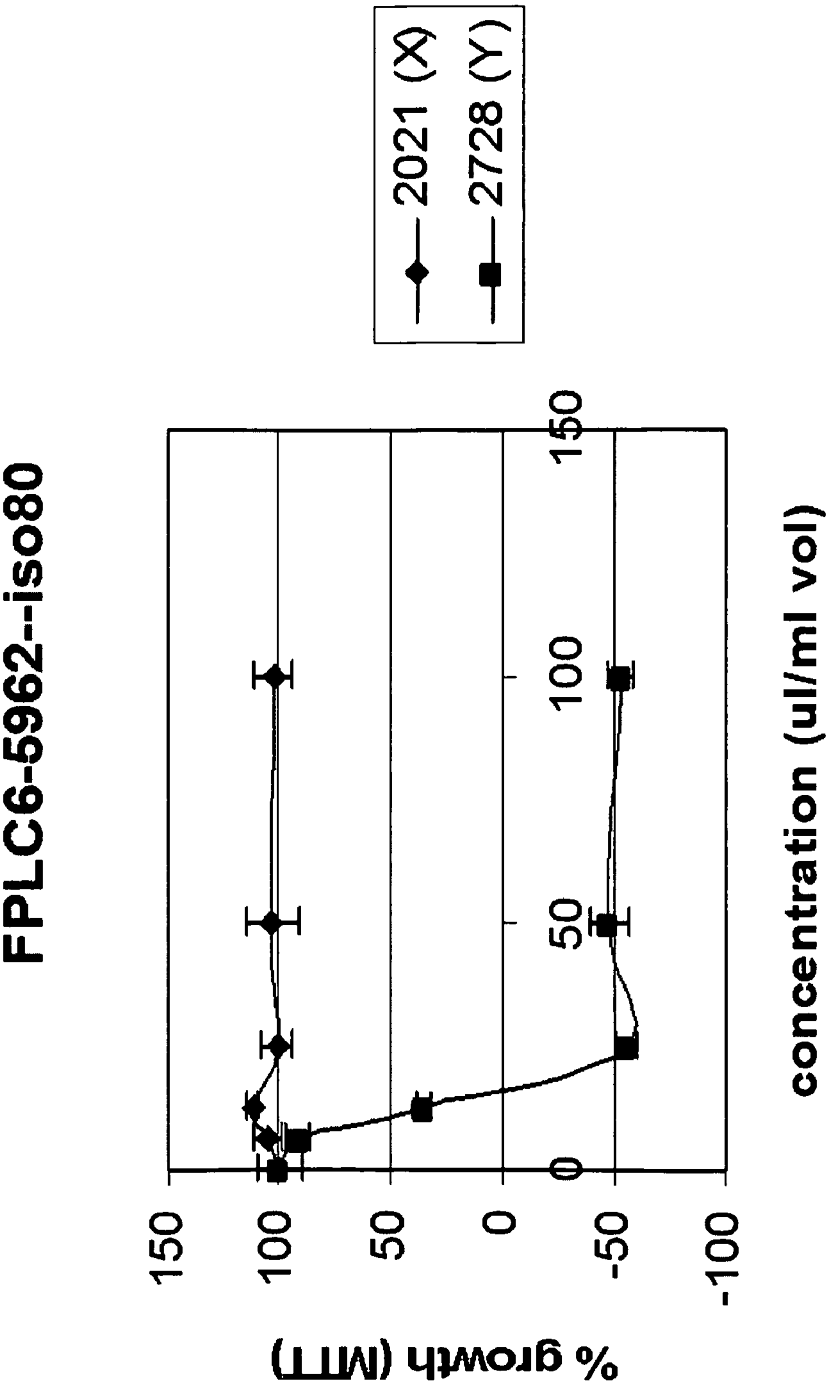
FPLC6-5962--iso80
% growth (MTT)
150
100
50
0
-50
-100
0
50
100
150
concentration (ul/ml vol)
2021 (X)
2728 (Y)

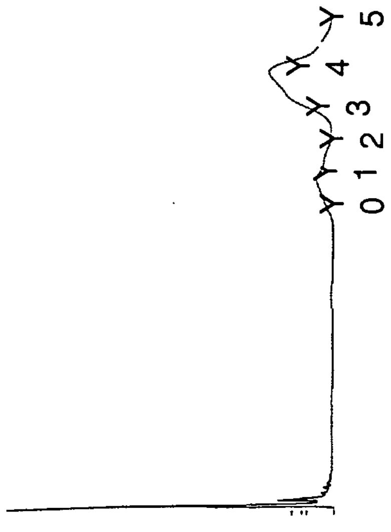


Figure 15

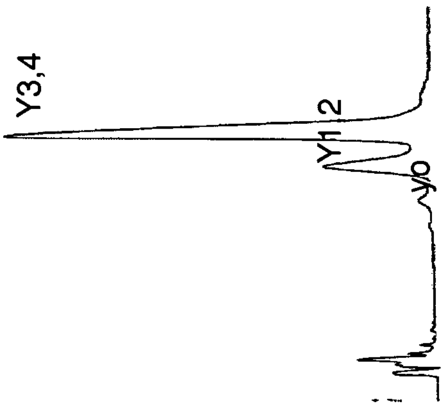


Figure 16

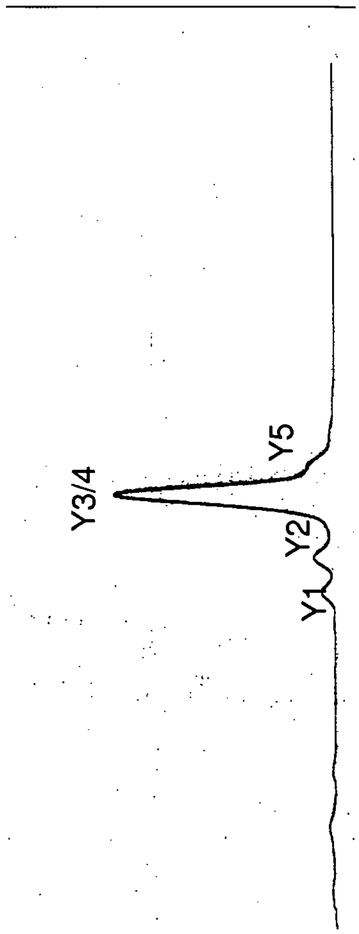


Figure 17

Figure 18

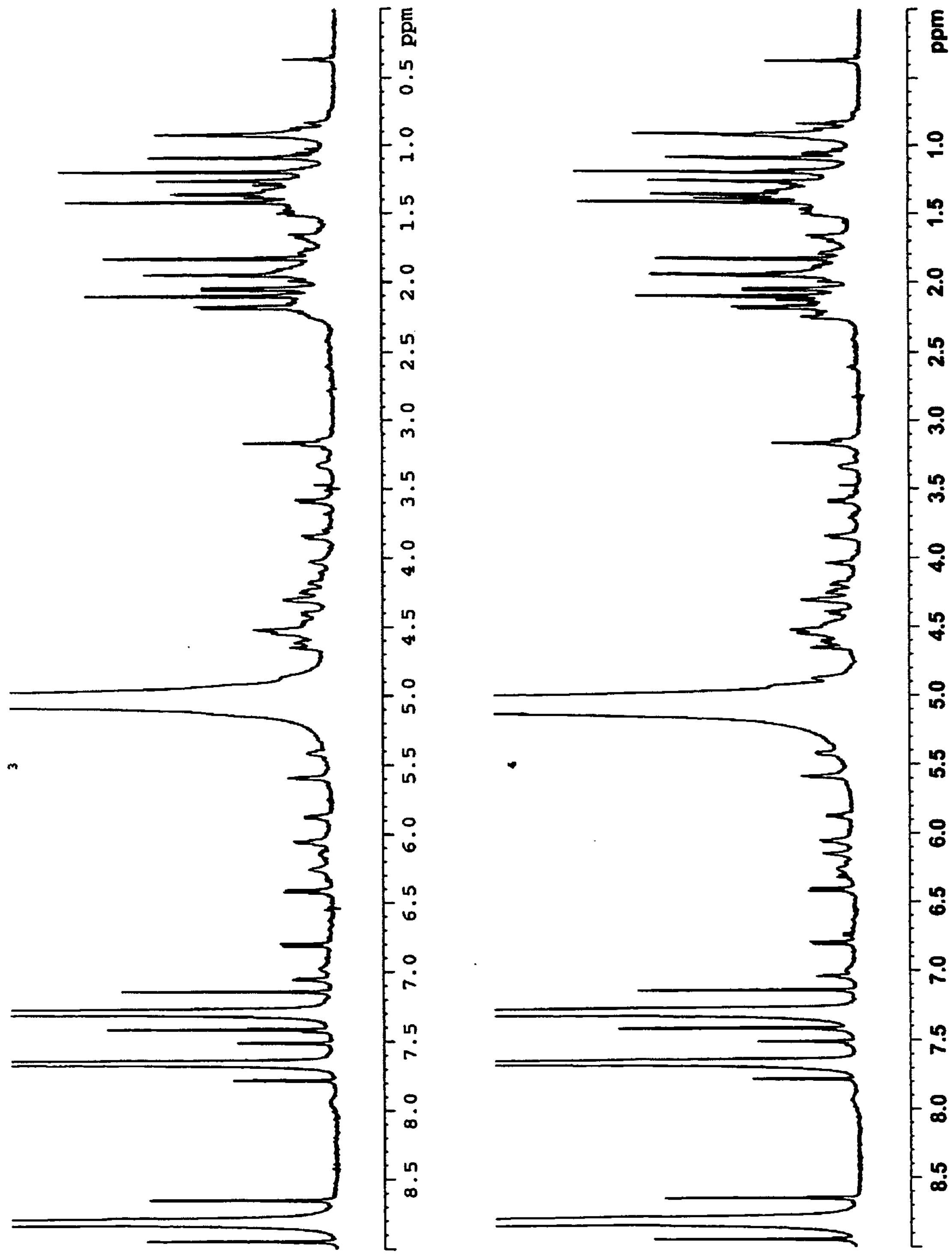

Figure 19

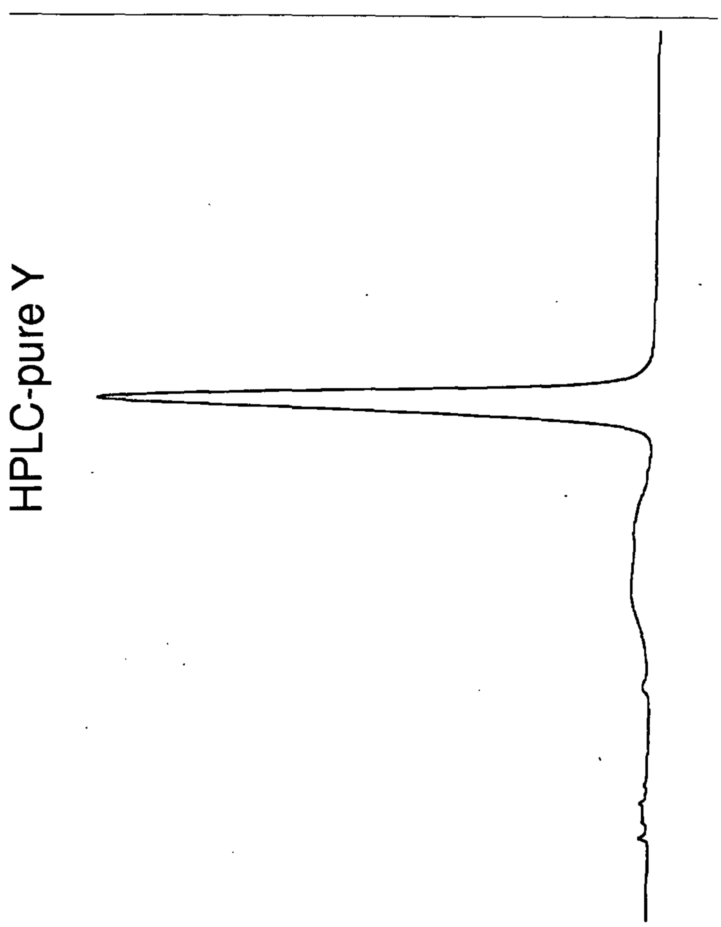

Figure 20A

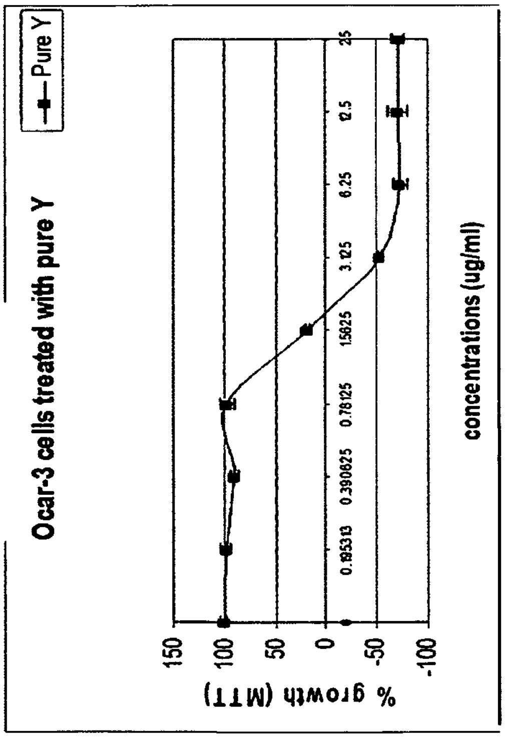


Figure 20B

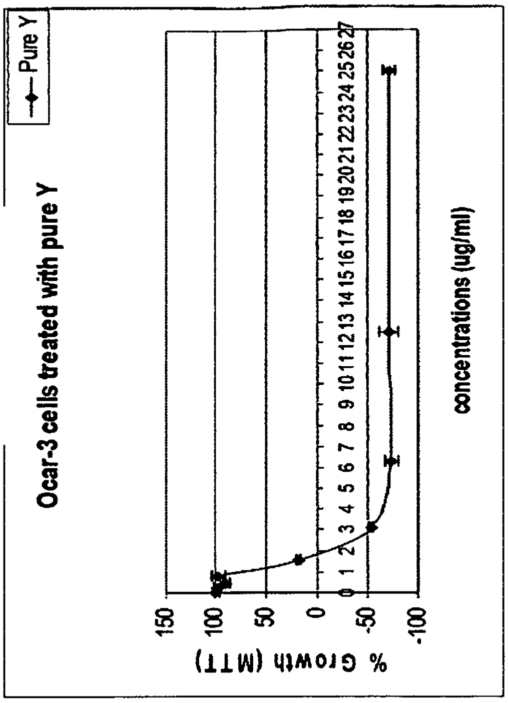


Figure 21

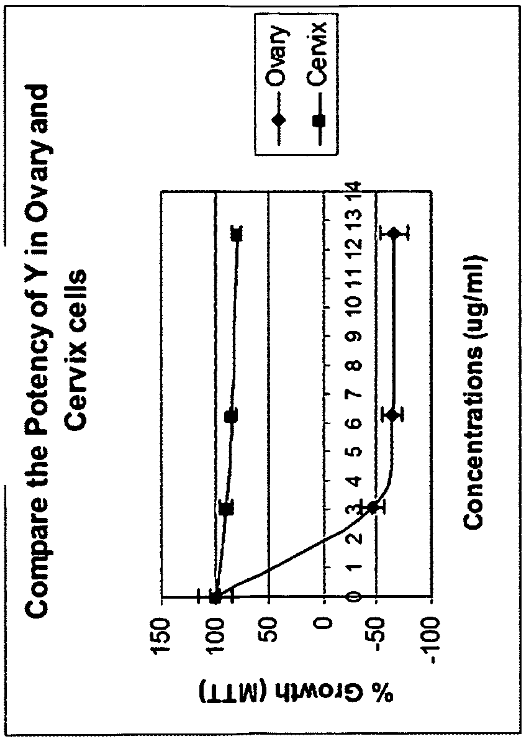

Figure 22

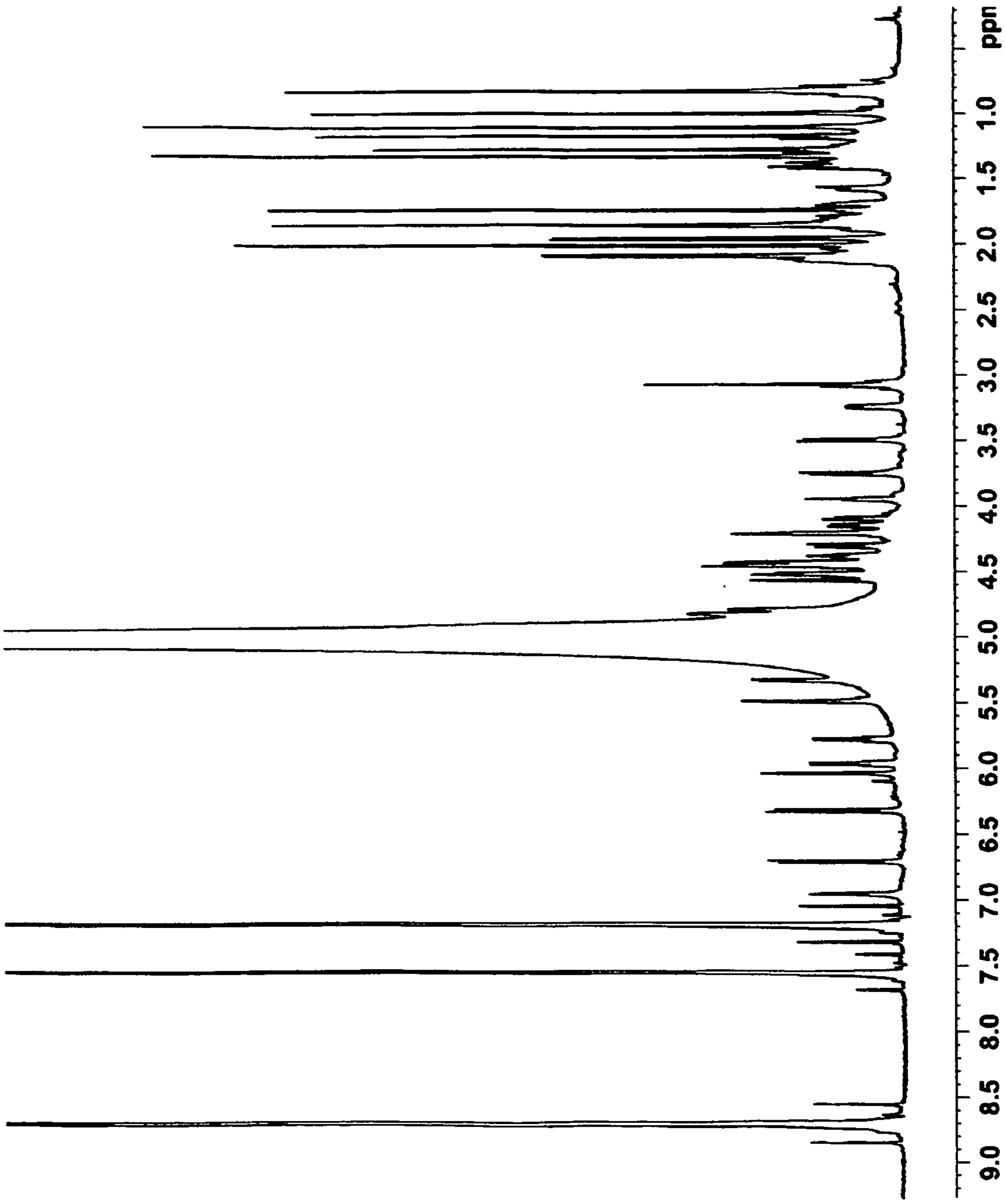

Figure 23

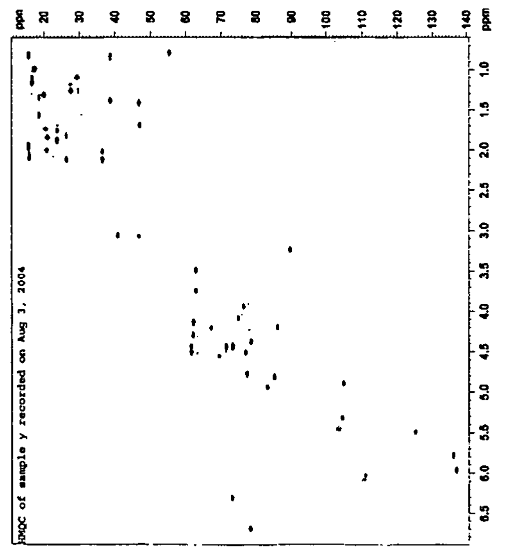


Figure 24B

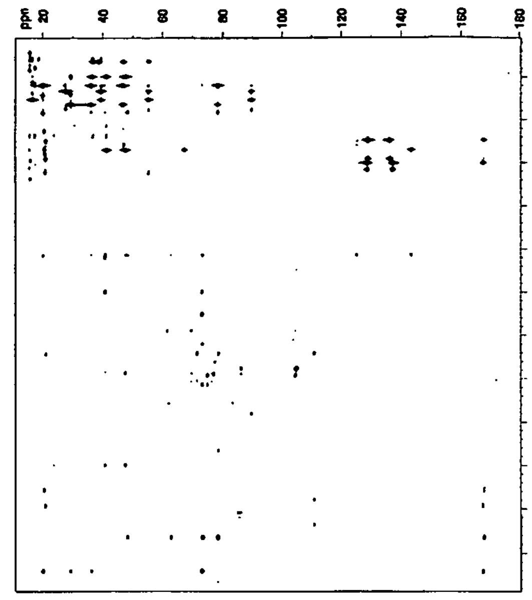

Figure 24A

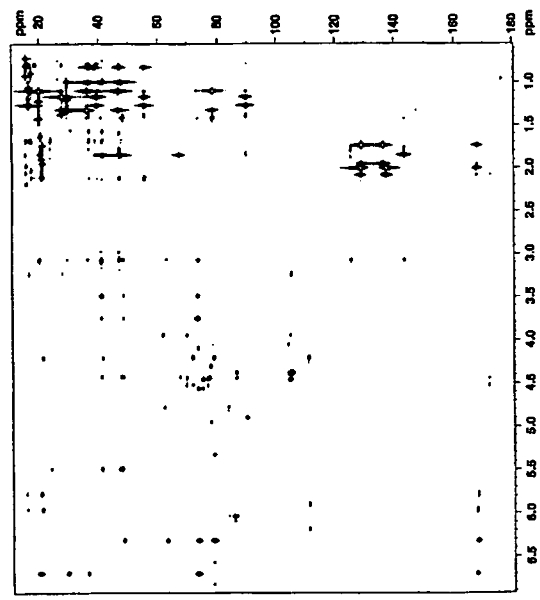

**Figure 25A-1**

Y + Matrix (CHCA) + Angiotensin 1 "two point Calibration"

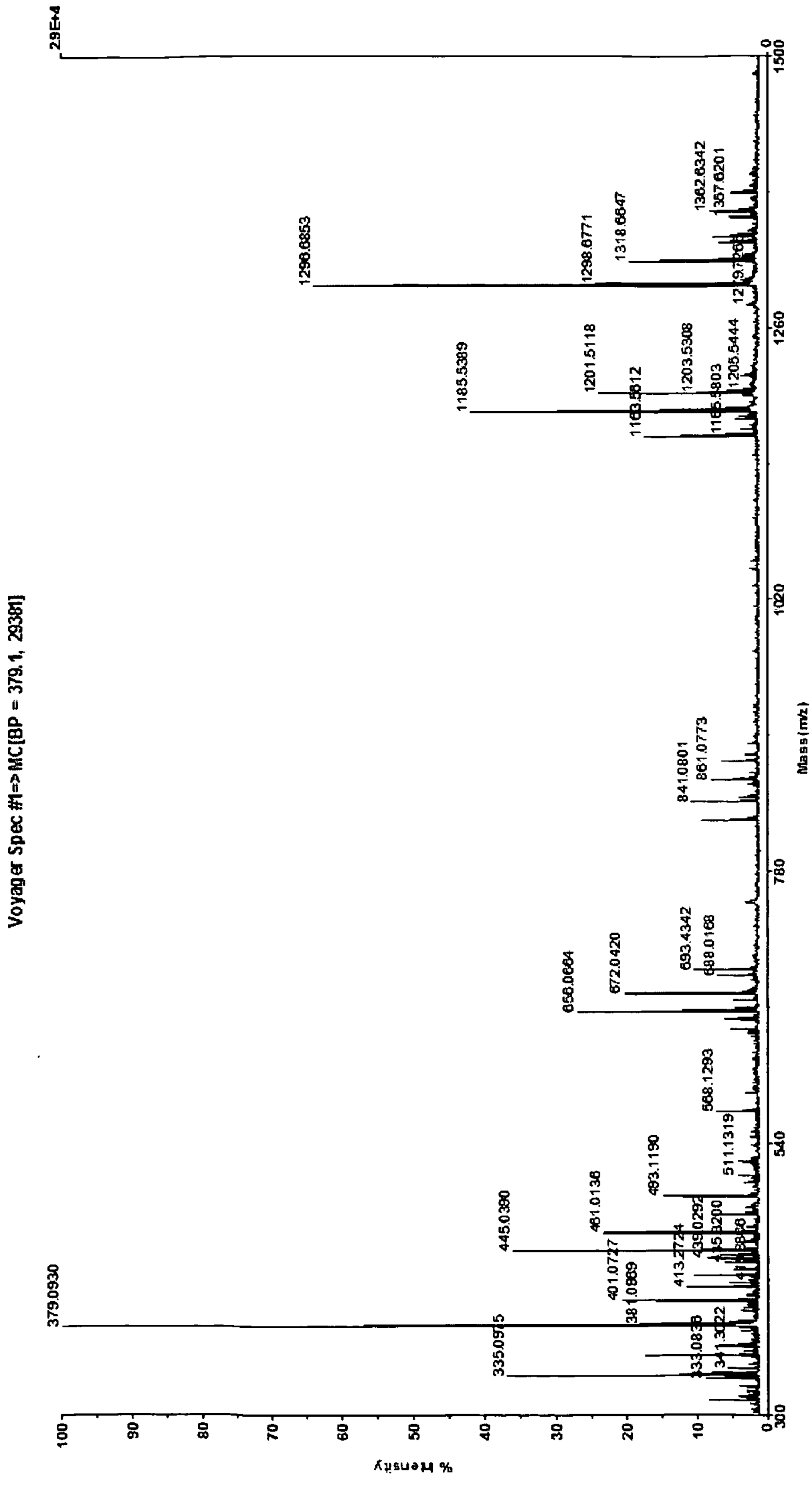

Figure 25A-2

Y + Matrix (CHCA) + Angiotensin "One point Calibration"

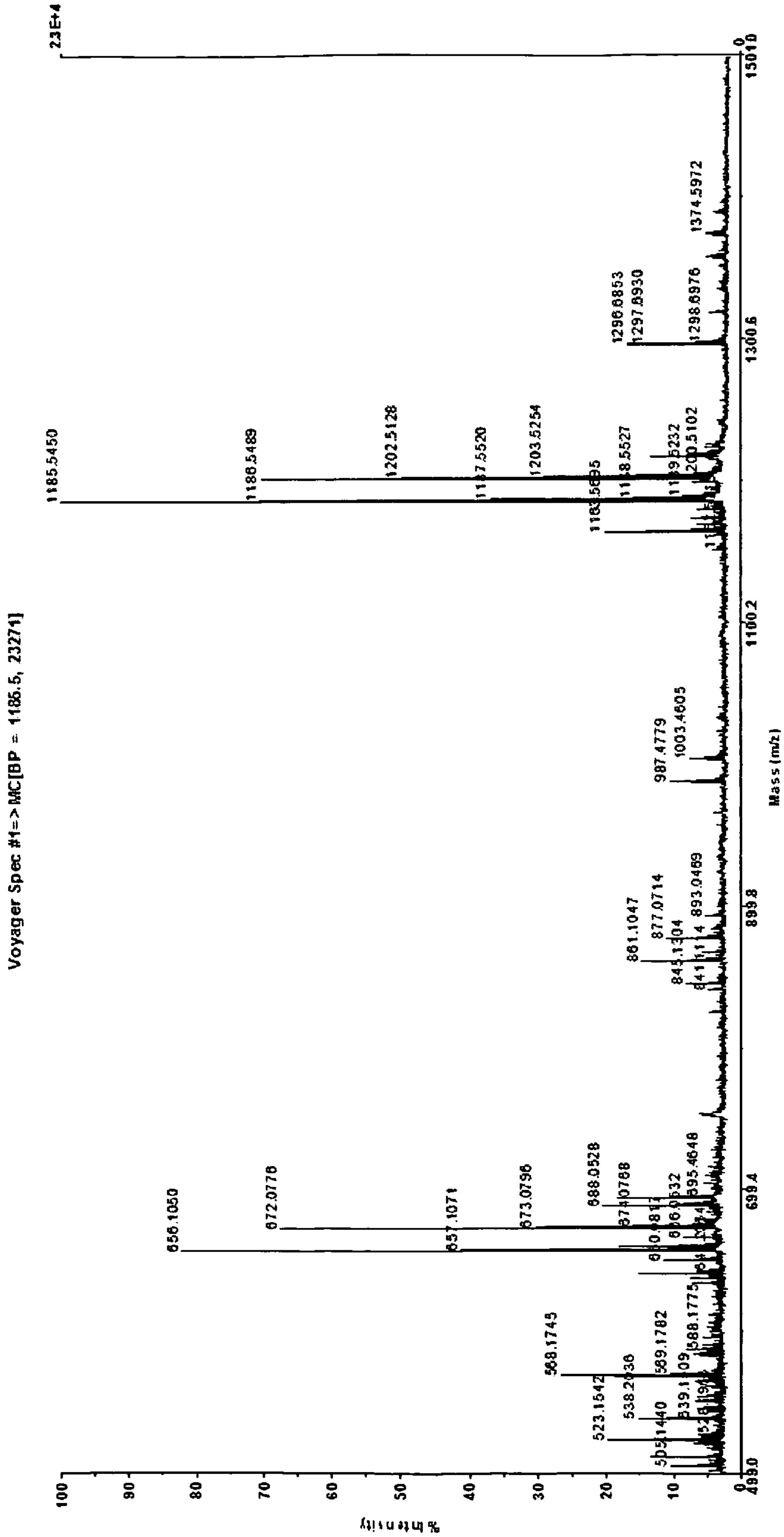

Y + Matrix (CHCA)

**Figure 25A-3**
MALDI-TOF

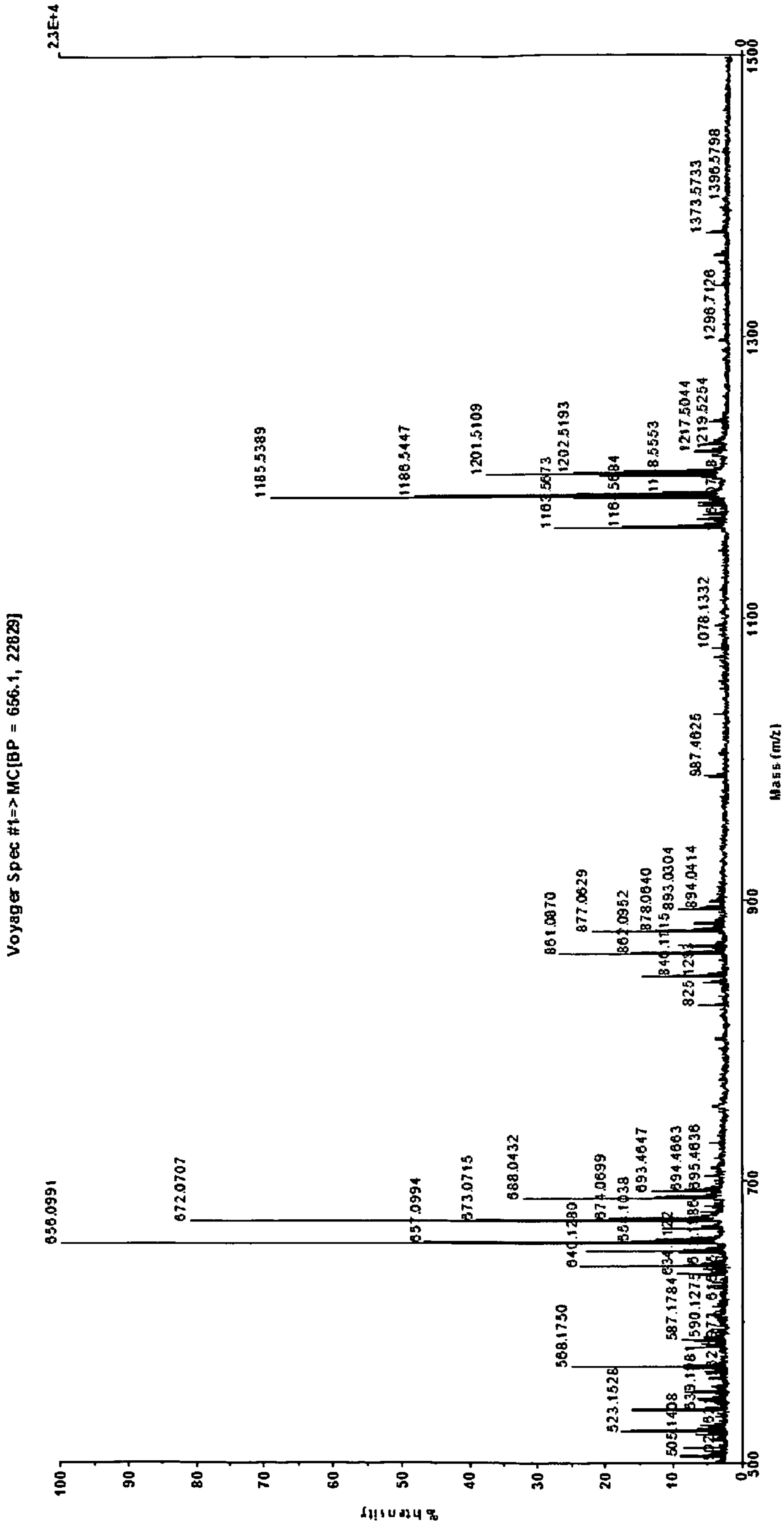

**Figure 25A-4**
MALDI-TOF

Matrix (CHCA) only

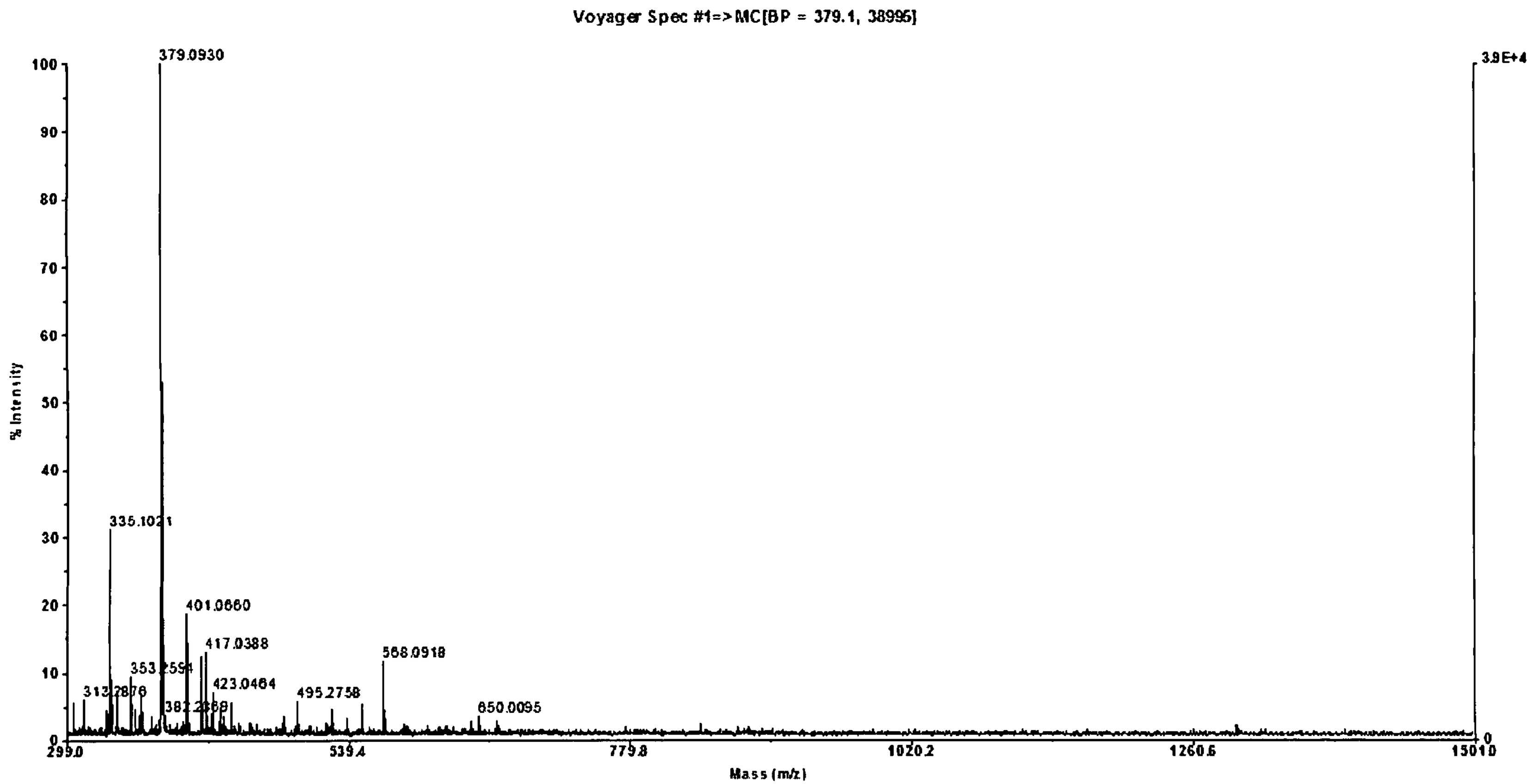

**Figure 25A-5**
MALDI-TOF

# Angiotensin 1 + Matrix (CHCA)

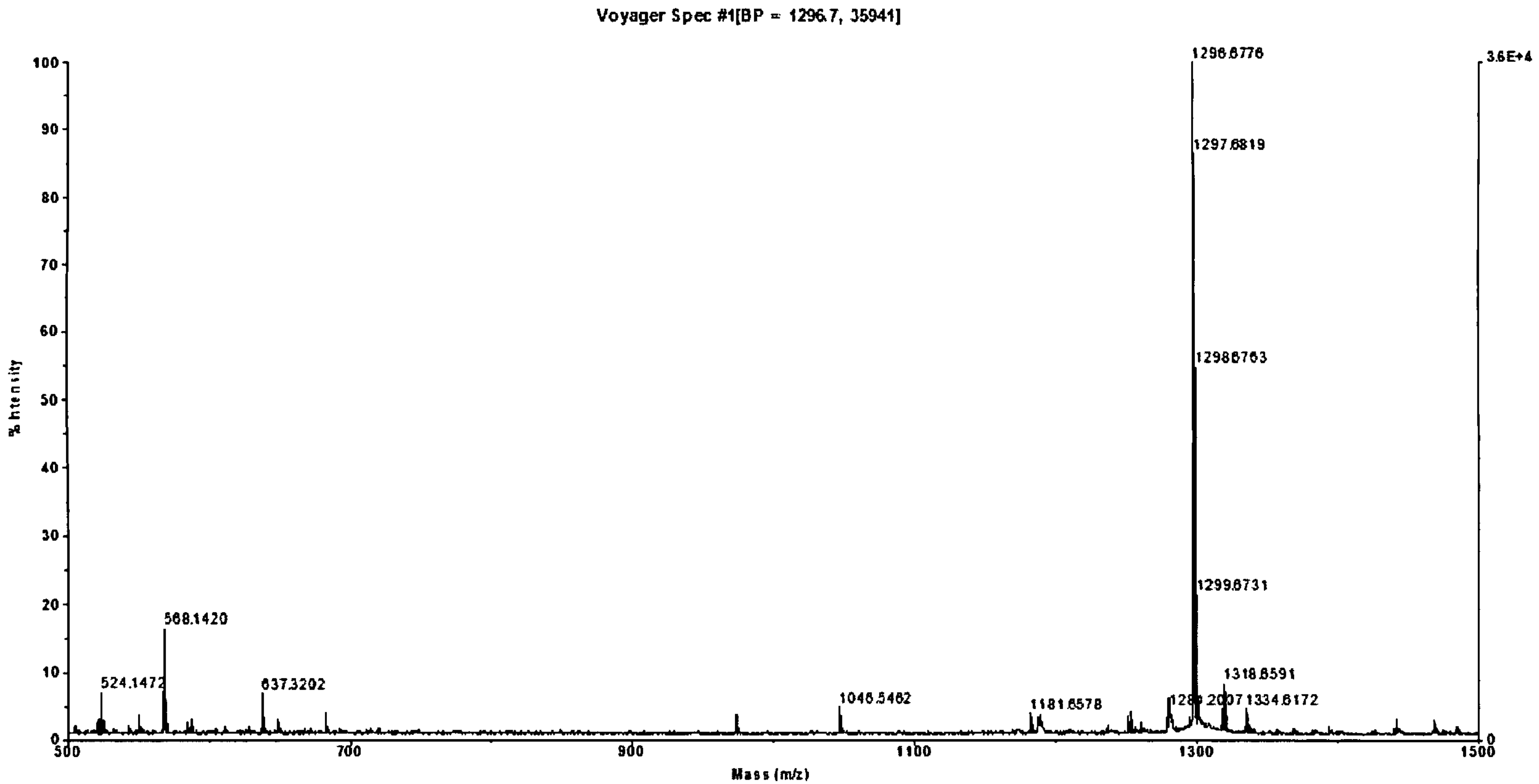

Figure 25B

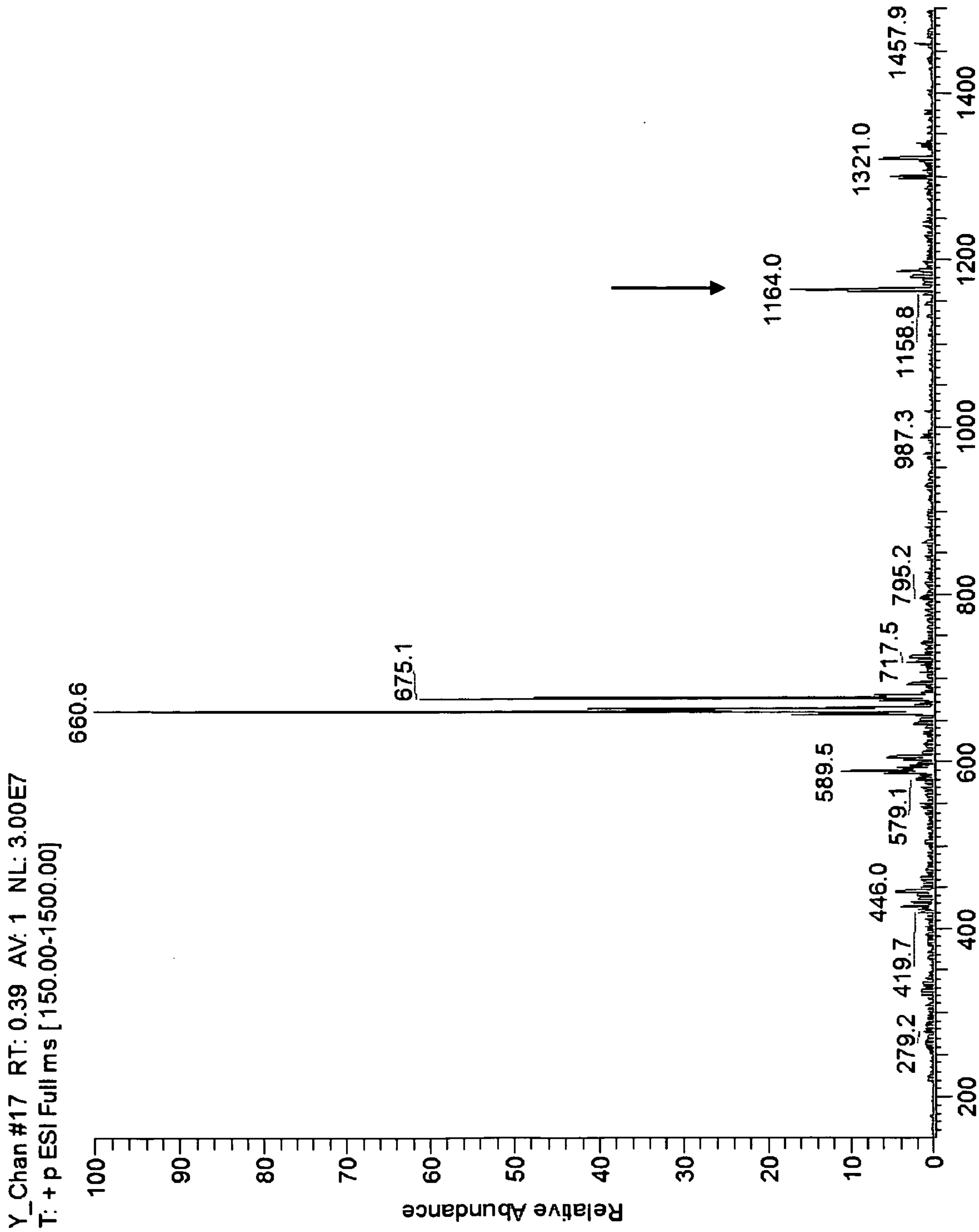
Y_Chan #17 RT: 0.39 AV: 1 NL: 3.00E7
T: + p ESI Full ms [ 150.00-1500.00]
Relative Abundance
m/z
660.6
675.1
589.5
717.5
579.1
795.2
446.0
987.3
419.7
1158.8
279.2
1164.0
1321.0
1457.9

Figure 26

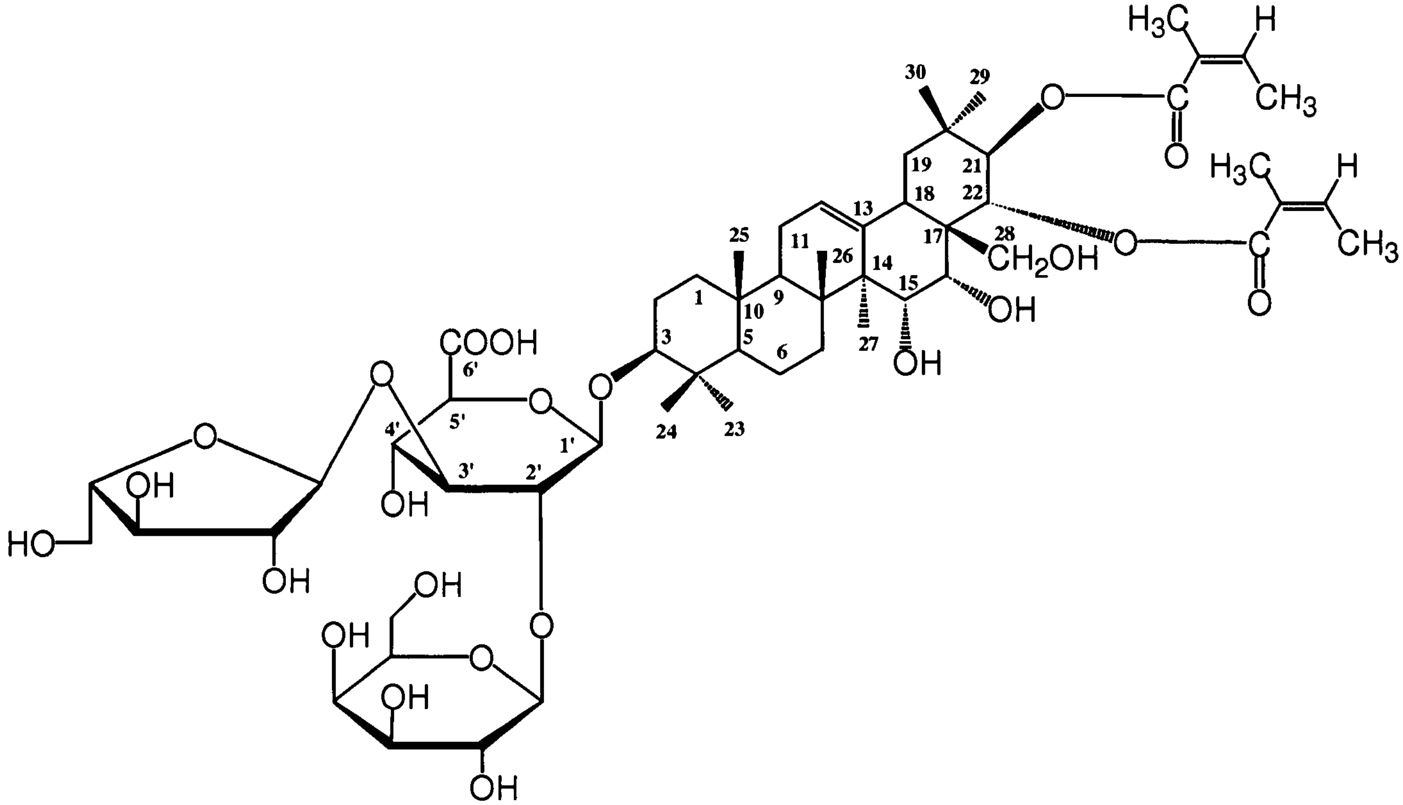

3-*O*-[*β*-D-galactopyranosyl(1→2)]-α-L-arabinofuranosyl(1→3)-*β*-D-glucuronopyranosyl-21,22-*O*-diangeloyl-3*β*, 15α, 16α, 21*β*, 22α, 28-hexahydroxyolean-12-ene.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60509851 A
- US 60532101 B
- CN 1092991 A
- CN 1092992 A
- CN 1052636 C
- US 20030096030 A
- US 20030082293 A
- US 6616943 B


### Non-patent literature cited in the description


- *Journal of Shenyang University of Pharmacy,* 2001, vol. 18 (1), 53-56
- **YINGJIE CHEN ; TADAHIRO TAKEDA ; YUKIO OGIHARA.** *Chem. Pharm. Bull,* 1985, vol. 33 (4), 1387-1394
- **YINGJIE CHEN ; TADAHIRO TAKEDA ; YUKIO OGIHARA.** *Chem. Pharm. Bull,* 1985, vol. 33 (3), 1043-1048
- **YINGJIE CHEN ; TADAHIRO TAKEDA ; YUKIO OGIHARA.** *Chem. Pharm. Bull,* 1985, vol. 33 (1), 127-134